(19) Europäisches Patentamt
European Patent Office
Office européen des brevets

(11) EP 4 083 051 A1

(12) **EUROPEAN PATENT APPLICATION**
published in accordance with Art. 153(4) EPC

(43) Date of publication:
**02.11.2022 Bulletin 2022/44**

(21) Application number: **20905209.1**

(22) Date of filing: **25.12.2020**

(51) International Patent Classification (IPC):
***C07K 1/06*** *(2006.01)*

(52) Cooperative Patent Classification (CPC):
**C07K 1/06;** Y02P 20/55

(86) International application number:
**PCT/JP2020/048651**

(87) International publication number:
**WO 2021/132545 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.12.2019 JP 2019238793**

(71) Applicant: **CHUGAI SEIYAKU KABUSHIKI KAISHA**
**Tokyo 115-8543 (JP)**

(72) Inventors:
- **KONDO, Yasuhiro**
  **Tokyo 115-8543 (JP)**
- **SETO, Kentarou**
  **Tokyo 115-8543 (JP)**
- **KOMIYA, Shio**
  **Tokyo 115-8543 (JP)**
- **HOU, Zengye**
  **Tokyo 115-8543 (JP)**
- **MURAKATA, Masatoshi**
  **Tokyo 115-8543 (JP)**

(74) Representative: **Vossius & Partner**
**Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) METHOD FOR SYNTHESIZING PEPTIDE COMPOUND

(57) The present inventors found that, in the synthesis of a peptide compound involving condensation of a C-terminal-activated substance of an acid component with an amine component, the C-terminal-activated substance can be removed by mixing a solution containing a residual C-terminal-activated substance after a condensation reaction with a tertiary amine and water or an aqueous solution.

EP 4 083 051 A1

## Description

[Technical Field]

The present invention relates to methods for efficiently producing a peptide compound of interest by efficiently removing an unnecessary C-terminal-activated substance generated during the course of synthesizing the peptide compound.

[Background Art]

In one form of peptide synthesis, a compound that is obtained by activating the C-terminal carboxyl group of amino acid or peptide is used so that it can react with amine such as amino acid or peptide to form an amide bond. In this case, the compound having an activated carboxyl group can become problematic when it remains in the reaction solution after completion of the reaction, as it causes deterioration of quality of the produced peptide.

Such a compound having the activated C-terminus includes a carboxyl group-activated compound used in the peptide synthesis reaction, and moreover a compound resulting from transformation of the carboxyl group-activated compound into, for example, azlactone, NCA (N-carboxyanhydride), or the like during the reaction, which compound has an activated state and thus is capable of reacting with amine (hereinafter, such compounds may be referred to as "C-terminal-activated substances"). The C-terminal-activated substance used in a peptide synthesis reaction is not limited to an active ester, a mixed acid anhydride, acylisourea, and the like, synthesized using a peptide condensing agent as described in NPL 1 or NPL 2, for example, and includes any compound as long as it is activated so as to be capable of reacting with amine.

Examples of known causes of a poor quality of the produced peptide include formation of an impurity peptide as a by-product and contamination of a peptide of an insertion sequence into the final product as an impurity, which arises due to the residual C-terminal-activated substance (PTL 1 and PTL 2).

In a known method for solving such a problem of a residual C-terminal-activated substance, the active ester is hydrolyzed by treatment with alkaline water, and removed as an alkaline aqueous solution of the corresponding amino acid (PTL 1). However, this method requires a hydrolysis treatment with alkaline water to be carried out multiple times, and the operation is complex. In addition, an increased number of treatments and an extended time of treatment with alkaline water are expected to result in side-reactions such as epimerization (isomerization) of the product, and thus robustness may be impaired.

In another known method, a residual C-terminal-activated substance is captured by polyamine having a primary amino group such as N,N-dimethylpropane-1,3-diamine and converted to a basic compound, and then the resulting amide compound derived from the residual C-terminal-activated substance is transferred to an aqueous layer by washing with an acidic aqueous solution and removed (PTL 3 and NPL 3). However, when a highly nucleophilic primary amine is used, an impurity is expected to arise due to the reaction to form a covalent bond between the primary amine and a highly electrophilic site of the peptide of interest, and thus the method is not suitable for synthesis of a high-purity peptide.

In another known method, a residual C-terminal-activated substance is reacted with a scavenger that is an amine containing a latent anion having a protecting group, and thus converted to an amide compound and removed (PTL 2). However, this method requires a series of steps including formation of an amide compound, an aqueous extraction step, hydrogenolysis, and another aqueous extraction step, and thus the operation is complex.

Meanwhile, when a C-terminal-activated substance remains, removal of the N-terminal protecting group of the C-terminal-activated substance may simultaneously occur at the time of removing the N-terminal protecting group of the produced peptide. The deprotection product of the residual C-terminal-activated substance is an impurity, and it is difficult to detect this deprotection product by commonly used HPLC when the absorption coefficient is small and, accordingly, the residual C-terminal-activated substance is not preferable in terms of quality control.

[Citation List]

[Patent Literature]

[PTL 1] Japanese Patent Publication No. 5171613

[PTL 2] Japanese Patent Publication No. 4142907

[PTL 3] Japanese Patent Publication No. 5212371

[Non-Patent Literature]

[NPL 1] Chem. Rev., 2011, 111, 6557.
[NPL 2] Organic Process Research & Development, 2016, 20, 140.
[NPL 3] Tetrahedron Lett., 1974, 15, 1785.

[Summary of Invention]

[Technical Problem]

The present invention was achieved in view of the above circumstances, and in one aspect, an objective of the present invention is to efficiently remove a residual C-terminal-activated substance in the synthesis of a peptide compound.

[Solution to Problem]

For the peptide compound synthesis comprising condensing a C-terminal-activated substance of an acid component with an amine component, the present inventors found a method capable of removing a residual C-terminal-activated substance present in a reaction mixture by allowing a tertiary amine to act on the residual C-terminal-activated substance.

The present invention encompasses the following in one non-limiting specific embodiment.

[1] A method of producing a peptide compound, comprising:

step A: a step of obtaining a reaction mixture comprising a peptide compound obtained by condensing a C-terminal-activated substance of an acid component with an amine component in a solvent; and
step B: a step of mixing the reaction mixture, a tertiary amine, and water or an aqueous solution to remove the C-terminal-activated substance.

[2] A method of producing a peptide compound, comprising:

step A: a step of obtaining a reaction mixture comprising a peptide compound obtained by condensing a C-terminal-activated substance of an acid component with an amine component in a solvent; and
step B: a step of mixing the reaction mixture, a tertiary amine, and water or an aqueous solution to allow the tertiary amine to act on an unreacted C-terminal-activated substance and thereby removing the C-terminal-activated substance.

[3] The method of [1] or [2], wherein the acid component is a first amino acid having an amino group protected with a protecting group, or a first peptide having an N-terminal amino group protected with a protecting group.
[4] The method of any one of [1] to [3], wherein the amine component is a second amino acid having a carboxyl group protected with a protecting group, or a second peptide having a C-terminal carboxyl group protected with a protecting group.
[5] The method of any one of [1] to [4], wherein step A is performed in the presence of a condensing agent.
[6] The method of any one of [1] to [5], wherein the tertiary amine is nucleophilic to the C-terminal-activated substance.
[7] The method of any one of [1] to [6], wherein the tertiary amine is an amine having small steric hindrance in the vicinity of nitrogen.
[8] The method of any one of [1] to [7], wherein the tertiary amine is represented by formula (A), (B), or (C) below:

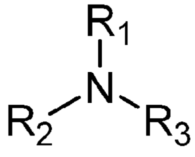 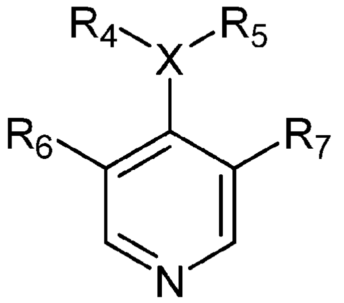 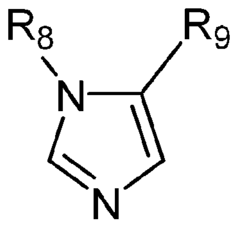

(A) (B) (C)

wherein

Ri to $R_3$ are (i) $R_1$ and $R_2$ which, together with a nitrogen atom to which they are attached, form a 5- to 6-membered non-aromatic heterocyclic ring, and $R_3$ which is $C_1$-$C_2$ alkyl or $C_2$ hydroxyalkyl, or (ii) $R_1$ to $R_3$ which are each independently $C_1$-$C_2$ alkyl or $C_2$ hydroxyalkyl;
X is N or O;
$R_4$ and $R_5$ are each independently $C_1$-$C_2$ alkyl or $C_2$ hydroxyalkyl, or $R_4$ and $R_5$, together with a nitrogen atom to which they are attached, form a 5- to 6-membered non-aromatic heterocyclic ring, provided that $R_5$ does not exist when X is O;
$R_6$ and $R_7$ are each independently H, $C_1$-$C_2$ alkyl, or methoxy; and
Rs and $R_9$ are each independently H, $C_1$-$C_2$ alkyl, or $C_2$ hydroxyalkyl, or $R_8$ and $R_9$, together with a nitrogen atom to which $R_8$ is attached and a carbon atom to which $R_9$ is attached, form a 5- to 6-membered non-aromatic heterocyclic ring.

[9] The method of [8], wherein $R_1$ to $R_3$ are each independently $C_1$-$C_2$ alkyl.
[10] The method of [8], wherein X is N, $R_4$ and $R_5$ are each independently $C_1$-$C_2$ alkyl, and $R_6$ and $R_7$ are H.
[11] The method of [8], wherein $R_5$ and $R_9$ are each independently H or $C_1$-$C_2$ alkyl.
[12] The method of any one of [1] to [11], wherein the tertiary amine is NMI, DMAP, or trimethylamine.
[13] The method of any one of [1] to [12], wherein the peptide compound comprises one or more non-natural amino acids.
[14] The method of any one of [1] to [13], wherein a temperature for allowing the tertiary amine to act on the C-terminal-activated substance is 25°C to 60°C.
[15] The method of any one of [1] to [14], wherein the tertiary amine is added in an amount of 0.5 equivalents or more relative to the amine component.
[16] The method of any one of [1] to [15], wherein a residual rate of the C-terminal-activated substance is 3% or less.
[17] The method of any one of [1] to [16], wherein step B further comprises separating the reaction mixture into an organic layer and an aqueous layer and then washing the organic layer, and wherein a residual amount of the C-terminal-activated substance after the washing is 1.0% or less.
[18] The method of any one of [1] to [17], wherein the solvent in step A is toluene, acetonitrile, tetrahydrofuran, 2-methyltetrahydrofuran, isopropyl acetate, ethyl acetate, methyl tert-butyl ether, cyclopentyl methyl ether, or N,N-dimethylformamide, or a mixed solvent thereof.
[19] The method of any one of [1] to [18], wherein in step B, the aqueous solution is an alkaline aqueous solution.
[20] The method of any one of [1] to [19], wherein a side chain of the first amino acid comprises one or more carbon atoms.
[21] The method of [20], wherein the side chain is optionally substituted alkyl, optionally substituted alkenyl, optionally substituted alkynyl, optionally substituted cycloalkyl, optionally substituted alkoxyalkyl, optionally substituted cycloalkylalkyl, optionally substituted aralkyl, or optionally substituted heteroarylalkyl.
[22] The method of any one of [1] to [21], wherein a time for allowing the tertiary amine to act on the C-terminal-activated substance is 2 hours or less.
[23] The method of any one of [1] to [22], wherein a time for allowing the tertiary amine to act on the C-terminal-activated substance is 2 minutes to 2 hours.
[24] The method of any one of [1] to [23], wherein a time for allowing the tertiary amine to act on the C-terminal-activated substance is 5 minutes to 60 minutes.
[25] The method of any one of [1] to [24], wherein a time for allowing the tertiary amine to act on the C-terminal-activated substance is 5 minutes to 50 minutes.

[26] The method of any one of [1] to [25], wherein the C-terminal-activated substance is formed in the presence of a condensing agent, and wherein the condensing agent comprises T3P, HATU, BEP, DMT-MM, a combination of EDC and PfpOH, a combination of EDC and HOOBt, or a combination of EDC and HOBt.
[27] The method of any one of [1] to [26], further comprising:
step C: a step of removing an N-terminal protecting group of the peptide compound.
[28] The method of [1] to [27], wherein the C-terminal-activated substance is allowed to be acted on by the tertiary amine and hydrolyzed, and is removed.
[29] A method for promoting hydrolysis of a C-terminal-activated substance, comprising a step of adding a tertiary amine and water or an aqueous solution to a solution comprising a residual C-terminal-activated substance to allow the tertiary amine to act on the C-terminal-activated substance.
[30] A method for removing a hydrolyed product of a residual C-terminal-activated substance, comprising a step of aqueously washing a solution comprising the hydrolyzed product.

[Effects of the Invention]

By using the method of the present invention, a C-terminal-activated substance remaining after a condensation reaction can be readily and efficiently removed in a short period of time by a single hydrolysis treatment and subsequent aqueous washing, and thus a peptide compound having high purity can be synthesized without column purification.

[Brief Description of the Drawings]

Fig. 1 is a graph showing a relative value of the residual amount of a C-terminal-activated substance.
Fig. 2 is a graph showing a relative value of the residual amount of a C-terminal-activated substance.
Fig. 3 is a graph showing a relative value of the residual amount of a C-terminal-activated substance.
Fig. 4 is a graph showing a change over time of the residual rate of a C-terminal-activated substance.

[Description of Embodiments]

Below, preferable non-limiting embodiments of the present disclosure are described.
All elements described in the present Examples below are described with such an intention that they are naturally deemed as being equally described in the present "Description of Embodiments" without being bound by any limitation such as patent practice, customs, laws and regulations that may be used to interpret the contents of the Examples in a limited way in countries where patent protection of the present patent application is sought.
Any combination of a part or the entirety of one or more elements described elsewhere in the present disclosure is also intended to be included in the present disclosure and described so as to be naturally interpreted by those skilled in the art as long as such a combination is not technically contradictory based on common general technical knowledge of those skilled in the art.

(Abbreviations)

The abbreviations used herein are as follows.

Abbreviations for amino acids

Aib: α-Methylalanine
Ala: Alanine
Arg: Arginine
Asn: Asparagine
Asp: Aspartic acid
Asp(tBu): O-t-Butyl aspartate
Aze: Azetidine-2-carboxylic acid
Cys: Cysteine
Glu: Glutamic acid
Gln: Glutamine

Gly: Glycine
His: Histidine
Hph: Homophenylalanine
Ile: Isoleucine
Leu: Leucine
Lys: Lysine
MeAla: N-Methylalanine
MeAsp(tBu): N-Methyl O-t-butyl aspartate
MeGly: N-Methylglycine
MeIle: N-Methylisoleucine
MeLeu: N-Methylleucine
MePhe: N-Methylphenylalanine
MeVal: N-Methylvalin
Met: Methionine
Phe: Phenylalanine
Phe-OtBu: O-t-Butylphenylalanine
Phe(3-F): 3-Fluorophenylalanine
Pro: Proline
Ser: Serine
Ser(tBu): O-t-Butylserine
Thr: Threonine
Thr(tBu): O-t-Butyl-threonine
Trp: Tryptophan
Tyr: Tyrosine
Val: Valine

Abbreviations for reagents/solvents

BEP: 2-Bromo-1-ethylpyridinium tetrafluoroborate
DABCO: 1,4-Diazabicyclo[2.2.2]octane
DBU: 1,8-Diazabicyclo[5.4.0]undec-7-ene
DCM: Dichloromethane
DIPEA: Diisopropyldiethylamine
DMAP: Dimethylaminopyridine
DMT-MM: 4- (4,6-Dimethoxy-1,3,5-triazin-2-yl)-4-methylmorpholinium chloride
EDC: 1- (3-Dimethylaminopropyl)-3-ethylcarbodiimide
HATU: O-(7-Azabenzotriazol-1-yl)-N,N,N',N'-tetramethyluronium hexafluorophosphate
HOAt: 1-Azahydroxybenzotriazole
HOBt: 1-Hydroxybenzotriazole
HOOBt: 3,4-Dihydro-3-hydroxy-4-oxo-1,2,3 -benzotriazine
HOSu: N-Hydroxysuccinimide
MTBE: Methyl t-butyl ether
NMI: N-Methylimidazole
NMM: N-Methylmorpholine
T3P: Propylphosphonic anhydride (cyclic trimer)
TBAF: Tetrabutylammonium fluoride
TsOH: p-Toluenesulfonic acid

Abbreviations for functional groups

Bn: Benzyl
Boc: t-Butoxycarbonyl
Cbz: Benzyloxycarbonyl
Pfp: Pentafluorophenyl

Teoc: 2-(Trimethylsilyl)ethoxycarbonyl

(Definitions of functional groups and the like)

Examples of "halogen atoms" herein include F, Cl, Br, and I.

"Alkyl" herein means a monovalent group derived by removing any one hydrogen atom from an aliphatic hydrocarbon, and has a subset of hydrocarbyl or hydrocarbon group structures not containing either a heteroatom (which refers to an atom other than carbon and hydrogen atoms) or an unsaturated carbon-carbon bond but containing hydrogen and carbon atoms in its backbone. The alkyl includes linear and branched alkyls. Specifically, the alkyl has 1 to 20 carbon atoms ($C_1$-$C_{20}$, hereinafter "$C_p$-$C_q$" means that the number of carbon atoms is p to q), and is preferably $C_1$-$C_{10}$ alkyl, more preferably $C_1$-$C_6$ alkyl, and further preferably $C_1$-$C_2$ alkyl. Specific examples of alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

"Alkenyl" herein means a monovalent group having at least one double bond (two adjacent $SP^2$ carbon atoms). Depending on the configuration of a double bond and a substituent (if present), the geometrical form of the double bond can be entgegen (E) or zusammen (Z) as well as cis or trans configuration. The alkenyl includes linear and branched alkenyls. The alkenyl is preferably $C_2$-$C_{10}$ alkenyl, and more preferably $C_2$-$C_6$ alkenyl, and specific examples include vinyl, allyl, 1-propenyl, 2-propenyl, 1-butenyl, 2-butenyl (including cis and trans forms), 3-butenyl, pentenyl, 3-methyl-2-butenyl, and hexenyl.

"Alkynyl" herein means a monovalent group having at least one triple bond (two adjacent SP carbon atoms). The alkynyl includes linear and branched alkynyls. The alkynyl is preferably $C_2$-$C_{10}$ alkynyl, and more preferably $C_2$-$C_6$ alkynyl, and specific examples include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

"Cycloalkyl" herein means a saturated or partially saturated cyclic monovalent aliphatic hydrocarbon group and includes a monocyclic ring, a bicyclo ring, and a spiro ring. The cycloalkyl is preferably $C_3$-$C_8$ cycloalkyl, and specific examples include cyclopropyl, cyclobutyl, cyclopentyl, cyclohexyl, cycloheptyl, cyclooctyl, bicyclo[2.2.1]heptyl, and spiro[3.3]heptyl.

"Aryl" herein means a monovalent aromatic hydrocarbon ring, and is preferably $C_6$-$C_{10}$ aryl. Specific examples of the aryl include phenyl and naphthyl (e.g., 1-naphthyl and 2-naphthyl).

"Heterocyclyl" herein means a non-aromatic cyclic monovalent group containing 1 to 5 hetero atoms in addition to carbon atoms. The heterocyclyl may have a double and/or triple bond within the ring, a carbon atom within the ring may be oxidized to form carbonyl, and heterocyclyl may be a monocyclic ring or a condensed ring. The number of atoms constituting the ring is preferably 4 to 10 (4- to 10-membered heterocyclyl), and more preferably 4 to 7 (4- to 7-membered heterocyclyl). Specific examples of the heterocyclyl include azetidinyl, oxiranyl, oxetanyl, azetidinyl, dihydrofuryl, tetrahydrofuryl, dihydropyranyl, tetrahydropyranyl, tetrahydropyridyl, tetrahydropyrimidyl, morpholinyl, thiomorpholinyl, pyrrolidinyl, piperidinyl, piperazinyl, pyrazolidinyl, imidazolinyl, imidazolidinyl, oxazolidinyl, isoxazolidinyl, thiazolidinyl, isothiazolidinyl, 1,2-thiazinane, thiadiazolidinyl, azetidinyl, oxazolidone, benzodioxanyl, benzoxazolyl, dioxolanyl, dioxanyl, tetrahydropyrrolo[1,2-c]imidazole, thietanyl, 3,6-diazabicyclo[3.1.1]heptanyl, 2,5-diazabicyclo[2.2.1]heptanyl, 3-oxa-8-azabicyclo[3.2.1]octanyl, sultam, and 2-oxaspiro[3.3]heptyl.

"Heteroaryl" herein means an aromatic cyclic monovalent group containing 1 to 5 heteroatoms in addition to carbon atoms. The ring may be a monocyclic ring, may be a condensed ring formed with another ring, or may be partially saturated. The number of atoms constituting the ring is preferably 5 to 10 (5- to 10-membered heteroaryl) and more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzoimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

"Alkoxy" herein means an oxy group to which the above-defined "alkyl" is bonded, and is preferably $C_1$-$C_6$ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

"Alkenyloxy" herein means an oxy group to which the above-defined "alkenyl" is bonded, and is preferably $C_2$-$C_6$ alkenyloxy. Specific examples of the alkenyloxy include vinyloxy, allyloxy, 1-propenyloxy, 2-propenyloxy, 1-butenyloxy, 2-butenyloxy (including cis and trans forms), 3-butenyloxy, pentenyloxy, and hexenyloxy.

"Cycloalkoxy" herein means an oxy group to which the above-defined "cycloalkyl" is bonded, and is preferably $C_3$-$C_8$ cycloalkoxy. Specific examples of the cycloalkoxy include cyclopropoxy, cyclobutoxy, and cyclopentyloxy.

"Aryloxy" herein means an oxy group to which the above-defined "aryl" is bonded, and is preferably $C_6$-$C_{10}$ aryloxy. Specific examples of the aryloxy include phenoxy, 1-naphthyloxy, and 2-naphthyloxy.

"Amino" herein means $-NH_2$ in a narrow sense and -NRR' in a broad sense, wherein R and R' are independently selected from hydrogen, alkyl, alkenyl, alkynyl, cycloalkyl, heterocyclyl, aryl, or heteroaryl, or R and R', together with the nitrogen atom to which they are attached, form a ring. The amino is preferably $-NH_2$, mono-$C_1$-$C_6$ alkylamino, di-$C_1$-$C_6$ alkylamino, 4- to 8-membered cyclic amino, or the like.

"Monoalkylamino" herein means a group corresponding to the above-defined "amino" wherein R is hydrogen and R' is the above-defined "alkyl", and is preferably mono-$C_1$-$C_6$ alkylamino. Specific examples of the monoalkylamino include methylamino, ethylamino, n-propylamino, i-propylamino, n-butylamino, s-butylamino, and t-butylamino.

"Dialkylamino" herein means a group corresponding to the above-defined "amino" wherein R and R' are independently the above-defined "alkyl", and is preferably di-$C_1$-$C_6$ alkylamino. Specific examples of the dialkylamino include dimethylamino and diethylamino.

"Cyclic amino" herein means a group corresponding to the above-defined "amino" wherein R and R', together with the nitrogen atom to which they are attached, form a ring, and is preferably 4- to 8-membered cyclic amino. Specific examples of the cyclic amino include 1-azetidyl, 1-pyrrolidyl, 1-piperidyl, 1-piperazyl, 4-morpholinyl, 3-oxazolidyl, 1,1-dioxidethiomorpholinyl-4-yl, and 3-oxa-8-azabicyclo[3.2.1]octan-8-yl.

"Hydroxyalkyl" herein means a group in which one or more hydrogens of the above-defined "alkyl" are replaced with hydroxyl groups, and is preferably $C_1$-$C_6$ hydroxyalkyl, and more preferably $C_2$ hydroxyalkyl. Specific examples of the hydroxyalkyl include hydroxymethyl, 1-hydroxyethyl, 2-hydroxyethyl, 2-hydroxy-2-methylpropyl, and 5-hydroxypentyl.

"Haloalkyl" herein means a group in which one or more hydrogens of the above-defined "alkyl" are replaced with halogen, and is preferably $C_1$-$C_6$ haloalkyl, and more preferably $C_1$-$C_6$ fluoroalkyl. Specific examples of the haloalkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

"Cyanoalkyl" herein means a group in which one or more hydrogens of the above-defined "alkyl" are replaced with cyano, and is preferably $C_1$-$C_6$ cyanoalkyl. Specific examples of the cyanoalkyl include cyanomethyl and 2-cyanoethyl.

"Aminoalkyl" herein means a group in which one or more hydrogens of the above-defined "alkyl" are replaced with the above-defined "amino", and is preferably $C_1$-$C_6$ aminoalkyl. Specific examples of the aminoalkyl include 1-pyridylmethyl, 2-(1-piperidyl)ethyl, 3-(1-piperidyl)propyl, and 4-aminobutyl.

"Carboxyalkyl" herein means a group in which one or more hydrogens of the above-defined "alkyl" are replaced with carboxy, and is preferably $C_2$-$C_6$ carboxyalkyl. Specific examples of the carboxyalkyl include carboxymethyl.

"Alkenyloxycarbonylalkyl" herein means a group in which one or more hydrogens of the above-defined "alkyl" are replaced with the above-defined "alkenyloxycarbonyl", and is preferably $C_2$-$C_6$ alkenyloxycarbonyl $C_1$-$C_6$ alkyl, and more preferably $C_2$-$C_6$ alkenyloxycarbonyl $C_1$-$C_2$ alkyl. Specific examples of the alkenyloxycarbonylalkyl include allyloxycarbonylmethyl and 2-(allyloxycarbonyl)ethyl.

"Alkoxyalkyl" herein means a group in which one of more hydrogens of the above-defined "alkyl" are replaced with the above-defined "alkoxy", and is preferably $C_1$-$C_6$ alkoxy Ci-$C_6$ alkyl, and more preferably $C_1$-$C_6$ alkoxy $C_1$-$C_2$ alkyl. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

"Cycloalkylalkyl" herein means a group in which one or more hydrogens of the above-defined "alkyl" are replaced with the above-defined "cycloalkyl", and is preferably $C_3$-$C_8$ cycloalkyl $C_1$-$C_6$ alkyl, and more preferably $C_3$-$C_6$ cycloalkyl $C_1$-$C_2$ alkyl. Specific examples of the cycloalkylalkyl include cyclopropylmethyl, cyclobutylmethyl, cyclopentylmethyl, and cyclohexylmethyl.

"Cycloalkoxylalkyl" herein means a group in which one or more hydrogens of the above-defined "alkyl" are replaced with the above-defined "cycloalkoxy", and is preferably $C_3$-$C_8$ cycloalkoxy $C_1$-$C_6$ alkyl, and more preferably $C_3$-$C_6$ cycloalkoxy $C_1$-$C_2$ alkyl. Specific examples of the cycloalkoxyalkyl include cyclopropoxymethyl and cyclobutoxymethyl.

"Heterocyclylalkyl" herein means a group in which one or more hydrogens of the above-defined "alkyl" are replaced with the above-defined "heterocyclyl", and is preferably 4- to 7-membered heterocyclyl $C_1$-$C_6$ alkyl, and more preferably 4- to 7-membered heterocyclyl $C_1$-$C_2$ alkyl. Specific examples of the heterocyclylalkyl include 2-(tetrahydro-2H-pyran-4-yl)ethyl and 2-(azetidin-3-yl)ethyl.

"Alkylsulfonylalkyl" herein means a group in which one or more hydrogens of the above-defined "alkyl" are replaced with the above-defined "alkylsulfonyl", and is preferably $C_1$-$C_6$ alkylsulfonyl $C_1$-$C_6$ alkyl, and more preferably $C_1$-$C_6$ alkylsulfonyl $C_1$-$C_2$ alkyl. Specific examples of the alkylsulfonylalkyl include methylsulfonylmethyl and 2-(methylsulfonyl)ethyl.

"Aminocarbonylalkyl" herein means a group in which one or more hydrogens of the above-defined "alkyl" are replaced with the above-defined "aminocarbonyl", and is preferably aminocarbonyl $C_1$-$C_6$ alkyl, and more preferably

aminocarbonyl $C_1$-$C_4$ alkyl. Specific examples of the aminocarbonylalkyl include methylaminocarbonylmethyl, dimethylaminocarbonylmethyl, t-butylaminocarbonylmethyl, 1-azetidinylcarbonylmethyl, 1-pyrrolidinylcarbonylmethyl, 1-piperidinylcarbonylmethyl, 4-morpholinylcarbonylmethyl, 2-(methylaminocarbonyl)ethyl,2-(dimethylaminocarbonyl)ethyl, 2-(1-azetidinylcarbonyl)ethyl, 2-(1-pyrrolidinylcarbonyl)ethyl, 2-(4-morpholinylcarbonyl)ethyl, 3-(dimethylaminocarbonyl)propyl, and 4-(dimethylaminocarbonyl)butyl.

"Aryloxyalkyl" herein means a group in which one or more hydrogens of the above-defined "alkyl" are replaced with the above-defined "aryloxy", and is preferably $C_6$-$C_{10}$ aryloxy $C_1$-$C_6$ alkyl, and more preferably $C_6$-$C_{10}$ aryloxy $C_1$-$C_2$ alkyl. Specific examples of the aryloxyalkyl include phenoxymethyl and 2-phenoxyethyl.

"Aralkyl (arylalkyl)" herein means a group in which one or more hydrogen atoms of the above-defined "alkyl" are replaced with the above-defined "aryl", and is preferably $C_7$-$C_{14}$ aralkyl, and more preferably $C_7$-$C_{10}$ aralkyl. Specific examples of the aralkyl include benzyl, phenethyl, and 3-phenylpropyl.

"Heteroarylalkyl" herein means a group in which one or more hydrogen atoms of the above-defined "alkyl" are replaced with the above-defined "heteroaryl", and is preferably 5- to 10-membered heteroaryl $C_1$-$C_6$ alkyl, and more preferably 5- to 10-membered heteroaryl $C_1$-$C_2$ alkyl. Specific examples of the heteroarylalkyl include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl)ethyl, and 2-(5-benzofuranyl)ethyl.

The "non-aromatic heterocyclic ring" herein means a non-aromatic heterocyclic ring in which atoms constituting the ring include 1 to 5 heteroatoms. The non-aromatic heterocyclic ring may have a double and/or triple bond within the ring, and a carbon atom within the ring may be oxidized to form carbonyl. The non-aromatic heterocyclic ring may be a monocyclic ring, a condensed ring, or a spiro ring. The number of atoms constituting the ring is not limited, and is preferably 5 to 6 (a 5- to 6-membered non-aromatic heterocyclic ring). Specific examples of the non-aromatic heterocyclic ring include azetidine, oxetane, thietane, pyrrolidine, tetrahydrofuran, tetrahydrothiophene, imidazolidine, pyrazolidine, oxazolidine, isoxazolidine, thiazolidine, isothiazolidine, dioxolane, dithiolane, piperidine, tetrahydropyran, thiane, piperazine, morpholine, thiomorpholine, dioxane, dithiane, azepane, oxepane, thiepane, and diazepan.

"Peptide chain" herein refers to a peptide chain in which 1, 2, 3, 4, or more natural amino acids and/or non-natural amino acids are connected by an amide bond and/or an ester bond.

"Optionally substituted" herein means that a group may be substituted with any substituent.

"One or more" herein means one or two or more. When "one or more" is used in a context relating to the substituent of a group, the phrase means a number encompassing one to the maximum number of substituents permitted by that group. Specific examples of "one or more" include 1, 2, 3, 4, 5, 6, 7, 8, 9, 10, and/or a greater number.

The "C-terminal-activated substance" herein includes, not only a carboxyl group-activated compound used in a peptide synthesis reaction (e.g., an activated ester that leads to the production of a peptide compound of interest), but also a compound resulting from transformation of the carboxyl group-activated compound into, for example, azlactone, NCA (N-carboxyanhydride), or the like during the reaction which has an activated state and thus is capable of reacting with amine (e.g., an amine component) to give a peptide compound of interest. The C-terminal-activated substance that is a compound having an activated carboxyl group used in a peptide synthesis reaction may be an active ester, a mixed acid anhydride, and acylisourea, synthesized using a peptide condensing agent as described in Chem. Rev., 2011, 111, 6557 or Organic Process Research & Development, 2016, 20(2), 140., but it is not limited thereto and includes any compound activated so as to be capable of reacting with amine.

The "active ester" herein is a compound which contains a carbonyl group that reacts with an amino group to form an amide bond, in which the carbony group is bonded by, for example, OBt, OAt, OSu, or OPfp, and with which a reaction with amine is promoted.

The term "amino acid" as used herein includes natural and unnatural amino acids. The term "natural amino acid" as used herein refers to Gly, Ala, Ser, Thr, Val, Leu, Ile, Phe, Tyr, Trp, His, Glu, Asp, Gln, Asn, Cys, Met, Lys, Arg, or Pro. Examples of the unnatural amino acid include, but are not particularly limited to, $\beta$-amino acids, $\gamma$-amino acids, D-amino acids, N-substituted amino acids, $\alpha$, $\alpha$-disubstituted amino acids, amino acids having side chains that are different from those of natural amino acids, and hydroxycarboxylic acids. Amino acids herein may have any conformation. There is no particular limitation on the selection of amino acid side chain, but in addition to a hydrogen atom, it can be freely selected from, for example, an alkyl group, an alkenyl group, an alkynyl group, an aryl group, a heteroaryl group, an aralkyl group, and a cycloalkyl group. One or two non-adjacent methylene groups in such a group are optionally substituted with an oxygen atom, a carbonyl group (-CO-), or a sulfonyl group ($-SO_2-$), a phosphoryl group, or a phosphonyl group. Each group may have a substituent, and there are no limitations on the substituent. For example, one or more substituents may be freely and independently selected from any substituents including a halogen atom, an O atom, an S atom, an N atom, a B atom, an Si atom, or a P atom. Examples include an optionally substituted alkyl group, alkenyl group, alkynyl group, aryl group, heteroaryl group, aralkyl group, and cycloalkyl group. In a non-limiting embodiment, amino acids herein may be compounds having a carboxy group and an amino group in the same molecule.

The main chain amino group of an amino acid may be unsubstituted (an $NH_2$ group) or substituted (i.e., an -NHR group, where R represents alkyl, alkenyl, alkynyl, aryl, heteroaryl, aralkyl, or cycloalkyl which may have a substituent, one or two non-adjacent methylene groups in such a group may be substituted with an oxygen atom, a carbonyl group (-CO-), or a sulfonyl group ($-SO_2-$), and the carbon chain bonded to the N atom and the carbon atom at the position $\alpha$ may form a ring, as in proline. Such amino acids in which the main chain amino group is substituted are herein called "N-substituted amino acids." Preferred examples of the "N-substituted amino acids" as used herein include, but are not limited to, N-alkylamino acids, N-$C_1$-$C_6$ alkylamino acids, N-$C_1$-$C_4$ alkylamino acids, and N-methylamino acids.

"Amino acids" as used herein which constitute a peptide compound include all isotopes corresponding to each amino acid. The isotope of the "amino acid" refers to one having at least one atom replaced with an atom of the same atomic number (number of protons) and different mass number (total number of protons and neutrons). Examples of isotopes contained in the "amino acid" constituting the peptide compounds of the present invention include a hydrogen atom, a carbon atom, a nitrogen atom, an oxygen atom, a phosphorus atom, a sulfur atom, a fluorine atom, and a chlorine atom, which respectively include $^2H$ and $^3H$; $^{13}C$ and $^{14}C$; $^{15}N$; $^{17}O$ and $^{18}O$; $^{31}P$ and $^{32}P$; $^{35}S$; $^{18}F$; and $^{36}Cl$.

Substituents containing a halogen atom as used herein include a halogen-substituted alkyl, cycloalkyl, alkenyl, alkynyl, aryl, heteroaryl , or aralkyl. More specific examples include fluoroalkyl, difluoroalkyl, and trifluoroalkyl.

Substituents containing an O atom include groups such as hydroxy (-OH), oxy (-OR), carbonyl (-C(=O)-R), carboxy ($-CO_2H$), oxycarbonyl (-C(=O)-OR), carbonyloxy (-O-C(=O)-R), thiocarbonyl (-C(=O)-SR), carbonylthio (-S-C(=O)-R), aminocarbonyl (-C(=O)-NHR), carbonylamino (-NH-C(=O)-R), oxycarbonylamino (-NH-C(=O)-OR), sulfonylamino ($-NH-SO_2-R$), aminosulfonyl ($-SO_2-NHR$), sulfamoylamino ($-NH-SO_2-NHR$), thiocarboxyl (-C(=O)-SH), and carboxylcarbonyl ($-C(=O)-CO_2H$).

Examples of oxy (-OR) include alkoxy, cycloalkoxy, alkenyloxy, alkynyloxy, aryloxy, heteroaryloxy, and aralkyloxy. The alkoxy is preferably $C_1$-$C_4$ alkoxy and $C_1$-$C_2$ alkoxy, and particularly preferably methoxy or ethoxy.

Examples of carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C(=O)-NHR) include alkylaminocarbonyl (examples of which include $C_1$-$C_6$ or $C_1$-$C_4$ alkylaminocarbonyl, in particular, ethylaminocarbonyl and methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. Additional examples include groups in which the H atom bonded to the N atom in -C(=O)-NHR is further replaced with alkyl, cycloalkyl, alkenyl, alkynyl, aryl, heteroaryl, or aralkyl.

Examples of carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples include groups in which the H atom bonded to the N atom in -NH-C(=O)-R is further replaced with alkyl, cycloalkyl, alkenyl, alkynyl, aryl, heteroaryl, or aralkyl.

Examples of oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino.

Additional examples include groups in which the H atom bonded to the N atom in -NH-C(=O)-OR is further replaced with alkyl, cycloalkyl, alkenyl, alkynyl, aryl, heteroaryl, or aralkyl.

Examples of sulfonylamino ($-NH-SO_2-R$) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples include groups in which the H atom attached to the N atom in $-NH-SO_2-R$ is further replaced with alkyl, cycloalkyl, alkenyl, alkynyl, aryl, heteroaryl, or aralkyl.

Examples of aminosulfonyl ($-SO_2-NHR$) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples include groups in which the H atom attached to the N atom in $-SO_2-NHR$ is further replaced with alkyl, cycloalkyl, alkenyl, alkynyl, aryl, heteroaryl, or aralkyl.

Examples of sulfamoylamino ($-NH-SO_2-NHR$) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. The two H atoms bonded to the N atoms in $-NH-SO_2-NHR$ may be further replaced with substituents independently selected from the group consisting of alkyl, cycloalkyl, alkenyl, alkynyl, aryl, heteroaryl, and aralkyl, and these two substituents may form a ring.

Substituents containing an S atom include groups such as thiol (-SH), thio (-S-R), sulfinyl (-S(=O)-R), sulfonyl

($-SO_2-R$), and sulfo ($-SO_3H$).

Examples of thio (-S-R) include alkylthio, cycloalkylthio, alkenylthio, alkynylthio, arylthio, heteroarylthio, and aralkylthio.

Examples of sulfonyl ($-SO_2-R$) include alkylsulfonyl, cycloalkylsulfonyl, alkenylsulfonyl, alkynylsulfonyl, arylsulfonyl, heteroarylsulfonyl, and aralkylsulfonyl.

Substituents containing an N atom include groups such as azido ($-N_3$, also called "azido group"), cyano (-CN), primary amino ($-NH_2$), secondary amino (-NH-R; also called monosubstituted amino), tertiary amino (-NR(R'); also called disubstituted amino), amidino ($-C(=NH)-NH_2$), substituted amidino (-C(=NR)-NR'R"), guanidino ($-NH-C(=NH)-NH_2$), substituted guanidino (-NR-C(=NR‴)-NR'R"), aminocarbonylamino (-NR-CO-NR'R"), pyridyl, piperidino, morpholino, and azetidinyl.

Examples of secondary amino (-NH-R; monosubstituted amino) include alkylamino, cycloalkylamino, alkenylamino, alkynylamino, arylamino, heteroarylamino, and aralkylamino.

Examples of tertiary amino (-NR(R'); disubstituted amino) include amino groups having any two substituents each independently selected from alkyl, cycloalkyl, alkenyl, alkynyl, aryl, heteroaryl, and aralkyl, such as alkyl(aralkyl)amino, where any two such substituents may form a ring. Specific examples include dialkylamino, in particular, $C_1$-$C_6$ dialkylamino, $C_1$-$C_4$ dialkylamino, dimethylamino, and diethylamino. The term "$C_p$-$C_q$ dialkylamino group" as used herein refers to an amino group substituted with two $C_p$-$C_q$ alkyl groups, where the two $C_p$-$C_q$ alkyl groups may be the same or different.

Examples of substituted amidino (-C(=NR)-NR'R") include groups in which three substituents R, R', and R" on the N atom are each independently selected from alkyl, cycloalkyl, alkenyl, alkynyl, aryl, heteroaryl, and aralkyl, such as alkyl(aralkyl)(aryl)amidino.

Examples of substituted guanidino (-NR-C(=NR‴)-NR'R") include groups in which R, R', R", and R‴ are each independently selected from alkyl, cycloalkyl, alkenyl, alkynyl, aryl, heteroaryl, and aralkyl, or groups in which these substituents form a ring.

Examples of aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, or groups in which these substituents form a ring.

Herein, an "amino acid residue" constituting the peptide compound may be simply referred to as an "amino acid".

(Methods of producing peptide compounds)

In an embodiment, the present invention relates to a method of producing a peptide compound, and the method comprises the following steps:

step A: a step of obtaining a reaction mixture comprising a peptide compound obtained by condensing a C-terminal-activated substance of an acid component with an amine component in a solvent; and

step B: a step of mixing the reaction mixture, a tertiary amine, and water or an aqueous solution to remove the C-terminal-activated substance.

Step A is the step of reacting an acid component and an amine component in a solvent using a condensing agent to obtain a reaction mixture containing a peptide compound. Without wishing to be bound by a particular theory, in step A, an acid component and a condensing agent react to form a C-terminal-activated substance of the acid component, then an amine component nucleophilically attacks the C-terminal-activated substance, thereby the reaction proceeds, and a peptide compound is produced.

The acid component used may be an amino acid having an amino group protected with a protecting group, or a peptide having an N-terminal amino group protected with a protecting group. Herein, the amino acid used as the acid component may be referred to as the "first amino acid", and the peptide used as the acid component may be referred to as the "first peptide".

The first amino acid is not particularly limited, and any natural amino acid or non-natural amino acid can be used. The first peptide is also not particularly limited, and a peptide in which any two or more natural amino acids and/or non-natural amino acids are connected can be used.

The first amino acid preferably contains one or more carbon atoms in its side chain. Specific examples of such an amino acid include those having optionally substituted alkyl, optionally substituted alkenyl, optionally substituted alkynyl, optionally substituted cycloalkyl, optionally substituted alkoxyalkyl, optionally substituted cycloalkylalkyl, optionally substituted aralkyl, optionally substituted heteroarylalkyl, or the like in the side chain. When the side chain has a functional group such as an amino group, a carboxyl group, or a hydroxyl group that may affect the reaction for forming a peptide bond, such a group is preferably protected with a suitable protecting group. Without wishing to be bound by a particular theory, when an amino acid has a bulky group in its side chain, hydrolysis of the residual C-terminal-activated

substance of the amino acid may not sufficiently proceed by a conventional method due to the steric hindrance by the bulky group. Even in such a case, the residual C-terminal-activated substance can be promptly and efficiently hydrolyzed by using the method of the present invention.

The side chain of the C-terminal amino acid contained in the first peptide may be the same as the side chain of the first amino acid.

The protecting group for the amino group of the first amino acid and the protecting group for the N-terminal amino group of the first peptide may be an amino group protecting group commonly used in the art. Specific examples of such a protecting group include Cbz, Boc, Teoc, Fmoc, Tfa, Alloc, Nosyl, dinitronosyl, t-Bu, trityl, and cumyl.

In an embodiment, the acid component is preferably used at least in an amount equivalent to the amine component, and preferably in an excessive amount relative to the amine component. Specifically, the acid component can be used in an amount of, for example, 1 to 1.1 equivalents, 1 to 1.2 equivalents, 1 to 1.3 equivalents, 1 to 1.4 equivalents, 1 to 1.5 equivalents, 1 to 2.0 equivalents, and 1 to 3.0 equivalents relative to the amine component.

In an embodiment, the C-terminal-activated substance of the acid component in the present invention can be formed by allowing the acid component to react with a condensing agent in a solvent. The condensing agent is not particularly limited as long as it can introduce a group having leaving ability in the hydroxy moiety of the carboxyl group of the acid component to enhance the electrophilic properties of carbonyl carbon of the acid component, and specific examples include T3P, HATU, BEP, carbodiimides (such as DIC and EDC), a combination of carbodiimide and an additive (such as oxyma, HOOBt, or HOBt), DMT-MM, and CDI.

The step of condensing the C-terminal-activated substance with the amine component to obtain a peptide compound (step A) can be carried out by stirring a reaction mixture for 1 minute to 48 hours and preferably 15 minutes to 4 hours at a temperature of -20°C to a temperature in the vicinity of the boiling point of the solvent, and preferably 0°C to 60°C.

In step A, the condensation reaction of the acid component and the amine component can proceed quantitatively.

The amine component used may be an amino acid having a carboxyl group protected with a protecting group, or a peptide having a C-terminal carboxyl group protected with a protecting group. Herein, the amino acid used as the amine component may be referred to as the "second amino acid", and the peptide used as the amine component may be referred to as the "second peptide".

The second amino acid is not particularly limited, and any natural amino acid or any non-natural amino acid can be used. The second peptide is also not particularly limited, and a peptide in which any two or more natural amino acids and/or non-natural amino acids are connected can be used.

The protecting group for the carboxyl group of the second amino acid and the protecting group for the C-terminal carboxyl group of the second peptide may be a carboxyl group protecting group commonly used in the art. Specific examples of such a protecting group include methyl, allyl, t-butyl, trityl, cumyl, benzyl, methoxytrityl, and 1-piperidinyl.

In an embodiment, the solvent in the present invention may be any solvent as long as the condensation reaction proceeds so that a peptide compound can be obtained. Specific examples of such a solvent include toluene, acetonitrile, tetrahydrofuran, 2-methyltetrahydrofuran, isopropyl acetate, ethyl acetate, methyl tert-butyl ether, cyclopentyl methyl ether, N,N-dimethylformamide, and a solvent obtained by mixing two or more solvents selected therefrom.

In an embodiment, the "peptide compound" in the present invention obtained by condensing the C-terminal-activated substance of the acid component with the amine component includes a linear or cyclic peptide compound in which two or more amino acids are connected. The cyclic peptide compound is synonymous with "a peptide compound having a cyclic moiety".

The "linear peptide compound" in the present invention is formed by natural amino acids and/or non-natural amino acids connected by an amide bond or an ester bond, and is not particularly limited as long as it is a compound having no cyclic moiety. The total number of natural amino acids or non-natural amino acids constituting the linear peptide compound may be 1, 2, 3, 4, 5, 6, 7, 8, 9, 10, 11, 12, 13, 14, 15, 20, 25, or 30, and preferable ranges are 6 to 20, 7 to 19, 7 to 18, 7 to 17, 7 to 16, 7 to 15, 8 to 14, and 9 to 13.

The "cyclic peptide compound" in the present invention is formed by natural amino acids and/or non-natural amino acids connected by an amide bond or an ester bond, and is not particularly limited as long as it is a compound having a cyclic moiety. The cyclic peptide compound may have one or more linear moieties. The total number of natural amino acids or non-natural amino acids constituting the cyclic peptide compound may be 1, 2, 3, 4, 5, 6, 7, 8, 9, 10, 11, 12, 13, 14, 15, 20, 25, or 30, and preferable ranges are 6 to 20, 7 to 19, 7 to 18, 7 to 17, 7 to 16, 7 to 15, 8 to 14, and 9 to 13.

The number of amino acids constituting the cyclic moiety of the cyclic peptide compound is not limited, and is, for example, 4 or more, 6 or more, 7 or more, 8 or more, 9 or more, 10 or more, 11 or more, 12 or more, 20 or less, 18 or less, 16 or less, 15 or less, 14 or less, 13 or less, 12 or less, 11 or less, 7, 8, 9, 10, 11, 12, 13, 14, 15, and 16. The number of amino acids constituting the cyclic moiety is preferably 5 to 15, more preferably 5 to 14, 7 to 14, or 8 to 14, even more preferably 8 to 13, 9 to 13, 8 to 12, 8 to 11, or 9 to 12, and particularly preferably 9 to 11.

The number of amino acids in the linear moiety of the cyclic peptide is preferably 0 to 8, more preferably 0 to 5, and even more preferably 0 to 3.

The peptide compound can contain 1 or more, 2 or more, 3 or more, 4 or more, 5 or more, or 6 or more non-natural amino acids. Further, the peptide compound can contain 20 or less, 15 or less, 14 or less, 13 or less, 12 or less, 10 or less, and 9 or less non-natural amino acids. When the peptide compound contains non-natural amino acids, the proportion of the number of non-natural amino acids is, for example, 30% or more, 40% or more, 50% or more, 60% or more, 70% or more, or 80% or more of the total number of amino acids constituting the peptide compound.

The peptide compound can be a linear or cyclic peptide that, in addition to or instead of satisfying the above-described requirement concerning the total number of natural amino acids and non-natural amino acids, contains at least two N-substituted amino acids (preferably 2, 3, 4, 5, 6, 7, 8, 9, 10, 11, 12, 13, 14, 15, 20, 25, 30, particularly preferably 5, 6, or 7, and preferable ranges being 2 to 30, 3 to 30, 6 to 20, 7 to 19, 7 to 18, 7 to 17, 7 to 16, 7 to 15, 8 to 14, and 9 to 13), and contains at least one amino acid that is not N-substituted. Examples of "N-substitution" include, but are not limited to, substitution of a hydrogen atom bonded to an N atom with a methyl group, an ethyl group, a propyl group, a butyl group, or a hexyl group. Preferable examples of the N-substituted amino acid include amino acids in which the amino group contained in a natural amino acid is N-methylated, N-ethylated, N-propylated, N-butylated, or N-pentylated, and such amino acids are referred to as N-methyl amino acid, N-ethyl amino acid, N-propyl amino acid, N-butyl amino acid, and N-pentyl amino acid. Conversion of an N-unsubstituted amino acid to an N-substituted amino acid is referred to as N-substitution, and may be referred to as N-alkylation, N-methylation, or N-ethylation. The proportion of the number of N-substituted amino acids contained in the peptide compound in the present invention is, for example, 30% or more, 40% or more, 50% or more, 60% or more, 70% or more, or 80% or more of the total number of amino acids constituting the peptide compound.

The peptide compound may include a salt of the compound or a solvate of the compound or the salt.

The term "side chain" herein is used in the context referring to a side chain of an amino acid, a side chain of a cyclic moiety of a cyclic peptide compound, or the like, and means a portion not included in each main-chain structure.

The "number of amino acids" herein is the number of amino acid residues constituting the peptide compound, and means the number of amino acid units generated when cleaving amide bonds, ester bonds, and bonds of cyclic moieties that connect amino acids.

Step B is the step of removing an unreacted C-terminal-activated substance contained in the reaction mixture obtained in step A. In an embodiment, removal of the unreacted C-terminal-activated substance is carried out by allowing the unreacted C-terminal-activated substance to be acted on by a tertiary amine. Herein, the unreacted C-terminal-activated substance, or specifically, for example, the C-terminal-activated substance remaining in the reaction mixture without reacting with the amine component during the course of condensation, may be referred to as the "residual C-terminal-activated substance". In step B, the reaction mixture obtained in step A, a tertiary amine, and water or an aqueous solution are mixed. When an excessive amount of the acid component relative to the amine component is used in step A, or when the condensation reaction does not sufficiently proceed in step A, the C-terminal-activated substance of the acid component left unreacted with the amine component remains as an impurity in the reaction solvent. This residual C-terminal-activated substance, when present in the system without being sufficiently decomposed, adversely affects the subsequent step of deprotecting a peptide compound and reaction of further elongating a peptide chain, and it is thus important to reliably remove the residual C-terminal-activated substance. Concerning conventional liquid phase synthesis procedures, for example, a method of hydrolyzing a residual active ester using an alkaline aqueous solution is known, but the present inventors confirmed that the decomposition of the residual C-terminal-activated substances may be insufficient when the residual C-terminal-activated substance is especially of an amino acid having a bulky functional group in its side chain or when the leaving ability of the leaving group of the residual C-terminal-activated substance is not so high as readily allowing a reaction with water to occur. Corresponding to this, this problem can be solved by using the method of the present invention in which the reaction mixture containing an unreacted C-terminal-activated substance is mixed with a tertiary amine and water or an aqueous solution, or the residual C-terminal-activated substance is contacted with a tertiary amine, to thereby hydrolyze the C-terminal-activated substance.

A tertiary amine that is nucleophilic to the residual C-terminal-activated substance of the acid component is preferably used. Such a tertiary amine is preferably an amine having small steric hindrance in the vicinity of nitrogen. Examples of such a tertiary amine include tertiary amines represented by formula (A), (B), or (C) below:

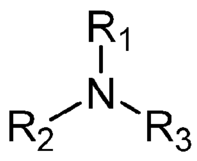
(A)

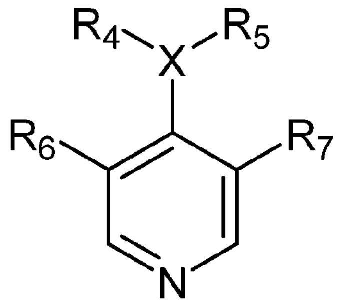
(B)

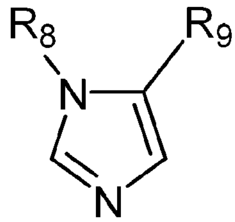
(C)

In an embodiment, concerning $R_1$ to $R_3$ in formula (A), $R_1$ and $R_2$, together with the nitrogen atom to which they are attached, form a 5- to 6-membered non-aromatic heterocyclic ring, and $R_3$ is $C_1$-$C_2$ alkyl (i.e., methyl or ethyl) or $C_2$ hydroxyalkyl. The 5- to 6-membered non-aromatic heterocyclic ring is preferably pyrrolidine, piperidine, or morpholine, and $C_2$ hydroxyalkyl is preferably 2-hydroxyethyl.

In another embodiment, in formula (A), $R_1$ to $R_3$ are each independently $C_1$-$C_2$ alkyl or $C_2$ hydroxyalkyl. $C_2$ hydroxyalkyl is preferably 2-hydroxyethyl.

In a preferable tertiary amine represented by formula (A), $R_1$ to $R_3$ are each independently $C_1$-$C_2$ alkyl.

Specific examples of the tertiary amine represented by formula (A) include trimethylamine, N,N-dimethylethylamine, N,N-diethylmethylamine, triethylamine, and triethanolamine. Among these, trimethylamine is particularly preferable.

In an embodiment, in formula (B), X is N or O. When X is N, $R_4$ and $R_5$ are each independently $C_1$-$C_2$ alkyl or $C_2$ hydroxyalkyl, or, together with the nitrogen atom to which they are attached, form a 5- to 6-membered non-aromatic heterocyclic ring. When X is O, $R_4$ is Ci-$C_2$ alkyl or $C_2$ hydroxyalkyl, and $R_5$ does not exist. The 5- to 6-membered non-aromatic heterocyclic ring is preferably pyrrolidine, piperidine, or morpholine, and $C_2$ hydroxyalkyl is preferably 2-hydroxyethyl. In formula (B), $R_6$ and $R_7$ are each independently H, $C_1$-$C_2$ alkyl, or methoxy.

In a preferable tertiary amine represented by formula (B), X is N, $R_4$ and $R_5$ are each independently $C_1$-$C_2$ alkyl, and $R_6$ and $R_7$ are H.

Specific examples of the tertiary amine represented by formula (B) include DMAP, 4-piperidinopyridine, and 4-morpholinopyridine. Among these, DMAP is particularly preferable.

In an embodiment, in formula (C), $R_5$ and $R_9$ are each independently H, $C_1$-$C_2$ alkyl, or $C_2$ hydroxyalkyl, or, together with the nitrogen atom to which $R_8$ is attached and the carbon atom to which $R_9$ is attached, form a 5- to 6-membered non-aromatic heterocyclic ring. The 5- to 6-membered non-aromatic heterocyclic ring is preferably pyrrolidine, piperidine, or morpholine, and $C_2$ hydroxyalkyl is preferably 2-hydroxyethyl.

In a preferable tertiary amine represented by formula (C), $R_5$ and $R_9$ are each independently H or $C_1$-$C_2$ alkyl, and more preferably $R_5$ is $C_1$-$C_2$ alkyl, and $R_9$ is H.

Specific examples of the tertiary amine represented by formula (C) include NMI, imidazol-1-ethanol, and 5,6,7,8-tetrahydroimidazo[1,5-α]pyridine. Among these, NMI is particularly preferable.

Without wishing to be bound by a particular theory, the tertiary amine of the present invention can promote hydrolysis of the residual C-terminal-activated substance by nucleophilically attacking the residual C-terminal-activated substance. A tertiary amine such as DIPEA has a bulky substituent, is thus poorly nucleophilic, and is undesirable. A hydrolyzed of the residual C-terminal-activated substance can be transferred to an aqueous layer and removed, and thus the produced peptide compound can be subjected to the next condensation reaction without undergoing a separate purification step such as column purification. Using the method of the present invention, the residual C-terminal-activated substance can be removed promptly (e.g., within 5 minutes) and efficiently by a hydrolysis treatment performed a small number of times (e.g., only once), and in an embodiment, 90%, 91%, 92%, 93%, 94%, 95%, 96%, 97%, 98%, or 99% or more of the residual C-terminal-activated substance can be removed. In other words, the residual rate of the C-terminal-activated substance can be 10% or less, 9% or less, 8% or less, 7% or less, 6% or less, 5% or less, 4% or less, 3% or less, 2% or less, or 1% or less by the present invention.

The tertiary amine may be used in a catalytic amount or in an amount equal to or greater than the stoichiometric amount relative to the amine component. Specifically, for example, 0.1 equivalents to 10 equivalents of tertiary amine relative to the amine component can be added to the reaction mixture, and 0.5 equivalents to 3 equivalents of tertiary amine is preferably added.

When allowing the tertiary amine to act on the residual C-terminal-activated substance, the reaction mixture can be stirred at a temperature of -20°C to the vicinity of the boiling point of the solvent, and preferably at a temperature of 25°C to 60°C, for 1 minute to 48 hours, preferably 2 hours or less, such as 2 minutes to 2 hours, 5 minutes to 60

minutes, 5 minutes to 50 minutes, or 5 minutes to 30 minutes.

In an embodiment, in the step of treating the residual C-terminal-activated substance with a tertiary amine, water or an aqueous solution can be added, and an alkaline aqueous solution can be preferably used as the aqueous solution. Such an alkaline aqueous solution is not particularly limited, and specific examples include an aqueous potassium carbonate solution, an aqueous lithium hydroxide solution, an aqueous sodium carbonate solution, sodium hydroxide, an aqueous potassium hydroxide solution, an aqueous sodium hydroxide solution, and an aqueous cesium carbonate solution. Among these, an aqueous potassium carbonate solution or an aqueous sodium carbonate solution, which has mild basicity, is preferable.

In an embodiment, the present invention further comprises, after allowing the tertiary amine to act on the residual C-terminal-activated substance, the step of separating the reaction mixture into an organic layer and an aqueous layer to obtain the organic layer, and then washing the organic layer. One example includes washing the organic layer with an acidic aqueous solution and a basic aqueous solution. In an embodiment, after this step, the residual amount of the residual C-terminal-activated substance can be 1.0% or less, 0.5% or less, and preferably 0.1% or less.

In an embodiment, the present invention further comprises the step of removing the N-terminal protecting group of the peptide compound (step C). Removal of a protecting group can be carried out by an ordinary method described in, for example, Greene's, "Protective Groups in Organic Synthesis" (5th edition, John Wiley & Sons 2014). In a conventional method a deprotection reaction may not sufficiently proceed due to the residual C-terminal-activated substance, whereas in the method of the present invention a deprotected product of the produced peptide compound can be obtained in high yield.

In an embodiment, the present invention comprises repeating step A and step B multiple times. Also, in an embodiment, the present invention comprises repeating step A, step B, and step C multiple times. Through such repetition, the peptide chain is elongated, and the peptide compound can be obtained.

In an embodiment, the present invention relates to a method of promoting hydrolysis of a residual C-terminal-activated substance, comprising the step of adding a tertiary amine and water or an aqueous solution to a solution comprising the residual C-terminal-activated substance to allow the tertiary amine to act on the C-terminal-activated substance. In this embodiment, the above-described residual C-terminal-activated substance and/or tertiary amine can be used. When an aqueous solution is added to a solution containing a residual C-terminal-activated substance, the aqueous solution is preferably the alkaline water described above.

In an embodiment, the present invention relates to a method of removing a hydrolyed product of a residual C-terminal-activated substrate, comprising the step of aqueously washing a solution comprising the hydrolyzed product. In this embodiment, aqueous washing can be carried out as washing with water or other alkaline aqueous solution. The alkaline aqueous solution is not particularly limited, and is preferably an aqueous potassium carbonate solution or an aqueous sodium carbonate solution. In another embodiment, when the base used forms a salt with a hydrolyzed product, and it makes the hydrolyzed product less likely to migrate to the aqueous layer, it is also possible to remove the base by washing with an acidic aqueous solution, and then wash the hydrolyzed product with an alkaline aqueous solution. The acidic aqueous solution is not particularly limited, and is preferably an aqueous potassium hydrogen sulfate solution or an aqueous sodium hydrogen sulfate solution. The alkaline aqueous solution is preferably an aqueous potassium carbonate solution or an aqueous sodium carbonate solution.

All prior-art documents cited herein are incorporated herein by reference.

[Example]

The present invention is further illustrated by way of the following Examples, but the present invention is not limited to the following Examples.

The purity of a peptide compound (a peptide synthesis substance of interest) and the residual amount of a C-terminal-activated substance were measured using an LCMS equipped with QDA and PDA detectors (column: Ascentis Express C18, 5 cm × 4.6 mm, 2.7 μm, mobile phase: 0.5% aqueous trifluoroacetic acid solution/0.5% trifluoroacetic acid acetonitrile solution = 95/5 to 0/100, 1.0 mL/min, detector: UV 210 nm).

The residual amount of a C-terminal-activated substance was evaluated after converting the residual C-terminal-activated substance to propylamide because the residual C-terminal-activated substance may possibly be hydrolyzed under analytical conditions (LCMS).

The purity of a peptide compound (a peptide synthesis substance of interest) was indicated as the peak area percent of LCMS. The residual rate of a C-terminal-activated substance and the relative value of the residual amount of a C-terminal-activated substance were calculated by the formulae provided in each Example. The total peak area was corrected by subtracting the area values of a blank peak and a solvent peak.

In the tables, nd means not detected.

(Example 1) Effect of added amine in hydrolysis of residual C-terminal-activated substance (Preparation of mixed acid anhydride)

463 mg (1.7 mmol) of Cbz-Ile-OH and 31 mg of pentamethylbenzene (internal standard substance: 0.21 mmol) were dissolved in 3.0 mL of 2-methyltetrahydrofuran. At room temperature, 1.1 mL (6.2 mmol) of diisopropylethylamine and 1.9 mL (3.2 mmol) of a 50% T3P/THF solution were added, and the mixture was stirred at 40°C for 1 hour to prepare a mixed acid anhydride (C-terminal-activated substance) solution. 5 μL of the prepared mixed acid anhydride solution was isolated, reacted with 100 μL (1.2 mmol) of normal propylamine, and then diluted with 0.9 mL of methanol, and the reaction conversion rate to the mixed acid anhydride was determined from the peak area of LC/MS (conversion rate: 97%). Cbz-Ile-NHPr/MS (ESI): m/z 307.1 [M+H]+.

$$\text{Conversion rate (\%)} = \{\text{Cbz-Ile-NHPr (area\%)} / [\text{Cbz-Ile-OH (area\%)} + \text{Cbz-Ile-NHPr (area\%)}]\} \times 100$$

(Hydrolysis treatment - no amine added)

1.0 mL was isolated from the entirety (6 mL) of the prepared mixed acid anhydride solution, then 0.5 mL of alkaline water (a 5% aqueous lithium hydroxide solution, a 5% aqueous sodium carbonate solution, a 5% aqueous potassium carbonate solution, a 5% aqueous potassium hydroxide solution, or a 5% aqueous cesium carbonate solution) was added, and the mixture was stirred (1200 rpm) with a stir bar at 25°C. After stirring was terminated, the mixture was left to stand still to separate the organic layer and the aqueous layer. 5 μL of the organic layer was isolated and added to 100 μL (1.2 mmol) of normal propylamine to convert the residual C-terminal-activated substance to propylamide, and then the mixture was diluted with 0.9 mL of methanol. This solution was subjected to LC/MS analysis, and the peak area ratio [propylamide: pentamethylbenzene (internal standard substance)] was calculated.

(Hydrolysis treatment - amine added)

1.0 mL was isolated from the entirety (6 mL) of the prepared mixed acid anhydride solution, then an amine additive (0.19 mmol) and 0.5 mL of a 5% aqueous potassium carbonate solution were added, and the mixture was stirred (1200 rpm) with a stir bar at 25°C. Stirring was terminated, and the mixture was left to stand still to separate the organic layer and the aqueous layer. 5 μL of the organic layer was isolated and added to 100 μL (1.2 mmol) of normal propylamine to convert the residual C-terminal-activated substance to propylamide, and then the mixture was diluted with 0.9 mL of methanol. This solution was subjected to LC/MS analysis, and the peak area ratio [propylamide : pentamethylbenzene (internal standard substance)] was calculated.

(Evaluation of residual amount of C-terminal-activated substance)

The LC/MS peak area ratio [propylamide/pentamethylbenzene (internal standard substance)] was used. The relative value of the residual amount of a C-terminal-activated substance in the table below is a relative value obtained when the value of a peak area ratio [propylamide/pentamethylbenzene] at the time of treatment with a 5% aqueous potassium carbonate solution for 5 minutes without adding an amine additive being 3.5 was regarded as 100 (column of entry 1 at 5 min).

$$\text{Relative value (\%) of residual amount of C-terminal-activated substance} = \{[\text{Propylamide (area\%)} / \text{Pentamethylbenzene (area\%)}] / 3.5\ ([\text{Propylamide (area\%)} / \text{Pentamethylbenzene (area\%)}] \text{ of entry 1 at 5 min}])\} \times 100$$

[Table 1]

| | | | Relative value of residual amount of C-terminal-activated substance (%) | | | |
|---|---|---|---|---|---|---|
| entry | Alkaline aqueous solution | Amine | 5 min | 15 min | 30 min | 60 min |
| 1 | 5% $K_2CO_3$ | non | 100 | n/a[1] | 96 | 95 |
| 2 | 5% KOH | non | 102 | 96 | 95 | 94 |
| 3 | 5% LiOH | non | 95 | 93 | 93 | 90 |
| 4 | 5% $Na_2CO_3$ | non | 93 | 91 | 88 | 86 |
| 5 | 5% $C_5CO_3$ | non | 91 | n/a[1] | 90 | 87 |
| 6 | 5% $K_2CO_3$ | DABCO | 99 | n/a[1] | 100 | 95 |
| 7 | 5% $K_2CO_3$ | $Et_3N$ | 94 | 92 | 90 | 87 |
| 8 | 5% $K_2CO_3$ | DBU | 90 | 88 | 86 | 85 |
| 9 | 5% $K_2CO_3$ | NMI | 46 | 10 | 6 | 7 |
| 10 | 5% $K_2CO_3$ | DMAP | 2 | 2 | 2 | 2 |
| 11 | 5% $K_2CO_3$ | Thiazole | 103 | 95 | 95 | 93 |
| 12 | 5% $K_2CO_3$ | Methyltriazole | 97 | 95 | 94 | 94 |
| 1) Not applicable. | | | | | | |

The relative value of the residual amount of a C-terminal-activated substance in Table 1 indicates that the smaller the value is, the more hydrolyzed the residual C-terminal-activated substance is. When alkaline water was used alone, the hydrolysis rate barely changed even when the counter cation of the alkali was changed, and hydrolysis was slower than when amine was added. It was also found that among the amines added, addition of DMAP and NMI dramatically promoted hydrolysis of the residual C-terminal-activated substance.

(Example 2) Effect of added amine in hydrolysis of residual C-terminal-activated substance (Preparation of active ester)

701 mg (2.64 mmol) of Cbz-Ile-OH and 46 mg (0.31 mmol) of pentamethylbenzene were dissolved in 7.0 mL of 2-methyltetrahydrofuran. At room temperature, 1.0 g (2.64 mmol) of HATU and 1.5 mL (8.79 mmol) of diisopropylethylamine were added, and the mixture was stirred at 60°C for 4 hours to prepare an active ester (C-terminal-activated substance) solution. 5 μL of the prepared active ester solution was isolated, reacted with 100 μL (1.2 mmol) of normal propylamine, and then diluted with 0.9 mL of methanol, and the reaction conversion rate to the active ester was determined from the peak area of LC/MS (conversion rate: 94%). Cbz-Ile-NHPr/MS (ESI): m/z 307.1 [M+H]+.

$$\text{Conversion rate (\%)} = \{\text{Cbz-Ile-NHPr (area\%)} / [\text{Cbz-Ile-OH (area\%)} + \text{Cbz-Ile-NHPr (area\%)}]\} \times 100$$

(Hydrolysis treatment with alkaline water alone)

1.5 mL was isolated from the entirety (9 mL) of the prepared active ester solution, 0.75 mL of alkaline water (5% aqueous potassium carbonate solution) was added, and the mixture was stirred (1200 rpm) with a stir bar at 25°C. After stirring was terminated, the mixture was left to stand still to separate the organic layer and the aqueous layer. 5 μL of the organic layer was isolated and added to 100 μL (1.2 mmol) of normal propylamine to convert the residual C-terminal-activated substance to propylamide, and the mixture was then diluted with 0.9 mL of methanol. This solution was subjected to LC/MS analysis, and the peak area ratio [propylamide : pentamethylbenzene (internal standard substance)] was calculated.

(Hydrolysis treatment with amine added)

1.5 mL was isolated from the entirety (9 mL) of the prepared active ester solution, then an amine additive (0.44 mmol) and 0.75 mL of a 5% aqueous potassium carbonate solution were added, and the mixture was stirred (1200 rpm) with a stir bar at 25°C. Stirring was terminated, the mixture was left to stand still to separate the organic layer and the aqueous layer, 5 μL of the organic layer was isolated and added to 100 μL (1.2 mmol) of normal propylamine to convert the residual C-terminal-activated substance to propylamide, and then the mixture was diluted with 0.9 mL of methanol. This solution was subjected to LC/MS analysis, and the peak area ratio [propylamide : pentamethylbenzene (internal standard substance)] was calculated.

(Evaluation of residual amount of C-terminal-activated substance)

The LC/MS peak area ratio [propylamide : pentamethylbenzene (internal standard substance)] was used. The relative value of the residual amount of a C-terminal-activated substance in the table below is a relative value obtained when the value of a peak area ratio [propylamide/pentamethylbenzene] at the time of treatment with a 5% aqueous potassium carbonate solution for 5 minutes without adding an amine additive being 3.0 was regarded as 100 (column of entry 1 at 5 min).

Relative value (%) of residual amount of C-terminal-activated substance = {[Propylamide (area%) / Pentamethylbenzene (area%)] / 3.0 ([Propylamide (area%) / Pentamethylbenzene (area%)] of entry 1 at 5 min])} × 100

[Table 2]

| | | | Relative value of residual amount of C-terminal-activated substance (%) | | | |
|---|---|---|---|---|---|---|
| entry | Alkaline aqueous solution | Amine | 5 min | 15 min | 30 min | 60 min |
| 1 | 5% $K_2CO_3$ | - | 100 | 84 | 72 | 50 |
| 2 | 5% $K_2CO_3$ | DBU | 35 | 20 | 18 | 11 |
| 3 | 5% $K_2CO_3$ | $Me_3N$ | 59 | 33 | 17 | 3.2 |
| 4 | 5% $K_2CO_3$ | NMI | 15 | 2.4 | 0.7 | 0.5 |
| 5 | 5% $K_2CO_3$ | DMAP | 0.5 | 0.5 | 0.5 | 0.4 |

The relative value of the residual amount of a C-terminal-activated substance in Table 2 indicates that the smaller the value is, the more hydrolyzed the residual C-terminal-activated substance is. It was found that hydrolysis of the residual C-terminal-activated substance was more promoted when amine was added than when alkaline water was used alone. That is to say, it was recognized that addition of DBU, $Me_3N$, NMI, and DMAP was effective, and, in particular, it was found that addition of NMI and DMAP was dramatically effective.

(Example 3) Effect of added amine in hydrolysis of residual C-terminal-activated substance

617 mg (2.6 mmol) of Cbz-MeAla-OH and 46 mg (0.31 mmol) of pentamethylbenzene were dissolved in a solution composed of 4.5 mL of 2-methyltetrahydrofuran, then 1.5 mL (8.6 mmol) of diisopropylethylamine and 2.6 mL (4.4 mmol) of a 50% T3P/THF solution were added at room temperature, and the mixture was stirred at 40°C for 1 hour to prepare a mixed acid anhydride solution (C-terminal-activated substance). Then, 5 μL of the prepared mixed acid anhydride solution was isolated, reacted with 100 μL (1.2 mmol) of normal propylamine, and then diluted with 0.9 mL of methanol, and the reaction conversion rate to the mixed acid anhydride was determined from the peak area of LC/MS (conversion rate: 90%). Cbz-MeAla-NHPr/MS (ESI): m/z 279.1 [M+H]+.

Conversion rate (%) = {Cbz-MeAla-NHPr (area%) / [Cbz-MeAla-OH (area%) + Cbz-MeAla-NHPr (area%)]} × 100

(Hydrolysis treatment with alkaline water alone)

1.5 mL was isolated from the entirety (9 mL) of the prepared mixed acid anhydride solution, then 0.75 mL of alkaline water (a 5% aqueous sodium carbonate solution or a 5% aqueous potassium carbonate solution) was added, and the mixture was stirred (1200 rpm) with a stir bar at 25°C. After stirring was terminated, the mixture was left to stand still to separate the organic layer and the aqueous layer. Then, 5 μL of the organic layer was isolated and added to 100 μL (1.2 mmol) of normal propylamine to convert the residual C-terminal-activated substance to propylamide, and then the mixture was diluted with 0.9 mL of methanol. This solution was subjected to LC/MS analysis, and the peak area ratio [propylamide : pentamethylbenzene (internal standard substance)] was calculated.

(Hydrolysis treatment with amine added)

1.5 mL was isolated from the entirety (9 mL) of the prepared mixed acid anhydride solution, then an amine additive (0.43 mmol, 0.67 equivalents) and 0.75 mL of a 5% aqueous potassium carbonate solution were added, and the mixture was stirred (1200 rpm) with a stir bar at 25°C. Stirring was terminated, the mixture was left to stand still to separate the organic layer and the aqueous layer, 5 μL of the organic layer was isolated and added to 100 μL (1.2 mmol) of normal propylamine to convert the residual C-terminal-activated substance to propylamide, and then the mixture was diluted with 0.9 mL of methanol. This solution was subjected to LC/MS analysis, and the peak area ratio [propylamide : pentamethylbenzene (internal standard substance)] was calculated.

(Evaluation of residual amount of C-terminal-activated substance)

The LC/MS peak area ratio [propylamide : pentamethylbenzene (internal standard substance)] was used. The relative value of the residual amount of a C-terminal-activated substance in the table below is a relative value obtained when the value of a peak area ratio [propylamide/pentamethylbenzene] at the time of treatment with a 5% aqueous sodium carbonate solution for 5 minutes without adding an amine additive being 1.1 was regarded as 100 (column of entry 1 at 5 min).

$$\text{Relative value (\%) of residual amount of C-terminal-activated substance} = \{[\text{Propylamide (area\%)} / \text{Pentamethylbenzene (area\%)}] / 1.1\ ([\text{Propylamide (area\%)} / \text{Pentamethylbenzene (area\%)}] \text{ of entry 1 at 5 min}]\} \times 100$$

[Table 3]

| | | | Relative value of residual amount of C-terminal-activated substance (%) | | | |
|---|---|---|---|---|---|---|
| entry | Alkaline aqueous solution | Amine | 5 min | 15 min | 30 min | 60 min |
| 1 | 5% $Na_2CO_3$ | non | 100 | 71 | 52 | 31 |
| 2 | 5% $K_2CO_3$ | non | 108 | 89 | 74 | 52 |
| 3 | 5% $K_2CO_3$ | $Me_3N$ | 127 | 100 | 89 | 60 |
| 4 | 5% $K_2CO_3$ | NMI | 2.7 | 3.0 | 3.0 | 3.1 |
| 5 | 5% $K_2CO_3$ | DMAP | 0 | 0 | 0 | 0 |

The relative value of the residual amount of a C-terminal-activated substance in Table 3 indicates that the smaller the value is, the more hydrolyzed the residual C-terminal-activated substance is. When alkaline water was used alone, the hydrolysis rate barely changed even when the counter cation of the alkali was changed. Also, it was found that addition of DMAP and NMI more promoted hydrolysis of the residual C-terminal-activated substance than alkaline water used alone. It was found that addition of DMAP and NMI was sufficiently effective even within 5 minutes, and, in particular, with DMAP added, the residual C-terminal-activated substance was completely hydrolyzed.

(Example 4) Synthesis of Cbz-Ile-Phe-OtBu

(Condensation reaction)

At room temperature, 1.6 mL (8.9 mmol) of diisopropylethylamine and 2.6 mL (4.4 mmol) of a 50% T3P/THF solution were added to a solution composed of 458 mg (1.8 mmol) of H-Phe-OtBu hydrochloride, 699 mg (2.7 mmol) of Cbz-Ile-OH, and 4.5 mL of 2-methyltetrahydrofuran, and the mixture was stirred at 40°C for 1 hour to carry out a peptide bond forming reaction. Then, 5 μL of the reaction solution was isolated, reacted with 100 μL (1.2 mmol) of normal propylamine, and then diluted with 0.9 mL of methanol, and the reaction conversion rate was determined from the peak area of LC/MS (conversion rate: 100%).

$$\text{Conversion rate (\%)} = \{\text{Cbz-Ile-Phe-OtBu (area\%)} / [\text{H-Phe-OtBu (area\%)} + \text{Cbz-Ile-Phe-OtBu (area\%)}]\} \times 100$$

(Hydrolysis treatment with alkaline water alone)

1.5 mL was isolated from the entirety (9 mL) of the prepared dipeptide solution above, 0.75 mL of a 5% aqueous potassium carbonate solution was added, and the mixture was stirred (1200 rpm) with a stir bar at 25°C. After stirring was terminated, the mixture was left to stand still to separate the organic layer and the aqueous layer. Then, 5 μL of the organic layer was isolated and added to 100 μL (1.2 mmol) of normal propylamine to convert the residual C-terminal-activated substance to propylamide, and the mixture was then diluted with 0.9 mL of methanol and subjected to LC/MS analysis to determine the peak area values of propylamide and the peptide of interest and calculate the residual rate (%) of the C-terminal-activated substance. The aqueous layer of the remaining reaction solution was removed, and the organic layer was successively washed with 0.5 mL of a 5% aqueous potassium hydrogen sulfate solution and 0.5 mL of a 5% aqueous potassium carbonate solution. Then, 5 μL of the organic layer was isolated and added to 100 μL (1.2 mmol) of normal propylamine to convert the residual C-terminal-activated substance to propylamide, the mixture was then diluted with 0.9 mL of methanol. This solution was subjected to LC/MS analysis to determine the peak area values of the peptide of interest and the residual C-terminal-activated substance (a converting substance to propylamide).

$$\text{Residual rate (\%) of C-terminal-activated substance} = \{\text{Propylamide (area\%)} / [\text{Propylamide (area\%)} + \text{Dipeptide (area\%)}]\} \times 100$$

(Hydrolysis treatment with amine added)

1.5 mL was isolated from the entirety (9 mL) of the prepared dipeptide solution, amine (0.15 mmol, 0.5 equivalents; 0.30 mmol, 1.0 equivalent; or 0.89 mmol, 3 equivalent: equivalents relative to H-Phe-OtBu hydrochloride) and 0.75 mL of a 5% aqueous potassium carbonate solution were added, and the mixture was stirred (1200 rpm) with a stir bar at 25°C. After stirring was terminated, the mixture was left to stand still to separate the organic layer and the aqueous layer. 5 μL of the organic layer was isolated and added to 100 μL (1.2 mmol) of propylamine to convert the residual C-terminal-activated substance to propylamide, and the mixture was then diluted with 0.9 mL of methanol and subjected to LC/MS analysis to determine the peak area values of propylamide and the peptide of interest and calculate the residual rate (%) of the C-terminal-activated substance. The aqueous layer of the remaining reaction solution was removed, and the organic layer was successively washed with 0.75 mL of a 5% aqueous potassium hydrogen sulfate solution and 0.75 mL of a 5% aqueous potassium carbonate solution. Then, 5 μL of the organic layer was isolated and added to 100 μL (1.2 mmol) of normal propylamine to convert the residual C-terminal-activated substance to propylamide, and the mixture was then diluted with 0.9 mL of methanol. This solution was subjected to LC/MS analysis to determine the peak area values of the peptide of interest and the residual C-terminal-activated substance (a converting substance to propylamide). MS (ESI): m/z 413.3 [M-tBu+H]+, 469.3 [M+H]+, 491.3[M+Na]+.

$$\text{Residual rate (\%) of C-terminal-activated substance} = \{\text{Propylamide (area\%)} / [\text{Propylamide (area\%)} + \text{Dipeptide (area\%)}]\} \times 100$$

[Table 4]

| | | Hydrolysis | | Organic layer area percentage value after liquid separation operation (%)[2] | |
|---|---|---|---|---|---|
| entry | Amine (eq)[1] | Residual rate of C-terminal-activated substance (%) | Treatment time (min) | Residual C-terminal-activated substance (converting substance to propylamide) | Dipeptide |
| 1 | non | 18.4 | 60 | 4.7 | 90.4 |
| 2 | NMI (0.5 eq) | 5.7 | 60 | Nd | 97.8 |
| 3 | NMI (1.0 eq) | 3.7 | 60 | Nd | 98.1 |
| 4 | NMI (3.0 eq) | 3.2 | 30 | Nd | 98.4 |
| 5 | DMAP (0.5 eq) | 3.7 | 5 | Nd | 98.0 |
| 6 | DMAP (1.0 eq) | 2.9 | 5 | Nd | 97.8 |
| 7 | DMAP (3.0 eq) | 2.0 | 5 | Nd | 98.0 |
| 1) Equivalent relative to N-terminal amino acid derivative (H-Phe-OtBu hydrochloride)<br>2) Peak area percentage of LCMS | | | | | |

It was found that the added amine NMI, when used in an amount of 0.5 to 3.0 equivalents relative to the N-terminal amino acid derivative, promoted hydrolysis more advantageously over hydrolysis by treatment with alkaline water alone. It was also found that the added amine DMAP, when used in an amount of 0.5 to 3.0 equivalents relative to the N-terminal amino acid derivative, promoted hydrolysis more advantageously over hydrolysis by treatment with alkaline water alone.

It was found that after adding amine and performing a hydrolysis treatment once, washing the organic layer with 5% $KHSO_4$ and 5% $K_2CO_3$ can completely remove the residual C-terminal-activated substance in the organic layer. At this time, the peptide of interest was obtained in high purity. On the other hand, after an alkaline water treatment alone, the C-terminal-activated substance remained, and the purity of dipeptide was also poor.

(Example 5) Synthesis of Cbz-Ile-Phe-OtBu

(Condensation reaction)

At room temperature, 1.5 mL (8.8 mmol) of diisopropylethylamine and 2.6 mL (4.4 mmol) of a 50% T3P/THF solution were added to a solution composed of 452 mg (1.8 mmol) of H-Phe-OtBu hydrochloride, 702 mg (2.6 mmol) of Cbz-Ile-OH, and 4.5 mL of 2-methyltetrahydrofuran, and the mixture was stirred at 40°C for 1 hour to carry out a peptide bond forming reaction. Then, 5 μL of the reaction solution was isolated, reacted with 100 μL (1.2 mmol) of normal propylamine, and then diluted with 0.9 mL of methanol, and the reaction conversion rate was determined from the peak area of LC/MS (conversion rate: 100%).

$$\text{Conversion rate (\%)} = \{\text{Cbz-Ile-Phe-OtBu (area \%)} / [\text{H-Phe-OtBu (area\%)} + \text{Cbz-Ile-Phe-OtBu (area\%)}]\} \times 100$$

(Hydrolysis treatment with alkaline water alone)

1.5 mL was isolated from the entirety (9 mL) of the prepared dipeptide solution above, 0.75 mL of a 5% aqueous potassium carbonate solution was added, and the mixture was stirred (1200 rpm) with a stir bar at 60°C. After stirring was terminated, the mixture was left to stand still to separate the organic layer and the aqueous layer. 5 μL of the organic layer was isolated and added to 100 μL (1.2 mmol) of normal propylamine to convert the residual C-terminal-activated substance to propylamide, the mixture was then diluted with 0.9 mL of methanol and subjected to LC/MS analysis to determine the peak area values of propylamide and the peptide of interest and calculate the residual rate (%) of the C-terminal-activated substance. The aqueous layer of the remaining reaction solution was removed, and the organic layer was successively washed with 0.75 mL of a 5% aqueous potassium hydrogen sulfate solution and 0.75 mL of a 5% aqueous potassium carbonate solution. Then, 5 μL of the organic layer was isolated and added to 100 μL (1.2 mmol) of normal propylamine to convert the residual C-terminal-activated substance to propylamide, and the mixture was then diluted with 0.9 mL of methanol. This solution was subjected to LC/MS analysis to determine the peak area values of the peptide of interest and the residual C-terminal-activated substance (a converting substance to propylamide).

$$\text{Residual rate (\%) of C-terminal-activated substance} = \{\text{Propylamide (area\%)} / [\text{Propylamide (area\%)} + \text{Dipeptide (area\%)}]\} \times 100$$

(Hydrolysis treatment with amine added)

1.5 mL was isolated from the entirety (9 mL) of the prepared dipeptide solution, amine (0.29 mmol) and 0.75 mL of a 5% aqueous potassium carbonate solution were added, and the mixture was stirred (1200 rpm) with a stir bar at 60°C. After stirring was terminated, the mixture was left to stand still to separate the organic layer and the aqueous layer. 5 μL of the organic layer was isolated and added to 100 μL (1.2 mmol) of propylamine to convert the residual C-terminal-activated substance to propylamide, and the mixture was then diluted with 0.9 mL of methanol and subjected to LC/MS analysis to determine the peak area values of propylamide and the peptide of interest and calculate the residual rate (%) of the C-terminal-activated substance. The aqueous layer of the remaining reaction solution was removed, and the organic layer was successively washed with 0.75 mL of a 5% aqueous potassium hydrogen sulfate solution and 0.75 mL of a 5% aqueous potassium carbonate solution. Then, 5 μL of the organic layer was isolated and added to 100 μL (1.2 mmol) of normal propylamine to convert the residual C-terminal-activated substance to propylamide, and the mixture was then diluted with 0.9 mL of methanol. This solution was subjected to LC/MS analysis to determine the peak area values of the peptide of interest and the residual C-terminal-activated substance (a converting substance to propylamide). MS(ESI): m/z 413.3 [M-tBu+H]+, 469.3 [M+H]+, 491.3 [M+Na]+.

$$\text{Residual rate (\%) of C-terminal-activated substance} = \{\text{Propylamide (area\%)} / [\text{Propylamide (area\%)} + \text{Dipeptide (area\%)}]\} \times 100$$

[Table 5]

| | | | Hydrolysis | | Organic layer area percentage value after liquid separation operation (%)[2] | |
|---|---|---|---|---|---|---|
| entry | Alkaline aqueous solution | Amine (1 eq) | Residual rate of C-terminal-activated substance (%) | Hydrolysis treatment time (min) | Residual C-terminal-activated substance (converting substance to propylamide) | Dipeptide |
| 1 | 5% $K_2CO_3$ | - | 20.1 | 60 | 3.4 | 89.7 |
| 2 | 5% $K_2CO_3$ | $Me_3N$ | 19.6 | 60 | 3.3 | 89.6 |
| 3 | 5% $K_2CO_3$ | NMI | 5.8 | 5 | nd | 97.5 |

(continued)

| | | | Hydrolysis | | Organic layer area percentage value after liquid separation operation (%)[2] | |
|---|---|---|---|---|---|---|
| entry | Alkaline aqueous solution | Amine (1 eq) | Residual rate of C-terminal-activated substance (%) | Hydrolysis treatment time (min) | Residual C-terminal-activated substance (converting substance to propylamide) | Dipeptide |
| 4 | 5% $K_2CO_3$ | DMAP | 3.4 | 5 | nd | 97.6 |
| 1) Peak area ratio of LCMS | | | | | | |

Even when the residual C-terminal-activate substance was hydrolyzed at 60°C with amine added, the peptide of interest was obtained in the same high purity as the purity attained when the hydrolysis treatment was carried out at 25°C. In particular, when the added amine was DMAP or NMI, hydrolysis proceeded faster than hydrolysis with alkaline water alone. It was also found that when the added amine was DMAP or NMI, hydrolysis proceeded effectively within 5 minutes in a single hydrolysis treatment, and the subsequent liquid separation operation (washing with 5% $KHSO_4$ and 5% $K_2CO_3$) could completely remove the residual C-terminal-activated substance from the organic layer.

(Example 6) Synthesis of Cbz-MeIle-MePhe-OMe

(Condensation reaction)

300 mg (1.3 mmol) of MePhe-OMe hydrochloride and 442 mg (1.6 mmol) of Cbz-MeIle-OH were suspended in 3.0 mL of acetonitrile, and 683 μL (3.9 mmol) of diisopropylethylamine was added. Then, 594 mg (1.6 mmol) of HATU was added at 25°C, and the mixture was stirred at 25°C for 30 minutes, then stirred at 40°C for 3 hours, and further stirred at 60°C for 3 hours to carry out a peptide bond forming reaction. 5 μL of the reaction solution was isolated and added to 100 μL (1.2 mmol) of normal propylamine to convert the residual C-terminal-activated substance to propylamide, and the mixture was then diluted with 0.9 mL of methanol. This solution was subjected to LC/MS analysis to determine the conversion rate from the peak area value of LC/MS (conversion rate: >99%).

Conversion rate (%) = {Cbz-MeIle-MePhe-OMe (area%) / [MePhe-OMe (area%) + Cbz-MeIle-MePhe-OMe (area%)]} × 100

(Hydrolysis treatment)

(1) When no amine was added

3.0 mL of MTBE and 3.0 mL of a 5% aqueous potassium carbonate solution were added to the peptide-containing reaction solution prepared above, and the mixture was stirred with a stir bar at 25°C for 30 minutes. Stirring was terminated to separate the organic layer and the aqueous layer. Then, 5 μL of the organic layer was isolated and added to 100 μL (1.2 mmol) of normal propylamine to convert the residual C-terminal-activated substance to propylamide, and the mixture was then diluted with 0.9 mL of methanol. From the peak area value of LC/MS, the residual rate of the C-terminal-activated substance was calculated according to the following calculation formula.

Residual rate (%) of C-terminal-activated substance = {Propylamide (area%) / [Propylamide (area%) + Dipeptide (area%)]} × 100

(2) When amine was added

3.0 mL of MTBE, 103 μL (1.3 mmol) of N-methylimidazole, and 3.0 mL of a 5% aqueous potassium carbonate solution were added to the peptide-containing reaction solution prepared above, and the mixture was stirred with a stir bar at 25°C for 30 minutes. Stirring was terminated to separate the organic layer and the aqueous layer. 5 μL of the organic layer was isolated and added to 100 μL (1.2 mmol) of normal propylamine to convert the residual C-terminal-activated substance to propylamide, and the mixture was then diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(Work-up)

After stirring was terminated, the mixture was left to stand still to separate the organic layer and the aqueous layer, and the aqueous layer was removed. Then, the organic layer was sequentially washed with 3 mL × 2 of a 10% aqueous potassium hydrogen sulfate solution, 3 mL of a 5% aqueous potassium carbonate solution, and 1 mL × 5 of common water. Stirring was terminated to separate the organic layer and the aqueous layer. Then, 5 μL of the organic layer was isolated and added to 100 μL (1.2 mmol) of normal propylamine to convert the residual C-terminal-activated substance to propylamide, and the mixture was then diluted with 0.9 mL of methanol and subjected to LC/MS analysis to determine the peak area values of the peptide of interest and the residual C-terminal-activated substance (a converting substance to propylamide). The remaining organic layer was concentrated to give a peptide. The concentrate (peptide) obtained by hydrolysis without addition of amine was 671.8 mg (yield 113%: calculated as solely containing peptide although the concentrate contained impurities (residual C-terminal-activated substance)). The concentrate obtained by hydrolysis with addition of amine was 563.7 mg (yield 95%). MS (ESI): m/z 455.2 [M+H]+, 477.2 [M+Na]+.

[Table 6]

| entry | | Immediately after hydrolysis treatment | Organic layer after liquid separation operation | | After concentration |
|---|---|---|---|---|---|
| | Amine | Residual rate of C-terminal-activated substance (%) | Dipeptide (%)[1] | Residual C-terminal-activated substance (%)[1] (converting substance to propylamide) | Yield (%) |
| 1 | NMI | 0 | 100 | Nd | 95 |
| 2 | non | 5.7 | 95.8 | 4.2 | 113 |
| 1) Peak area ratio of LCMS | | | | | |

By hydrolysis with alkaline water alone, the residual C-terminal-activated substance was not completely hydrolyzed, and it was also not possible to remove the residual C-terminal-activated substance by subsequent aqueous washing; however, it was found that when hydrolysis was carried out with addition of NMI, hydrolysis of the residual C-terminal-activated substance was completely achieved, and the residual C-terminal-activated substance could be completely removed. Moreover, at this time, the dipeptide of interest was obtained in a purity of 100% (yield 95%).

(Example 7) Synthesis of Cbz-MeVal-MeAsp(tBu)-piperidine

(Condensation reaction)

303 mg (1.1 mmol) of MeAsp(tBu)-piperidine and 448 mg (1.7 mmol) of Cbz-MeVal-OH were suspended in a mixed solvent of 0.6 mL of acetonitrile and 2.4 mL of cyclopentyl methyl ether, and 586 μL (3.4 mmol) of diisopropylethylamine was added. Then, 642 mg (1.7 mmol) of HATU was added at 25°C, and the mixture was stirred at 25°C for 6.5 hours to carry out a peptide bond forming reaction. 5 μL of the reaction solution was isolated and added to 100 μL (1.2 mmol) of normal propylamine to convert the residual C-terminal-activated substance to propylamide, and the mixture was then diluted with 0.9 mL of methanol. This solution was subjected to LC/MS analysis to determine the conversion rate from the peak area value of LC/MS (conversion rate: 100%).

$$\text{Conversion rate (\%)} = \{\text{Cbz-MeVal-MeAsp(tBu)-piperidine (area\%)} / [\text{MeAsp(tBu)-piperidine (area\%)} + \text{Cbz-MeVal-MeAsp(tBu)-piperidine (area\%)}]\} \times 100$$

(Hydrolysis treatment)

(1) When no amine was added

3.0 mL of a 5% aqueous potassium carbonate solution was added to the peptide-containing reaction solution prepared above, and the mixture was stirred with a stir bar at 25°C for 5 minutes. Stirring was terminated to separate the organic layer and the aqueous layer. 5 μL of the organic layer was isolated and added to 100 μL (1.2 mmol) of normal propylamine to convert the residual C-terminal-activated substance to propylamide, and the mixture was then diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(2) When amine was added

136 mg (1.1 mmol) of DMAP and 3.0 mL of a 5% aqueous potassium carbonate solution were added to the peptide-containing reaction solution prepared above, and the mixture was stirred with a stir bar at 25°C for 5 minutes. Stirring was terminated to separate the organic layer and the aqueous layer. 5 μL of the organic layer was isolated and added to 100 μL (1.2 mmol) of normal propylamine to convert the residual C-terminal-activated substance to propylamide, and the mixture was then diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(Work-up)

After stirring was terminated, the mixture was left to stand still to separate the organic layer and the aqueous layer, and the aqueous layer was removed. Then, the organic layer was sequentially washed with 3 mL × 2 of a 10% aqueous potassium hydrogen sulfate solution, 3 mL × 2 of a 5% aqueous potassium carbonate solution, and 1.5 mL × 3 of common water. Stirring was terminated to separate the organic layer and the aqueous layer. 5 μL of the organic layer was isolated and added to 100 μL (1.2 mmol) of normal propylamine to convert the residual C-terminal-activated substance to propylamide, and the mixture was then diluted with 0.9 mL of methanol and subjected to LC/MS analysis to determine the peak area values of the peptide of interest and the residual C-terminal-activated substance (a converting substance to propylamide). The remaining organic layer was concentrated to give a peptide. The concentrate (peptide) obtained by hydrolysis without addition of amine was 782.0 mg (yield 136%: calculated as solely containing peptide although the concentrate contained impurities (residual C-terminal-activated substance)). The concentrate obtained by hydrolysis with addition of amine was 530.6 mg (yield 92%). MS (ESI): m/z 518.4 [M+H]+, 540.4 [M+Na]+.

[Table 7]

| entry | | Immediately after hydrolysis treatment | Organic layer after liquid separation operation | | After concentration |
|---|---|---|---|---|---|
| | Amine | Residual rate of C-terminal-activated substance (%) | Dipeptide (%)1 | Residual C-terminal-activated substance (%)1 (converting substance to propylamide) | Yield (%) |
| 1 | DMAP | 0 | 100 | Nd | 92 |
| 2 | none | 23.0 | 80.4 | 17.6 | 136 |
| 1) Peak area ratio of LCMS | | | | | |

By hydrolysis with alkaline water alone, the residual C-terminal-activated substance was not completely hydrolyzed, and it was also not possible to remove the residual C-terminal-activated substance by subsequent aqueous washing; however, it was found that when hydrolysis was carried out with addition of DMAP, hydrolysis of the residual

C-terminal-activated substance was completely achieved, and the residual C-terminal-activated substance could be completely removed. Moreover, at this time, the dipeptide of interest was obtained in a purity of 100% (yield 92%).

(Example 8) Synthesis of Cbz-MeVal-MeAsp(tBu)-piperidine

(Condensation reaction)

299 mg (1.1 mmol) of MeAsp(tBu)-piperidine and 458 mg (1.7 mmol) of Cbz-MeVal-OH were suspended in 4.5 mL of a 2-MeTHF solvent, and 775 μL (4.4 mmol) of diisopropylethylamine was added. Then, 1.6 mL (2.8 mmol) of a 50% T3P/THF solution was added at 25°C, and the mixture was stirred at 25°C for 15 hours to carry out a peptide bond forming reaction. 5 μL of the reaction solution was isolated and added to 100 μL (1.2 mmol) of normal propylamine to convert the residual C-terminal-activated substance to propylamide, and the mixture was then diluted with 0.9 mL of methanol. This solution was subjected to LC/MS analysis to determine the conversion rate from the peak area value of LC/MS (conversion rate: 100%).

$$\text{Conversion rate (\%)} = \{\text{Cbz-MeVal-MeAsp(tBu)-piperidine (area\%)} / [\text{MeAsp(tBu)-piperidine (area\%)} + \text{Cbz-MeVal-MeAsp(tBu)-piperidine (area\%)}]\} \times 100$$

(Hydrolysis treatment)

(1) When no amine was added

3.0 mL of a 5% aqueous potassium carbonate solution was added to the peptide-containing reaction solution prepared above, and the mixture was stirred with a stir bar at 25°C for 5 minutes. Stirring was terminated to separate the organic layer and the aqueous layer. 5 μL of the organic layer was isolated and added to 100 μL (1.2 mmol) of normal propylamine to convert the residual C-terminal-activated substance to propylamide, and the mixture was then diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(2) When amine was added

141 mg (1.1 mmol) of DMAP and 3.0 mL of a 5% aqueous potassium carbonate solution were added to the peptide-containing reaction solution prepared above, and the mixture was stirred with a stir bar at 25°C for 5 minutes. Stirring was terminated to separate the organic layer and the aqueous layer. 5 μL of the organic layer was isolated and added to 100 μL (1.2 mmol) of normal propylamine to convert the residual C-terminal-activated substance to propylamide, and the mixture was then diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(Work-up)

After stirring was terminated, the mixture was left to stand still to separate the organic layer and the aqueous layer, and the aqueous layer was removed. Then, the organic layer was sequentially washed with 3 mL of a 10% aqueous potassium hydrogen sulfate solution and 3 mL of a 5% aqueous potassium carbonate solution. Stirring was terminated to separate the organic layer and the aqueous layer. 5 μL of the organic layer was isolated and added to 100 μL (1.2 mmol) of normal propylamine to convert the residual C-terminal-activated substance to propylamide, and the mixture was then diluted with 0.9 mL of methanol and subjected to LC/MS analysis to determine the peak area values of the peptide of interest and the residual C-terminal-activated substance (a converting substance to propylamide). The remaining organic layer was concentrated to give a peptide. The concentrate (peptide) obtained by hydrolysis without addition of amine was 561.6 mg (yield 98%: calculated as solely containing peptide although the concentrate contained

impurities (residual C-terminal-activated substance)). The concentrate obtained by hydrolysis with addition of amine was 501.1 mg (yield 87%). MS (ESI): m/z 518.4 [M+H]+, 540.4 [M+Na]+.

[Table 8]

| entry | | Immediately after hydrolysis treatment | Organic layer after liquid separation operation | | After concentration |
|---|---|---|---|---|---|
| | Amine | Residual rate of C-terminal-activated substance (%) | Dipeptide (%)[1] | Residual C-terminal-activated substance (%)[1] (converting substance to propylamide) | Yield (%) |
| 1 | DMAP | 2.8 | 100 | Nd | 87 |
| 2 | non | 14.0 | 88.0 | 6.7 | 98 |
| 1) Peak area ratio of LCMS | | | | | |

By hydrolysis with alkaline water alone, the residual C-terminal-activated substance was not completely hydrolyzed, and it was also not possible to remove the residual C-terminal-activated substance by subsequent aqueous washing; however, it was found that when hydrolysis was carried out with addition of DMAP, hydrolysis of the residual C-terminal-activated substance was completely achieved, and the residual C-terminal-activated substance could be completely removed. Moreover, at this time, the dipeptide of interest was obtained in a purity of 100% (yield 87%).

(Example 9) Synthesis of Cbz-Ile-MeVal-MeAsp(tBu)-piperidine

(Cbz deprotection reaction using dipeptide obtained by hydrolysis treatment without addition of amine)

782 mg of Cbz-MeVal-MeAsp(tBu)-piperidine (containing 17.6 area% of a residual C-terminal-activated substance) synthesized under the amine-free condition of Example 7 was dissolved in 4.2 mL of cyclopentyl methyl ether. The mixture was subjected to a hydrogenolysis reaction with 115 mg of 5% Pd/C (50% wet) and hydrogen gas. Since the reaction barely proceeded, Pd/C was filtered off using a filter, the mixture was concentrated to dryness, again dissolved in 4.2 mL of cyclopentyl methyl ether, mixed with 105 mg of 5% Pd/C (50% wet), and again subjected to a hydrogenolysis reaction. However, despite total 3 hours of attempting a reaction, the reaction barely proceeded (reaction conversion rate: 1.6%). The reaction conversion rate was determined from the peak area value of LC/MS by isolating 5 μL of the reaction solution, diluting the solution with 1.0 mL of acetonitrile, and then subjecting the filtrated solution to LC/MS analysis.

$$\text{Conversion rate (\%)} = \{\text{MeVal-MeAsp(tBu)-piperidine (area\%)} / [\text{MeVal-MeAsp(tBu)-piperidine (area\%)} + \text{Cbz-MeVal-MeAsp(tBu)-piperidine (area\%)}]\} \times 100$$

(Cbz deprotection reaction using dipeptide obtained by hydrolysis treatment with addition of amine)

543 mg (1.0 mmol) of Cbz-MeVal-MeAsp(tBu)-piperidine synthesized under the amine-added condition of Example 7 was dissolved in 4.3 mL of cyclopentyl methyl ether. The mixture was subjected to a hydrogenolysis reaction with 124 mg of 5% Pd/C (50% wet) and hydrogen gas. The mixture was stirred at room temperature for 2 hours to give Cbz-removed product MeVal-MeAsp(tBu)-piperidine (conversion rate 100%). The reaction conversion rate was determined from the peak area value of LC/MS by isolating 5 μL of the reaction solution, diluting the solution with 1.0 mL of acetonitrile, and then subjecting the filtrated solution to LC/MS analysis. MS (ESI): m/z 384.3 [M+H]+.

$$\text{Conversion rate (\%)} = \{\text{MeVal-MeAsp(tBu)-piperidine (area\%)} / [\text{MeVal-MeAsp(tBu)-piperidine (area\%)} + \text{Cbz-MeVal-MeAsp(tBu)-piperidine (area\%)}]\} \times 100$$

(Condensation reaction)

The reaction solution was passed through a filter to filter off Pd/C, and then concentrated to dryness. The dried residue was dissolved in 4.3 mL of 2-methyltetrahydrofuran, and 362 mg (1.3 mmol) of Cbz-Ile-OH and 715 μL (4.1 mmol) of diisopropylethylamine were added. Then, 1.4 mL (2.4 mmol) of a 50% T3P/THF solution was added at 25°C,

and the mixture was stirred at 40°C for 7 hours and further stirred at room temperature for 14 hours to carry out a peptide bond forming reaction (conversion rate: 100%). To the prepared reaction solution, 81 μL (1.0 mmol) of N-methylimidazole and 2.6 mL of a 20% aqueous potassium carbonate solution were added, and the mixture was stirred with a stir bar at 25°C for 45 minutes. After stirring was terminated, the mixture was left to stand still to separate the organic layer and the aqueous layer, and the aqueous layer was removed. Next, the organic layer was sequentially washed with 5.2 mL of a 10% aqueous potassium hydrogen sulfate solution and 5.2 mL × 2 of a 5% aqueous potassium carbonate solution. 5 μL of the resulting organic layer was added to 100 μL of normal propylamine and diluted with 0.9 mL of methanol. This solution was subjected to LC/MS analysis to determine the peak area percentages of the peptide of interest and the residual C-terminal-activated substance. The peptide of interest Cbz-Ile-MeVal-MeAsp(tBu)-piperidine was 95.1%, and Cbz-Ile-NHPr derived from the residual C-terminal-activated substance was not detected. The remaining organic layer was concentrated to give 542.7 mg of a concentrate (yield 82%). MS (ESI): m/z 631.5 $[M+H]^+$, 653.4 $[M+Na]^+$.

It was found that when a peptide solution treated solely with alkaline water and containing a residual C-terminal-activated substance was used, the Cbz deprotection reaction barely proceeded. On the other hand, it was found that when a peptide solution obtained by treatment with addition of DMAP and completely free of a residual C-terminal-activated substance was used, the Cbz deprotection reaction proceeded smoothly, thus enabling the subsequent peptide synthesis reaction. That is to say, it was found that the use of the method of the present invention enabled the reductive removal reaction of the N-terminal protecting group of the produced peptide compound to proceed without stagnation. Accordingly, it was possible to efficiently produce a high-purity peptide compound having a desired amino acid sequence.

(Example 10) Synthesis of Cbz-Phe(3-F)-Phe-OtBu

200 mg (0.8 mmol) of Phe-OtBu hydrochloride and 297 mg (0.9 mmol) of Cbz-Phe(3-F)-OH were suspended in 3.0 mL of toluene, and 407 μL (2.3 mmol) of diisopropylethylamine was added. Then, 0.9 mL (1.6 mmol) of a 50% T3P/THF solution was added at 25°C, and the mixture was stirred at room temperature for 30 minutes to carry out a peptide bond forming reaction (conversion rate: 100%). The reaction conversion rate was determined from the peak area value of LC/MS by isolating 5 μL of the reaction solution, adding it to 100 μL of normal propylamine, diluting the mixture with 0.9 mL of methanol, and then subjecting the solution to LC/MS analysis.

Conversion rate (%) = {Cbz-Phe(3-F)-Phe-OtBu (area%) / [Phe-OtBu (area%) + Cbz-Phe(3-F)-Phe-OtBu (area%)]} × 100

95 mg (0.8 mmol) of DMAP and 2.0 mL of a 5% aqueous potassium carbonate solution were added to the above reaction solution, and the mixture was stirred with a stir bar at 25°C for 5 minutes. After stirring was terminated, the mixture was left to stand still to separate the organic layer and the aqueous layer, and the aqueous layer was removed. Then, the organic layer was sequentially washed with 1 mL of a 10% aqueous potassium hydrogen sulfate solution, 1 mL of a 5% aqueous potassium carbonate solution, and 1 mL of common water. 5 μL of the resulting organic layer was added to 100 μL of normal propylamine, and the mixture was diluted with 0.9 mL of methanol. This solution was subjected to LC/MS analysis to determine the peak area percentages of the peptide of interest and the residual C-terminal-activated substance. The purity of the peptide of interest Cbz-Phe(3-F)-Phe-OtBu was 100%, and Cbz-Phe(3-F)-NHPr derived from the residual C-terminal-activated substance was not detected. The remaining organic layer was concentrated to give 387.2 mg of a concentrate (yield 96%). MS (ESI): m/z 465.2 [M-tBu+H]+, 521.1 [M+H]+, 543.2 [M+Na]+.

When hydrolysis was carried out with addition of DMAP, removal of the residual C-terminal-activated substance was completely achieved, and it was possible to obtain the dipeptide of interest in a purity of 100% (yield 96%).

(Example 11) Synthesis of Cbz-Ser(OtBu)-Phe-OtBu

300 mg (1.2 mmol) of Phe-OtBu hydrochloride and 450 mg (1.5 mmol) of Cbz-Ser(OtBu)-OH were suspended in 3.6 mL of 2-methyltetrahydrofuran, and 610 μL (3.5 mmol) of diisopropylethylamine was added. Then, 1.4 mL (2.3 mmol) of a 50% T3P/THF solution was added at 25°C, and the mixture was stirred at room temperature for 1 hour to carry out a peptide bond forming reaction (conversion rate: 100%). The reaction conversion rate was determined from the peak area value of LC/MS by isolating 5 μL of the reaction solution, adding it to 100 μL of normal propylamine, diluting the mixture with 0.9 mL of methanol, and then subjecting the solution to LC/MS analysis.

Conversion rate (%) = {Cbz-Ser(tBu)-Phe-OtBu (area%) / [Phe-OtBu (area%) + Cbz-Ser(tBu)-Phe-OtBu (area%)]} × 100

143 mg (1.2 mmol) of DMAP and 1.5 mL of a 20% aqueous potassium carbonate solution were added to the above reaction solution, and the mixture was stirred with a stir bar at 25°C for 5 minutes. After stirring was terminated, the mixture was left to stand still to separate the organic layer and the aqueous layer, and the aqueous layer was removed. The organic layer was sequentially washed with 3.0 mL × 2 of a 10% aqueous potassium hydrogen sulfate solution, 3.0 mL of a 5% aqueous potassium carbonate solution, and 3.0 mL of common water. 5 μL of the resulting organic layer was added to 100 μL of normal propylamine, and the mixture was diluted with 0.9 mL of methanol. This solution was subjected to LC/MS analysis to determine the peak area percentages of the peptide of interest and the residual C-terminal-activated substance. The purity of the peptide of interest Cbz-Ser(OtBu)-Phe-OtBu was 100%, and Cbz-Ser(OtBu)-NHPr derived from the residual C-terminal-activated substance was not detected. The remaining organic layer was concentrated to give 556.4 mg of a concentrate (yield 96%). MS (ESI): m/z 387.1 [M-2tBu+H]+, 499.3 [M+H]+, 521.2 [M+Na]+.

When hydrolysis was carried out with addition of DMAP, removal of the residual C-terminal-activated substance was completely achieved, and it was possible to obtain the dipeptide of interest in a purity of 100% (yield 96%).

(Example 12) Synthesis of Boc-MeVal-Phe-piperidine

(Boc deprotection reaction)

471 mg (1.4 mmol) of Boc-Phe-piperidine was dissolved in 4.7 mL of dichloromethane, and 180 μL (2.8 mmol) of methanesulfonic acid was added. The mixture was stirred at 35°C for 2 hours to carry out a Boc removal reaction (conversion rate 100%). The reaction conversion rate was determined from the peak area value of LC/MS by isolating 5 μL of the reaction solution, diluting the solution with 1.0 mL of acetonitrile, and then subjecting the solution to LC/MS analysis.

$$\text{Conversion rate (\%)} = \{\text{Phe-piperidine (area\%)} / [\text{Boc-Phe-piperidine (area\%)} + \text{Phe-piperidine (area\%)}]\} \times 100$$

(Condensation reaction)

After 742 μL (4.3 mmol) of diisopropylethylamine was added to the above reaction solution, the solvent was distilled off. Then, 1.4 mL of acetonitrile, 3.3 mL of 2-methyltetrahydrofuran, 742 μL (4.3 mmol) of diisopropylethylamine, and 492 mg (2.1 mmol) of Boc-MeVal-OH were added. Then, 804 mg (2.2 mmol) of HATU was added at 25°C, and the mixture was stirred at room temperature for 1 hour to carry out a peptide bond forming reaction (conversion rate: 100%). The reaction conversion rate was determined from the peak area value of LC/MS by isolating 5 μL of the reaction solution, adding it to 100 μL of normal propylamine, diluting the mixture with 0.9 mL of methanol, and then subjecting the solution to LC/MS analysis.

$$\text{Conversion rate (\%)} = \{\text{Boc-MeVal-Phe-piperidine (area\%)} / [\text{Phe-piperidine (area\%)} + \text{Boc-MeVal-Phe-piperidine (area\%)}]\} \times 100$$

To the reaction solution prepared above, 168 mg (1.4 mmol) of DMAP and 4.6 mL of a 5% aqueous potassium carbonate solution were added, and the mixture was stirred with a stir bar at 25°C for 5 minutes. After stirring was terminated, the mixture was left to stand still to separate the organic layer and the aqueous layer, and the aqueous layer was removed. The organic layer was sequentially washed with 4.6 mL of a 10% aqueous potassium hydrogen sulfate solution, 4.6 mL of a 5% aqueous potassium carbonate solution, and 1.5 mL × 6 of common water. 5 μL of the resulting organic layer was added to 100 μL of normal propylamine, and the mixture was diluted with 0.9 mL of methanol. This solution was subjected to LC/MS analysis to determine the peak area percentages of the peptide of interest and the residual C-terminal-activated substance. The purity of the peptide of interest Boc-MeVal-Phe-OtBu was 99.7%, and Boc-MeVal-NHPr derived from the residual C-terminal-activated substance was not detected. The remaining organic layer was concentrated to give 542.3 mg of a concentrate (yield 86%). MS (ESI): m/z 346.2 [M-Boc+H]+, 446.3 [M+H]+, 468.3 [M+Na]+.

When hydrolysis was carried out with addition of DMAP, removal of the residual C-terminal-activated substance was completely achieved, and it was possible to obtain the dipeptide of interest in a purity of 99.7% (yield 86%) even when the N-terminal protecting group was Boc.

(Example 13) Synthesis Example of Cbz-Ile-MeAla-Aze-MePhe-MeGly-OtBu/SEQ ID NO: 1 (5 mer)

(Synthesis of Cbz-MePhe-MeGly-OtBu)

(Condensation reaction)

2.0 g (11.0 mmol) of MeGly-OtBu hydrochloride was suspended in 16 mL of isopropyl acetate and 4 mL of acetonitrile, and 7.7 mL (44.0 mmol) of diisopropyldiethylamine and 3.6 g (11.5 mmol) of Cbz-MePhe-OH were added. The reaction solution was cooled to 0°C, 9.7 mL (16.5 mmol) of a T3P/ethyl acetate solution was added, and then the mixture was stirred at room temperature for 30 minutes to carry out a peptide bond forming reaction (conversion rate: 100%). The reaction conversion rate was determined from the peak area value of LC/MS by isolating 3 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

Then, 1.7 mL (22.0 mmol) of NMI and 20 mL of a 5% aqueous sodium carbonate solution were added, and the mixture was stirred at 50°C for 5 minutes. Stirring was terminated to separate the organic layer and the aqueous layer. The aqueous layer was removed, the remaining organic layer was washed with a 5% aqueous potassium sulfate solution and a 5% aqueous potassium carbonate solution × 2, and then the resulting organic layer was concentrated to give 5.0 g of a concentrate (yield: quant.). This concentrate was subjected to LC/MS analysis to determine the peak area percentage of the intended Cbz-MePhe-MeGly-OtBu (100 area%).
MS (ESI): m/z 441.2 $[M+H]^+$, 463.2 $[M+Na]^+$.

(Synthesis of Cbz-Aze-MePhe-MeGly-OtBu)

(Cbz deprotection reaction)

The entirety of Cbz-MePhe-MeGly-OtBu obtained by the above method was dissolved in 75 mL of isopropyl acetate and subjected to a hydrogenolysis reaction with 0.98 g of 10% Pd/C (3% wet) and hydrogen gas. The mixture was stirred at room temperature for 2 hours to give a Cbz-removed product (conversion rate: 100%). The reaction conversion rate was determined from the peak area value of LC/MS by isolating 3 μL of the reaction solution, diluting the solution with 1.0 mL of methanol, and subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

(Condensation reaction)

The reaction solution was passed through a filter, and toluene was added to carry out azeotropic dehydration. The concentrate was dissolved in 39 mL of isopropyl acetate and 9.7 mL of acetonitrile, and the mixture was cooled to 0°C. 2.6 g (11.0 mmol) of Cbz-Aze-OH, 13.0 mL (22.0 mmol) of a 50% T3P/ethyl acetate solution, and 7.7 mL (44.0 mmol) of diisopropylethylamine were added, and then the mixture was stirred at room temperature for 30 minutes to carry out a peptide bond forming reaction (conversion rate: >99%). The reaction conversion rate was determined from the peak area value of LC/MS by isolating 3 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

Then, 1.7 mL (22.0 mmol) of NMI and 34 mL of a 5% aqueous sodium carbonate solution were added, and the mixture was stirred at 50°C for 5 minutes. Stirring was terminated to separate the organic layer and the aqueous layer. The aqueous layer was removed, the remaining organic layer was washed with 34 mL of a 5% aqueous potassium sulfate solution and 34 mL of a 5% aqueous potassium carbonate solution, and then the resulting organic layer was concentrated to give 5.5 g of a concentrate (yield 96%). This concentrate was subjected to LC/MS analysis to determine

the peak area percentage of the intended Cbz-Aze-MePhe-MeGly-OtBu (99.8 area%). MS (ESI): m/z 546.2 $[M+Na]^+$.

(Synthesis of Cbz-MeAla-Aze-MePhe-MeGly-OtBu/SEQ ID NO: 2)

(Cbz deprotection reaction)

5.5 g (10.6 mmol) of Cbz-Aze-MePhe-MeGly-OtBu obtained by the above method was dissolved in 75 mL of isopropyl acetate and subjected to a hydrogenolysis reaction with 0.95 g of 10% Pd/C (3% wet) and hydrogen gas. The mixture was stirred at 50°C for 2 hours to give a Cbz-removed product (conversion rate: 100%). The reaction conversion rate was determined from the peak area value of LC/MS by isolating 3 μL of the reaction solution, diluting the solution with 1.0 mL of methanol, and subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

(Condensation reaction)

The reaction solution was passed through a filter, and toluene was added to carry out azeotropic dehydration twice. The concentrate was dissolved in 32.8 mL of isopropyl acetate and 8.2 mL of acetonitrile. After 2.7 g (11.1 mmol) of Cbz-MeAla-OH and 7.4 mL (42.3 mmol) of diisopropylethylamine were added, 12.5 mL (21.1 mmol) of a 50% T3P/ethyl acetate solution and 7.4 mL (42.3 mmol) of diisopropylethylamine were added. After the mixture was stirred at room temperature for 2 hours, 0.39 g (1.7 mmol) of Cbz-MeAla-OH, 1.9 mL (3.2 mmol) of a T3P/ethyl acetate solution, and 1.1 mL (6.3 mmol) of diisopropylethylamine were added, and the mixture was further stirred at room temperature for 2 hours to carry out a peptide bond forming reaction (conversion rate: 97%). The reaction conversion rate was determined from the peak area value of LC/MS by isolating 3 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

Then, 1.7 mL (21.1 mmol) of NMI and 41 mL of a 5% aqueous sodium carbonate solution were added, and the mixture was stirred at 50°C for 5 minutes. Stirring was terminated to separate the organic layer and the aqueous layer. The aqueous layer was removed, the remaining organic layer was washed with 41 mL of a 5% aqueous potassium sulfate solution and 41 mL of a 5% aqueous potassium carbonate solution, and then the resulting organic layer was concentrated to give 5.8 g of a concentrate (yield 91%). This concentrate was subjected to LC/MS analysis to determine the peak area percentage of the intended Cbz-MeAla-Aze-MePhe-MeGly-OtBu (SEQ ID NO: 2) (99.5 area%). MS (ESI): m/z 609.3 $[M+H]^+$ 631.3 $[M+Na]^+$.

(Synthesis of Cbz-Ile-MeAla-Aze-MePhe-MeGly-OtBu/SEQ ID NO: 1)

(Cbz deprotection reaction)

5.8 g (9.6 mmol) of Cbz-MeAla-Aze-MePhe-MeGly-OtBu (SEQ ID NO: 2) obtained by the above method was dissolved in 88 mL of isopropyl acetate and subjected to a hydrogenolysis reaction with 0.93 g of 10% Pd/C (3% wet) and hydrogen gas. After being stirred at room temperature for 5 hours, the reaction solution was passed through a filter (conversion rate: 100%). The reaction conversion rate was determined from the peak area value of LC/MS by isolating 3 μL of the reaction solution, diluting the solution with 1.0 mL of methanol, and subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The filtrate was concentrated to give 4.4 g of a concentrate (yield 97%). This concentrate was subjected to LC/MS analysis to determine the peak area percentage of the intended MeAla-Aze-MePhe-MeGly-OtBu (SEQ ID NO: 3) (99.7 area%). MS (ESI): m/z 475.3 $[M+H]^+$.

(Condensation reaction)

1.5 g (3.2 mmol) of the above concentrate and 1.3 g (4.7 mmol) of Cbz-Ile-OH were dissolved in 18 mL of isopropyl acetate and 4.5 mL of acetonitrile. 2.2 mL (12.6 mmol) of diisopropylethylamine and 2.4 g (6.3 mmol) of HATU were added, and the mixture was stirred at room temperature for 30 minutes to carry out a peptide bond forming reaction (conversion rate: >99%). The reaction conversion rate was determined from the peak area value of LC/MS by isolating 3 μL of the reaction solution, diluting the solution with 1.0 mL of methanol, and then subjecting the solution to LC/MS analysis.

Conversion rate (%) = {Compound of interest (area%) / [Starting material (area%) + Compound of interest (area%)]} × 100

Then, 0.75 mL (9.5 mmol) of NMI and 22.5 mL of a 5% aqueous sodium carbonate solution were added, and the mixture was stirred at 50°C for 20 minutes. Stirring was terminated to separate the organic layer and the aqueous layer. The aqueous layer was removed, the remaining organic layer was washed with 22.5 mL × 2 of a 5% aqueous potassium sulfate solution and 22.5 mL × 3 of a 5% aqueous potassium carbonate solution, and then the resulting organic layer was concentrated to give 2.4 g of a concentrate (yield: quant.). This concentrate was subjected to LC/MS analysis to determine the peak area percentage of the intended Cbz-Ile-MeAla-Aze-MePhe-MeGly-OtBu (SEQ ID NO: 1) (99.1 area%). MS (ESI): m/z 744.3 $[M+Na]^+$.

NMI was added to carry out hydrolysis once, the mixture was then aqueously washed, thereby complete removal of the residual C-terminal-activated substance was achieved, and the intended pentapeptide was obtained in a purity of 99.1%. The yield thereof was total 87% from the initial amino acid. This result demonstrates that in continuous liquid phase peptide synthesis, synthesis of a high-purity pentapeptide was achieved in a high yield by completely removing the residual C-terminal-activated substance using an amine additive.

(Example 14)

Synthesis of Cbz-MeAla-MePhe-Leu-MeLeu-Val-MeGly-MeIle-Ser(tBu)-MePhe-MeVal-Asp(tBu)-piperidine/SEQ ID NO: 4 (11 mer)

(Synthesis of Cbz-MeVal-Asp(tBu)-piperidine)

(Condensation reaction)

8.6 g (33.5 mmol) of Asp(tBu)-piperidine was dissolved in 108 mL of cyclopentyl methyl ether. 9.79 g (36.9 mmol) of Cbz-MeVal-OH and 17.6 mL (101 mmol) of diisopropylethylamine were added. After 13.8 g (50.3 mmol) of BEP was dissolved in 21.5 mL of acetonitrile and added to the reaction solution, the mixture was stirred at room temperature for 3 minutes to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

Conversion rate (%) = {Compound of interest (area%) / [Starting material (area%) + Compound of interest (area%)]} × 100

The reaction solution was washed with 150 mL of a 10% aqueous potassium hydrogen sulfate solution, then 150 mL of a 5% aqueous potassium carbonate solution and 9.52 g (101 mmol) of trimethylamine hydrochloride were added, and the mixture was stirred at 40°C for 90 minutes. Stirring was terminated to separate the organic layer and the aqueous layer. The aqueous layer was removed, the remaining organic layer was washed with 150 mL of a 5% aqueous potassium carbonate solution, and then the resulting organic layer was concentrated to give 17 g of a concentrate (yield: quant.). This concentrate was subjected to LC/MS analysis to determine the peak area percentage of the intended Cbz-MeVal-Asp(tBu)-piperidine (99.7 area%).

(Synthesis of Cbz-MePhe-MeVal-Asp(tBu)-piperidine)

(Cbz deprotection reaction)

9.5 g (9.6 mmol) of Cbz-MeVal-Asp(tBu)-piperidine obtained by the above method was dissolved in 50 mL of cyclopentyl methyl ether, and the mixture was subjected to a hydrogenolysis reaction with 1.9 g of 10% Pd/C (3% wet) and hydrogen gas, and stirred at 35°C for 2 hours (conversion rate: 100%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The same operation was repeated, and the combined reaction solution was passed through a filter. The filtrate was concentrated to give 14.0 g of a concentrate (yield: quant.). This concentrate was subjected to LC/MS analysis to determine the peak area percentage of the intended MeVal-Asp(tBu)-piperidine (99.5 area%).

(Condensation reaction)

The above concentrate was dissolved in 126 mL of cyclopentyl methyl ether and 14 mL of acetonitrile. Then, 13.0 g (41.7 mmol) of Cbz-MePhe-OH and 52.9 mL (303 mmol) of diisopropylethylamine were added. 67.0 mL (114 mmol) of a 50% T3P/ethyl acetate solution was added, and the mixture was stirred at room temperature for 1 hour to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The reaction solution was washed with 140 mL of a 5% aqueous potassium hydrogen sulfate solution, then 140 mL of a 5% aqueous potassium carbonate solution and 10.9 g (114 mmol) of trimethylamine hydrochloride were added, and the mixture was stirred at room temperature for 30 minutes. Stirring was terminated to separate the organic layer and the aqueous layer. The aqueous layer was removed, the remaining organic layer was washed with 140 mL of a 5% aqueous potassium carbonate solution, and then the resulting organic layer was concentrated to give 24.1 g of a concentrate (yield 96%). This concentrate was subjected to LC/MS analysis to determine the peak area percentage of the intended Cbz-MePhe-MeVal-Asp(tBu)-piperidine (99.6 area%).

(Synthesis of Cbz-Ser(tBu)-MePhe-MeVal-Asp(tBu)-piperidine/SEQ ID NO: 5)

(Cbz deprotection reaction)

11.5 g (9.6 mmol) of Cbz-MePhe-MeVal-Asp(tBu)-piperidine obtained by the above method was dissolved in 58 mL of cyclopentyl methyl ether, and the mixture was subjected to a hydrogenolysis reaction with 2.3 g of 10% Pd/C and hydrogen gas, and stirred at 35°C for 2 hours (conversion rate: 100%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The same operation was repeated, and the combined reaction solution was passed through a filter. The filtrate was concentrated to give 18.1 g of a concentrate (yield 99%).

(Condensation reaction)

17.3 g (32.6 mmol) of the concentrate was dissolved in 153 mL of cyclopentyl methyl ether and 17 mL of acetonitrile. 10.6 g (35.9 mmol) of Cbz-Ser(tBu)-OH and 45.5 mL (261 mmol) of diisopropylethylamine were added. 57.6 mL (98.0 mmol) of a 50% T3P/ethyl acetate solution was added, and the mixture was stirred at room temperature for 15 minutes to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The reaction solution was washed with 170 mL of a 5% aqueous potassium hydrogen sulfate solution, then 170 mL of a 5% aqueous potassium carbonate solution and 9.4 g (98.0 mmol) of trimethylamine hydrochloride were added, and the mixture was stirred at room temperature for 2 hours. Stirring was terminated to separate the organic layer and the aqueous layer. The aqueous layer was removed, the remaining organic layer was washed with 170 mL of a 5% aqueous potassium carbonate solution, and then the resulting organic layer was concentrated to give 26.5 g of a concentrate (yield: quant.). This concentrate was subjected to LC/MS analysis to determine the peak area percentage of the intended Cbz-Ser(tBu)-MePhe-MeVal-Asp(tBu)-piperidine (SEQ ID NO: 5) (98.9 area%). MS (ESI): 830.4 $[M+Na]^+$.

(Synthesis of Cbz-MeIle-Ser(tBu)-MePhe-MeVal-Asp(tBu)-piperidine/SEQ ID NO: 6)

(Cbz deprotection reaction)

12.0 g (14.9 mmol) of Cbz-Ser(tBu)-MePhe-MeVal-Asp(tBu)-piperidine (SEQ ID NO: 5) was dissolved in 60 mL of cyclopentyl methyl ether, and the mixture was subjected to a hydrogenolysis reaction with 2.4 g of 10% Pd/C and hydrogen gas, and stirred at 35°C for 2 hours (conversion rate: >98%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The same operation was repeated, and the combined reaction solution was passed through a filter. The filtrate was concentrated to give 19.5 g of a concentrate (yield 97%).

(Condensation reaction)

16.0 g (23.7 mmol) of the above concentrate was dissolved in 200 mL of cyclopentyl methyl ether. 7.3 g (26.1 mmol) of Cbz-MeIle-OH and 12.4 mL (71.2 mmol) of diisopropylethylamine were added. After 9.8 g (35.6 mmol) of BEP was dissolved in 40 mL of acetonitrile and added to the reaction solution, the mixture was stirred at room temperature for 5 minutes to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The reaction solution was washed with 240 mL of a 10% aqueous sodium hydrogen sulfate solution, then 240 mL of a 5% aqueous potassium carbonate solution and 6.7 g (71.2 mmol) of trimethylamine hydrochloride were added, and the mixture was stirred at 40°C for 1.5 hours. Stirring was terminated to separate the organic layer and the aqueous layer. The aqueous layer was removed, the remaining organic layer was washed with 240 mL of a 5% aqueous potassium carbonate solution, and then the resulting organic layer was concentrated to give 22.2 g of a concentrate (yield: quant.). This concentrate was subjected to LC/MS analysis to determine the peak area percentage of the intended Cbz-MeIle-Ser(tBu)-MePhe-MeVal-Asp(tBu)-piperidine (SEQ ID NO: 6) (99.4 area%).

(Synthesis of Cbz-MeGly-MeIle-Ser(tBu)-MePhe-MeVal-Asp(tBu)-piperidine/SEQ ID NO: 7) (Cbz deprotection reaction)

9.5 g (10.2 mmol) of Cbz-MeIle-Ser(tBu)-MePhe-MeVal-Asp(tBu)-piperidine (SEQ ID NO: 6) was dissolved in 48 mL of cyclopentyl methyl ether, and the mixture was subjected to a hydrogenolysis reaction with 1.9 g of 10% Pd/C and hydrogen gas, and stirred at 35°C for 2 hours. The same operation was repeated, and the combined reaction solution was passed through a filter. The filtrate was concentrated to give 15.6 g of a concentrate (yield 96%).

(Condensation reaction)

15.3 g (19.1 mmol) of the above concentrate was dissolved in 138 mL of cyclopentyl methyl ether and 15 mL of acetonitrile. 4.7 g (21.0 mmol) of Cbz-MeGly-OH and 26.7 mL (153 mmol) of diisopropylethylamine were added. 33.8 mL (57.3 mmol) of a 50% T3P/ethyl acetate solution was added, and the mixture was stirred at room temperature for 15 minutes to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The reaction solution was washed with 153 mL of a 5% aqueous potassium hydrogen sulfate solution, then 153 mL of a 5% aqueous potassium carbonate solution was added, and the mixture was stirred at room temperature for 5 minutes. Stirring was terminated to separate the organic layer and the aqueous layer, the aqueous layer was removed, then 153 mL of a 5% aqueous potassium carbonate solution was added, and the mixture was stirred at room temperature for 1 hour. After the aqueous layer was removed, the resulting organic layer was concentrated to give 19.5 g of a concentrate (yield: quant.). This concentrate was subjected to LC/MS analysis to determine the peak area percentage of the intended Cbz-MeGly-MeIle-Ser(tBu)-MePhe-MeVal-Asp(tBu)-piperidine (SEQ ID NO: 7) (99.6 area%).

(Synthesis of Cbz-Val-MeGly-MeIle-Ser(tBu)-MePhe-MeVal-Asp(tBu)-piperidine/SEQ ID NO: 8)

(Cbz deprotection reaction)

9.5 g (10.2 mmol) of Cbz-MeGly-MeIle-Ser(tBu)-MePhe-MeVal-Asp(tBu)-piperidine (SEQ ID NO: 7) was dissolved in 48 mL of cyclopentyl methyl ether, and the mixture was subjected to a hydrogenolysis reaction with 1.9 g of 10% Pd/C and hydrogen gas, and stirred at 35°C for 3 hours (conversion rate: 100%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The same operation was repeated, and the combined reaction solution was passed through a filter. The filtrate was concentrated to give 16.3 g of a concentrate (yield 99%).

(Condensation reaction)

16.0 g (18.4 mmol) of the above concentrate was dissolved in 144 mL of cyclopentyl methyl ether and 16 mL of acetonitrile. 5.1 g (20.2 mmol) of Cbz-Val-OH and 25.6 mL (147 mmol) of diisopropylethylamine were added. 32.4 mL (55.0 mmol) of a 50% T3P/ethyl acetate solution was added, and the mixture was stirred at room temperature for 30 minutes to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The reaction solution was washed with 160 mL of a 5% aqueous potassium hydrogen sulfate solution, then 153 mL of a 5% aqueous potassium carbonate solution and 5.3 g (55.0 mmol) of trimethylamine hydrochloride were added, and the mixture was stirred at 60°C for 1 hour. Stirring was terminated to separate the organic layer and the aqueous layer, the aqueous layer was removed, and then the resulting organic layer was washed with 160 mL of a 5% aqueous potassium carbonate solution and concentrated to give 20.0 g of a concentrate (yield 99%). This concentrate was subjected to LC/MS analysis to determine the peak area percentage of the intended Cbz-Val-MeGly-MeIle-Ser(tBu)-MePhe-MeVal-Asp(tBu)-piperidine (SEQ ID NO: 8) (99.6 area%).

(Synthesis of Cbz-MeLeu-Val-MeGly-MeIle-Ser(tBu)-MePhe-MeVal-Asp(tBu)-piperidine/SEQ ID NO: 9)

(Cbz deprotection reaction)

9.2 g (8.3 mmol) of Cbz-Val-MeGly-MeIle-Ser(tBu)-MePhe-MeVal-Asp(tBu)-piperidine (SEQ ID NO: 8) was dissolved in 46 mL of cyclopentyl methyl ether, and the mixture was subjected to a hydrogenolysis reaction with 1.8 g of 10% Pd/C and hydrogen gas, and stirred at 35°C for 6 hours and further at 45°C for 4 hours (conversion rate: 100%). The reaction conversion rate was determined from the peak area value of LC/MS by isolating 5 μL of the reaction solution, diluting the solution with 1.0 mL of methanol, and then subjecting the solution to LC/MS analysis.

Conversion rate (%) = {Compound of interest (area%) / [Starting material (area%) + Compound of interest (area%)]} × 100

The same operation was repeated, and the combined reaction solution was passed through a filter. The filtrate was concentrated to give 15.9 g of a concentrate (yield 98%). This concentrate was subjected to LC/MS analysis to determine the peak area percentage of the intended Val-MeGly-MeIle-Ser(tBu)-MePhe-MeVal-Asp(tBu)-piperidine (SEQ ID NO: 10) (97.9 area%).

(Condensation reaction)

14.5 g (14.9 mmol) of the above concentrate was dissolved in 181 mL of cyclopentyl methyl ether. 4.6 g (16.4 mmol) of Cbz-MeLeu-OH and 7.8 mL (44.8 mmol) of diisopropylethylamine were added. After 4.9 g (17.9 mmol) of BEP was dissolved in 36 mL of acetonitrile, the resulting BEP solution was added to the reaction solution, and the mixture was then stirred at 40°C for 1 minute to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

Conversion rate (%) = {Compound of interest (area%) / [Starting material (area%) + Compound of interest (area%)]} × 100

The reaction solution was washed with 128 mL of a 10% aqueous sodium hydrogen sulfate solution, then 128 mL of a 5% aqueous potassium carbonate solution and 4.2 g (44.8 mmol) of trimethylamine hydrochloride were added, and the mixture was stirred at 40°C for 30 minutes. Stirring was terminated to separate the organic layer and the aqueous layer, the aqueous layer was removed, and then the resulting organic layer was washed with 128 mL of a 5% aqueous potassium carbonate solution and concentrated to give 18.0 g of a concentrate (yield 98%). This concentrate was subjected to LC/MS analysis to determine the peak area percentage of the intended Cbz-MeLeu-Val-MeGly-MeIle-Ser(tBu)-MePhe-MeVal-Asp(tBu)-piperidine (SEQ ID NO: 9) (96.0 area%).

(Synthesis of Cbz-Leu-MeLeu-Val-MeGly-MeIle-Ser(tBu)-MePhe-MeVal-Asp(tBu)-piperidine/SEQ ID NO: 11)

(Cbz deprotection reaction)

8.0 g (6.5 mmol) of Cbz-MeLeu-Val-MeGly-MeIle-Ser(tBu)-MePhe-MeVal-Asp(tBu)-piperidine (SEQ ID NO: 9) was dissolved in 40 mL of cyclopentyl methyl ether, and the mixture was subjected to a hydrogenolysis reaction with 1.6 g of 10% Pd/C and hydrogen gas, and stirred at 45°C for 4 hours (conversion rate: 100%). The reaction conversion rate was determined from the peak area value of LC/MS by isolating 5 μL of the reaction solution, diluting the solution with 1.0 mL of methanol, and then subjecting the solution to LC/MS analysis.

Conversion rate (%) = {Compound of interest (area%) / [Starting material (area%) + Compound of interest (area%)]} × 100

The same operation was repeated, and the combined reaction solution was passed through a filter. The filtrate was concentrated to give 14.3 g of a concentrate (yield: quant.). This concentrate was subjected to LC/MS analysis to determine the peak area percentage of the intended MeLeu-Val-MeGly-MeIle-Ser(tBu)-MePhe-MeVal-Asp(tBu)-piperidine (SEQ ID NO: 12) (95.8 area%). MS (ESI): m/z 1098.6 $[M+H]^+$.

(Condensation reaction)

13.0 g (11.8 mmol) of the above concentrate was dissolved in 117 mL of cyclopentyl methyl ether and 13 mL of acetonitrile. 3.5 g (13.0 mmol) of Cbz-Leu-OH and 16.5 mL (95.0 mmol) of diisopropylethylamine were added. 20.9 mL (35.5 mmol) of a 50% T3P/ethyl acetate solution was added to the reaction solution, and the mixture was stirred at room temperature for 30 minutes to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

Conversion rate (%) = {Compound of interest (area%) / [Starting material (area%) + Compound of interest (area%)]} × 100

The reaction solution was washed with 130 mL of a 5% aqueous potassium hydrogen sulfate solution, then 130 mL of a 5% aqueous potassium carbonate solution and 3.4 g (35.5 mmol) of trimethylamine hydrochloride were added, and the mixture was stirred at 60°C for 45 minutes. Stirring was terminated to separate the organic layer and the aqueous layer, the aqueous layer was removed, and then the resulting organic layer was washed with 130 mL of a 5% aqueous potassium carbonate solution and concentrated to give 15.6 g of a concentrate (yield 98%). This concentrate was subjected to LC/MS analysis to determine the peak area percentage of the intended Cbz-Leu-MeLeu-Val-MeGly-MeIle-Ser(tBu)-MePhe-MeVal-Asp(tBu)-piperidine (SEQ ID NO: 11) (97.2 area%).

(Synthesis of Cbz-MePhe-Leu-MeLeu-Val-MeGly-MeIle-Ser(tBu)-MePhe-MeVal-Asp(tBu)-piperidine/SEQ ID NO: 12)

(Cbz deprotection reaction)

10.0 g (7.4 mmol) of Cbz-Leu-MeLeu-Val-MeGly-MeIle-Ser(tBu)-MePhe-MeVal-Asp(tBu)-piperidine (SEQ ID NO: 11) was dissolved in 50 mL of cyclopentyl methyl ether, and the mixture was subjected to a hydrogenolysis reaction with 2.0 g of 10% Pd/C and hydrogen gas, and stirred at 45°C for 4 hours (conversion rate: 100%). After the reaction solution was passed through a filter, the filtrate was concentrated to give 8.9 g of a concentrate (yield 99%). The reaction conversion rate was determined from the peak area value of LC/MS by isolating 5 μL of the reaction solution, diluting the solution with 1.0 mL of methanol, and then subjecting the solution to LC/MS analysis. MS (ESI): m/z 1211.7 $[M+H]^+$, 1233.7 $[M+Na]^+$.

Conversion rate (%) = {Compound of interest (area%) / [Starting material (area%) + Compound of interest (area%)]} × 100

(Condensation reaction)

7.0 g (5.8 mmol) of the above concentrate was dissolved in 87.5 mL of cyclopentyl methyl ether. 2.0 g (6.4 mmol) of Cbz-MePhe-OH and 3.0 mL (17.3 mmol) of diisopropylethylamine were added. After 1.9 g (17.9 mmol) of BEP was dissolved in 17.5 mL of acetonitrile, the resulting BEP solution was added to the reaction solution, and the mixture was then stirred at room temperature for 3 minutes to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

Conversion rate (%) = {Compound of interest (area%) / [Starting material (area%) + Compound of interest (area%)]} × 100

The reaction solution was washed with 105 mL of a 10% aqueous sodium hydrogen sulfate solution, then 105 mL of a 5% aqueous potassium carbonate solution and 1.7 g (17.3 mmol) of trimethylamine hydrochloride were added, and the mixture was stirred at 40°C for 30 minutes. Stirring was terminated to separate the organic layer and the aqueous layer, the aqueous layer was removed, and then the resulting organic layer was washed with 105 mL of a 5% aqueous potassium carbonate solution and concentrated to give 8.6 g of a concentrate (yield 99%). This concentrate was subjected to LC/MS analysis to determine the peak area percentage of the intended Cbz-MePhe-Leu-MeLeu-Val-MeGly-MeIle-Ser(tBu)-MePhe-MeVal-Asp(tBu)-piperidine (SEQ ID NO: 12) (97.0 area%).

(Synthesis of Cbz-MeAla-MePhe-Leu-MeLeu-Val-MeGly-MeIle-Ser(tBu)-MePhe-MeVal-Asp(tBu)-piperidine/SEQ ID NO: 4)

(Cbz deprotection reaction)

7.6 g (5.0 mmol) of Cbz-MePhe-Leu-MeLeu-Val-MeGly-MeIle-Ser(tBu)-MePhe-MeVal-Asp(tBu)-piperidine (SEQ ID NO: 12) was dissolved in 38 mL of cyclopentyl methyl ether, and the mixture was subjected to a hydrogenolysis reaction with 2.0 g of 10% Pd/C and hydrogen gas, and stirred at 45°C for 4 hours (conversion rate: 100%). After the reaction solution was passed through a filter, the filtrate was concentrated to give 6.8 g of a concentrate (yield 98%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

(Condensation reaction)

500 mg (0.4 mmol) of the above concentrate was dissolved in 4.5 mL of cyclopentyl methyl ether and 0.5 mL of acetonitrile. 95.0 mg (0.4 mmol) of Cbz-MeAla-OH and 509 μL (2.9 mmol) of diisopropylethylamine were added. 644 μL (1.1 mmol) of a 50% T3P/ethyl acetate solution was added, and the mixture was stirred at room temperature for 2 hours to carry out a peptide bond forming reaction (conversion rate: >99%). The reaction conversion rate was determined from the peak area value of LC/MS by isolating 5 μL of the reaction solution, diluting the solution with 1.0 mL of methanol, and then subjecting the solution to LC/MS analysis.

$$\text{Conversion rate (\%)} = \{\text{Compound of interest (area\%)} / [\text{Starting material (area\%)} + \text{Compound of interest (area\%)}]\} \times 100$$

The reaction solution was washed with 5.0 mL of a 10% aqueous sodium hydrogen sulfate solution, then 5.0 mL of a 5% aqueous potassium carbonate solution and 104 mg (1.1 mmol) of trimethylamine hydrochloride were added, and the mixture was stirred at room temperature for 1 hour. Stirring was terminated to separate the organic layer and the aqueous layer, the aqueous layer was removed, and then the resulting organic layer was washed with 5.0 mL of a 5% aqueous potassium carbonate solution and concentrated to give 555 mg of a concentrate (yield 96%, Cbz-MeAla-MePhe-Leu-MeLeu-Val-MeGly-MeIle-Ser(tBu)-MePhe-MeVal-Asp(tBu)-piperidine (SEQ ID NO: 4) was 95.3 area%). MS (ESI): m/z 1591.9 $[M+H]^+$, 1613.9 $[M+Na]^+$.

Trimethylamine was added to carry out hydrolysis once, then the mixture was aqueously washed, thereby complete removal of the residual C-terminal-activated substance was achieved, and it was thus possible to obtain a peptide composed of 11 amino acids in a high purity of 95.3%. The yield thereof was total 75.3% from the initial amino acid. This result demonstrates that in continuous liquid phase peptide synthesis, synthesis of a high-purity polypeptide can be achieved by removing the residual C-terminal-activated substance using an amine additive.

(Example 15) Synthesis of Teoc-MeLeu-Phe-OtBu

(Synthesis of Teoc-MeLeu-Opfp)

2.35 g (16.2 mmol) of MeLeu-OH was dissolved in 23.5 mL of 1,4-dioxane, and then 4.61 g (17.8 mmol) of Teoc-OSu, 23.5 mL of water, and 4.5 mL (32.4 mmol) of triethylamine were added. The mixture was stirred at room temperature for 1 hour to carry out a Teoc introduction reaction. The reaction solution was acidified by adding a 5% aqueous potassium hydrogen sulfate solution and then extracted with 50 mL of ethyl acetate, and the organic layer was

washed with saturated brine. The resulting organic layer was concentrated to dryness, and the concentrate was dissolved in 30 mL of dichloromethane. 3.10 g (16.2 mmol) of Pfp-OH and 4.53 g (24.3 mmol) of EDC hydrochloride were added, and the mixture was stirred at room temperature for 30 minutes to carry out a Pfp introduction reaction. After the reaction solution was washed with saturated brine, the aqueous layer was extracted with 50 mL of ethyl acetate. The combined organic layers were concentrated, and the resulting concentrate was purified by column chromatography (ethyl acetate/heptane) to give 6.63 g of Teoc-MeLeu-OPfp (yield: 90%).

(Condensation reaction)

201 mg (0.8 mmol) of Phe-OtBu hydrochloride and 536 mg (1.2 mmol) of Teoc-MeLeu-OPfp were suspended in 3.0 mL of isopropyl acetate, 257 μL (2.3 mmol) of 4-methylmorpholine was added, and the mixture was stirred at 25°C for 3 hours to carry out a peptide bond forming reaction. Then, 5 μL of the reaction solution was isolated and added to 100 μL (1.2 mmol) of propylamine to convert the residual C-terminal-activated substance to propylamide, and then the mixture was diluted with 0.9 mL of methanol. This solution was subjected to LC/MS analysis to determine the conversion rate from the peak area value of LC/MS (conversion rate: 100%.

Conversion rate (%) = {Teoc-MeLeu-Phe-OtBu (area%) / [Phe-OtBu (area%) + Teoc-MeLeu-Phe-OtBu (area%)]} × 100

(Hydrolysis treatment)

(1) When no amine was added

To the peptide-containing reaction solution prepared above, 2.0 mL of a 5% aqueous sodium carbonate solution was added, and the mixture was stirred with a stir bar at 25°C for 20 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

Residual rate (%) of C-terminal-activated substance = {Propylamide (area%) / [Propylamide (area%) + Dipeptide (area%)]} × 100

(2) When amine was added

To the peptide-containing reaction solution prepared above, 95 mg (0.8 mmol) of DMAP and 2.0 mL of a 5% aqueous sodium carbonate solution were added, and the mixture was stirred with a stir bar at 25°C for 20 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

Residual rate (%) of C-terminal-activated substance = {Propylamide (area%) / [Propylamide (area%) + Dipeptide (area%)]} × 100

(Work-up)

After stirring was terminated, the mixture was left to stand still to separate the organic layer and the aqueous layer, and the aqueous layer was removed. Then, the organic layer was sequentially washed with 2 mL × 2 of a 10% aqueous potassium hydrogen sulfate solution and 2 mL of a 5% aqueous sodium carbonate solution. Moreover, washing with 1 mL of a 5% aqueous potassium carbonate solution and 1 mL × 2 of common water was repeated 3 times. 5 μL of the resulting organic layer was isolated and added to 100 μL (1.2 mmol) of propylamine to convert the residual C-terminal-activated substance to propylamide, the mixture was then diluted with 0.9 mL of methanol and subjected to LC/MS analysis to determine the peak areas of the peptide of interest and the residual C-terminal-activated substance

(a converting substance to propylamide). The concentrate (peptide) obtained by hydrolysis without addition of amine was 576.6 mg (yield 150%: calculated as solely containing peptide although the concentrate contained impurities (residual C-terminal-activated substance). The concentrate obtained by hydrolysis with addition of amine was 369.6 mg (yield 96%). MS (ESI): m/z 437.3 [M-tBu+H]+, 493.3 [M+H]+, 515.3 [M+Na]+.

[Table 9]

| entry | | Immediately after hydrolysis treatment | Organic layer after liquid separation operation | | After concentration |
|---|---|---|---|---|---|
| | Amine | Residual rate of C-terminal-activated substance (%) | Dipeptide (%)[1] | Residual C-terminal-activated substance (%)[1] (converting substance to propylamide) | Yield (%) |
| 1 | DMAP | 0 | 96.3 | nd | 96 |
| 2 | non | 9.1 | 90.0 | 8.9 | 150 |
| 1) Peak area ratio of LCMS | | | | | |

Hydrolysis treatment with alkaline water alone of a residual C-terminal-activated substance in which the protecting group was Teoc, and the C-terminal-activated substance moiety was Pfp, did not completely hydrolyze the residual C-terminal-activated substance, and it was also not possible to remove the residual C-terminal-activated substance by the subsequent aqueous washing. On the other hand, it was found that when hydrolysis was performed with addition of DMAP, hydrolysis of the residual C-terminal-activated substance was completely achieved, and complete removal of the residual C-terminal-activated substance was also possible. The dipeptide of interest was obtained in a purity of 96.3% (yield 96%).

(Example 16) Synthesis of Cbz-Aib-MeLeu-Phe-OtBu

(Teoc deprotection reaction using dipeptide obtained by hydrolysis treatment without addition of amine)

576.6 mg of Teoc-MeLeu-Phe-OtBu (containing 8.9 area% of residual C-terminal-activated substance) synthesized under the amine-free condition of Example 15 was dissolved in 2.0 mL of 2-methyltetrahydrofuran. After 1.5 mL (1.5 mmol) of an 8.4% hydrous tetrahydrofuran solution of TBAF was added, the mixture was stirred at 50°C for 2.5 hours. Since the reaction did not complete, 0.75 mL (0.75 mmol) of an 8.4% hydrous tetrahydrofuran solution of TBAF was added, and the mixture was stirred for 2.5 hours. Further, 0.75 mL (0.75 mmol) of an 8.4% hydrous tetrahydrofuran solution of TBAF was added, and the mixture was stirred for 30 minutes to give a Teoc-removed product MeLeu-Phe-OtBu (conversion rate: 100%). The reaction conversion rate was determined from the peak area value of LC/MS by isolating 5 μL of the reaction solution, diluting the solution with 1.0 mL of acetonitrile, and then subjecting the solution to LC/MS analysis.

Conversion rate (%) = {MeLeu-Phe-OtBu (area%) / [Teoc-MeLeu-Phe-OtBu (area%) + MeLeu-Phe-OtBu (area%)]} × 100

(Condensation reaction)

After concentration until the content volume was about 1 mL, 2 mL of 2-methyltetrahydrofuran was added. This operation was repeated two more times, and 0.5 mL of acetonitrile, 276 mg (1.1 mmol) of Cbz-Aib-OH, and 0.66 mL (3.8 mmol) of diisopropylethylamine were added to the resulting 2-methyltetrahydrofuran solution. Then, 441 mg (1.1 mmol) of HATU was added at 25°C, and the mixture was stirred at room temperature for 14 hours. Then, 579 mg (1.5 mmol) of HATU was added, the mixture was stirred at 40°C for 1 hour, then 684 mg (1.8 mmol) of HATU was added, the temperature was raised to 60°C, and the mixture was stirred for 4.5 hours. Moreover, 455 mg (1.1 mmol) of HATU was added, and the mixture was stirred at 60°C for 2 hours, at room temperature for 12 hours, and at 60° C for 2 hours, but no progress of a condensation reaction was observed (conversion rate 0%). The reaction conversion rate was determined from the peak area value of LC/MS by adding 5 μL of the reaction solution to 100 μL of propylamine, diluting the mixture with 0.9 mL of methanol, and then subjecting the mixture to LC/MS analysis.

$$\text{Conversion rate (\%)} = \{\text{Cbz-Aib-MeLeu-Phe-OtBu (area\%)} / [\text{MeLeu-Phe-OtBu (area\%)} + \text{Cbz-Aib-MeLeu-Phe-OtBu (area\%)}]\} \times 100$$

(Teoc deprotection reaction using dipeptide obtained by hydrolysis treatment with addition of amine)

369.6 mg (0.75 mmol) of Teoc-MeLeu-Phe-OtBu synthesized under the amine-added condition of Example 15 was dissolved in 2.0 mL of 2-methyltetrahydrofuran. After 1.5 mL (1.5 mmol) of an 8.4% hydrous tetrahydrofuran solution of TBAF was added, the mixture was stirred at 50°C for 2.5 hours to give a Teoc-removed product MeLeu-Phe-OtBu (conversion rate: 100%). The reaction conversion rate was determined from the peak area value of LC/MS by isolating 5 μL of the reaction solution, diluting the solution with 1.0 mL of acetonitrile, and then subjecting the solution to LC/MS analysis.

$$\text{Conversion rate (\%)} = \{\text{MeLeu-Phe-OtBu (area\%)} / [\text{Teoc-MeLeu-Phe-OtBu (area\%)} + \text{MeLeu-Phe-OtBu (area\%)}]\} \times 100$$

(Condensation reaction)

After concentration until the content volume was about 1 mL, 2 mL of 2-methyltetrahydrofuran was added. This operation was repeated two more times, and 0.5 mL of acetonitrile, 273 mg (1.1 mmol) of Cbz-Aib-OH, and 0.66 mL (3.8 mmol) of diisopropylethylamine were added to the resulting 2-methyltetrahydrofuran solution. Then, 439 mg (1.1 mmol) of HATU was added at 25°C, and the mixture was stirred at room temperature for 14 hours. 576 mg (1.5 mmol) of HATU was added, and the mixture was stirred at 40°C for 5.5 hours. Moreover, 452 mg (1.1 mmol) of HATU was added, and the mixture was stirred at 60°C for 2 hours, at room temperature for 12 hours, and at 60° C for 2 hours (conversion rate 86%). The reaction conversion rate was determined from the peak area value of LC/MS by adding 5 μL of the reaction solution to 100 μL of propylamine, diluting the mixture with 0.9 mL of methanol, and then subjecting the mixture to LC/MS analysis.

$$\text{Conversion rate (\%)} = \{\text{Cbz-Aib-MeLeu-Phe-OtBu (area\%)} / [\text{MeLeu-Phe-OtBu (area\%)} + \text{Cbz-Aib-MeLeu-Phe-OtBu (area\%)}]\} \times 100$$

To the prepared reaction solution, 92 mg (0.75 mmol) of DMAP and 4.0 mL of a 10% aqueous potassium carbonate solution were added, and the mixture was stirred with a stir bar at 25°C for 1 hour. After stirring was terminated, the mixture was left to stand still to separate the organic layer and the aqueous layer, and the aqueous layer was removed. 5 μL of the resulting organic layer was added to 100 μL of propylamine, and the mixture was diluted with 0.9 mL of methanol. This solution was subjected to LC/MS analysis to determine the peak area percentages of the peptide of interest and the residual C-terminal-activated substance. The peptide of interest Cbz-Aib-MeLeu-Phe-OtBu was 86.7%, the starting material MeLeu-Phe-OtBu was 13.3%, and Cbz-Aib-NHPr derived from the residual C-terminal-activated substance was not detected. The remaining organic layer was concentrated to give 295.6 mg of a concentrate having the above composition. MS (ESI): m/z 568.4 [M+H]+, 590.4 [M+Na]+.

It was found that, with the C-terminal-activated substance remaining in a solution of a peptide having a protected N-terminal, a large excess of a reagent was required when removing the N-terminal protecting group (Teoc) of the peptide with a hydrous fluorine reagent. This is presumably because the deprotecting reagent also reacts with the residual C-terminal-activated substance.

Also, it was revealed that the condensation reaction (a peptide bond forming reaction) with another C-terminal-activated substance in the next step subsequent to deprotection did not proceed at all. This is presumably because the excessive reagent used in the previous step (N-terminal deprotection) degraded the C-terminal-activated substance. Accordingly, it was found that, with the C-terminal-activated substance remaining, a large excess of a reagent was required when deprotecting the N-terminal, which leads to interfering the progress of the subsequent condensation reaction. On the other hand, it was found that when a peptide solution from which the residual C-terminal-activated substance was completely removed was used as a starting material, the deprotection reaction completed with a suitable amount of a deprotecting reagent, thus enabling the condensation reaction of the next step to be carried out.

(Example 17) Synthesis of Cbz-MeAla-Phe-OtBu

(Condensation reaction)

302 mg (1.2 mmol) of Phe-OtBu hydrochloride, 417 mg (1.7 mmol) of Cbz-MeAla-OH, and 290 mg (1.8 mmol) of HOOBt were suspended in 0.9 mL of acetonitrile and 3.6 mL of MTBE, and 1.0 mL (5.8 mmol) of diisopropylethylamine was added. Then, 443 mg (2.3 mmol) of EDC hydrochloride was added, and the mixture was stirred at 25°C for 30 minutes to carry out a peptide bond forming reaction. 5 μL of the reaction solution was isolated and added to 100 μL (1.2 mmol) of propylamine to convert the residual C-terminal-activated substance to propylamide, and then the mixture was diluted with 0.9 mL of methanol. This solution was subjected to LC/MS analysis to determine the conversion rate from the peak area value of LC/MS (conversion rate: 100%).

Conversion rate (%) = {Cbz-MeAla-Phe-OtBu (area%) / [Phe-OtBu (area%) + Cbz-MeAla-Phe-OtBu (area%)]} × 100

(Hydrolysis treatment)

(1) When no amine was added

To the peptide-containing reaction solution prepared above, 3.0 mL of a 5% aqueous sodium carbonate solution was added, and the mixture was stirred with a stir bar at 25°C for 5 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

Residual rate (%) of C-terminal-activated substance = {Propylamide (area%) / [Propylamide (area%) + Dipeptide (area%)]} × 100

(2) When amine was added

To the peptide-containing reaction solution prepared above, 147 mg (1.1 mmol) of DMAP and 3.0 mL of a 5% aqueous sodium carbonate solution were added, and the mixture was stirred with a stir bar at 25°C for 5 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

Residual rate (%) of C-terminal-activated substance = {Propylamide (area%) / [Propylamide (area%) + Dipeptide (area%)]} × 100

(Work-up)

After stirring was terminated, the mixture was left to stand still to separate the organic layer and the aqueous layer, and the aqueous layer was removed. The organic layer was sequentially washed with 3 mL × 2 of a 10% aqueous sodium hydrogen sulfate solution and 3 mL of a 5% aqueous sodium carbonate solution. 5 μL of the resulting organic layer was added to 100 μL of propylamine, and the mixture was diluted with 0.9 mL of methanol. This solution was subjected to LC/MS analysis to determine the peak area percentages of the peptide of interest and the residual C-terminal-activated substance. MS (ESI): m/z 385.2 [M-tBu+H]+, 441.3 [M+H]+, 463.2 [M+Na]+.

[Table 10]

| entry | | Immediately after hydrolysis treatment | Organic layer after liquid separation operation | |
|---|---|---|---|---|
| | Amine | Residual rate of C-terminal-activated substance (%) | Dipeptide (%)[1] | Residual C-terminal-activated substance (%)[1] (converting substance to propylamide) |
| 1 | DMAP | 0 | 98.4 | nd |
| 2 | non | 17.0 | 83.8 | 13.8 |
| 1) Peak area ratio of LCMS | | | | |

Also with alanine, which is an amino acid having a small substituent, hydrolysis treatment with alkaline water alone did not completely hydrolyze the residual C-terminal-activated substance, and it was also not possible to sufficiently remove the residual C-terminal-activated substance by the subsequent separation operation (aqueous washing). On the other hand, it was found that when hydrolysis was performed with addition of DMAP, hydrolysis of the residual C-terminal-activated substance was completely achieved, and thus the residual C-terminal-activated substance could also be completely removed. Moreover, at this time, the dipeptide of interest was obtained in a purity of 98.4%.

(Example 18) Synthesis of Cbz-Hph-MeAla-Phe-OtBu

(Cbz deprotection reaction using dipeptide obtained by hydrolysis treatment without addition of amine)

An MTBE solution of Cbz-MeAla-Phe-OtBu synthesized under the amine-free condition of Example 17 was concentrated and replaced with 2-methyltetrahydrofuran. The solution was subjected to a hydrogenolysis reaction with 101 mg of 5% Pd/C (50% wet) and hydrogen gas. Despite stirring at 25°C for 6 hours, the reaction did not complete (conversion rate 35%). The reaction conversion rate was determined from the peak area value of LC/MS by isolating 5 μL of the reaction solution, diluting the solution with 1.0 mL of acetonitrile, and then subjecting the solution to LC/MS analysis.

$$\text{Conversion rate (\%)} = \{\text{MeAla-Phe-OtBu (area\%)} / [\text{Cbz-MeAla-Phe-OtBu (area\%)} + \text{MeAla-Phe-OtBu (area\%)}]\} \times 100$$

(Cbz deprotection reaction using dipeptide obtained by hydrolysis treatment with addition of amine)

An MTBE solution of Cbz-MeAla-Phe-OtBu synthesized under the amine-added condition of Example 17 was concentrated and replaced with 2-methyltetrahydrofuran. The mixture was subjected to a hydrogenolysis reaction with 102 mg of 5% Pd/C (50% wet) and hydrogen gas. The mixture was stirred at 25°C for 3 hours to give a Cbz-removed product MeAla-Phe-OtBu (conversion rate 100%). The reaction conversion rate was determined from the peak area value of LC/MS by isolating 5 μL of the reaction solution, diluting the solution with 1.0 mL of acetonitrile, and then subjecting the solution to LC/MS analysis. MS (ESI): m/z 307.2 [M+H]+.

$$\text{Conversion rate (\%)} = \{\text{MeAla-Phe-OtBu (area\%)} / [\text{Cbz-MeAla-Phe-OtBu (area\%)} + \text{MeAla-Phe-OtBu (area\%)}]\} \times 100$$

(Condensation reaction)

A reaction solution of a Cbz-removed product of dipeptide obtained by hydrolysis treatment with addition of amine was passed through a filter to filter off Pd/C, and then concentrated to dryness. The dried residue was dissolved in 2.5 mL of 2-methyltetrahydrofuran, and 474 mg (1.5 mmol) of Cbz-Hph-OH and 610 μL (3.5 mmol) of diisopropylethylamine were added. Then, 1.37 mL (2.33 mmol) of a T3P/2-methyltetrahydrofuran solution was added, and the mixture was stirred at 25°C for 1.5 hours to carry out a peptide bond forming reaction (conversion rate: 100%). The reaction conversion rate was determined from the peak area value of LC/MS by adding 5 μL of the reaction solution to 100 μL of propylamine, diluting the mixture with 0.9 mL of methanol, and then subjecting the solution to LC/MS analysis.

$$\text{Conversion rate (\%)} = \{\text{Cbz-Hph-MeAla-Phe-OtBu (area\%)} / [\text{MeAla-Phe-OtBu (area\%)} + \text{Cbz-Hph-MeAla-Phe-OtBu (area\%)}]\} \times 100$$

To the prepared reaction solution, 74 mg (0.6 mmol) of DMAP and 3.0 mL of a 5% aqueous sodium carbonate solution were added, and the mixture was stirred at 25°C for 15 minutes. After stirring was terminated and the mixture was left to stand still, the organic layer and the aqueous layer were separated, and the aqueous layer was removed. The organic layer was sequentially washed with 3 mL of a 10% aqueous sodium hydrogen sulfate solution, 3 mL of a 5% aqueous sodium carbonate solution, and 3 mL of common water. 5 μL of the resulting organic layer was added to 100 μL of propylamine, and the mixture was diluted with 0.9 mL of methanol. This solution was subjected to LC/MS analysis to determine the peak area percentages of the peptide of interest and the residual C-terminal-activated substance. The peptide of interest Cbz-Hph-MeAla-Phe-OtBu was 99.0%, and Cbz-Hph-NHPr derived from the residual C-terminal-activated substance was not detected. The remaining organic layer was concentrated to give 618.4 mg of a concentrate (yield 88% from Phe-OtBu of Example 17). MS (ESI): m/z 602.4 [M+H]+, 624.4 [M+Na]+.

It was revealed that when a C-terminal-activated substance derived from EDC and HOOBt remained, the Cbz deprotection reaction after peptide elongation barely proceeds. On the other hand, it was found that when a peptide solution completely free of a residual C-terminal-activated substance was used as a starting material, the Cbz deprotection reaction proceeded smoothly, thus enabling a peptide synthesis reaction. That is to say, it was found that, as in the case of Example 9, the use of the method of the present invention enabled the reductive removal reaction of the N-terminal protecting group of the produced peptide compound to proceed without stagnation.

(Example 19) Synthesis of Cbz-Aib-D-Val-OBn

(Condensation reaction)

502 mg (1.3 mmol) of D-Val-OBn TsOH salt and 478 mg (2.0 mmol) of Cbz-Aib-OH were suspended in 6.0 mL of 2-MeTHF, and 1.2 mL (6.9 mmol) of diisopropylethylamine was added. Then, 1.9 mL (3.3 mmol) of a 50% T3P/2-methyltetrahydrofuran solution was added at 25°C, and the mixture was stirred at 25°C for 15 hours to carry out a peptide bond forming reaction. 5 μL of the reaction solution was isolated and added to 100 μL (1.2 mmol) of propylamine to convert the residual C-terminal-activated substance to propylamide, and then the mixture was diluted with 0.9 mL of methanol. This solution was subjected to LC/MS analysis to determine the conversion rate from the peak area of LC/MS (conversion rate: 100%).

$$\text{Conversion rate (\%)} = \{\text{Cbz-Aib-D-Val-OBn (area\%)} / [\text{D-Val-OBn (area\%)} + \text{Cbz-Aib-D-Val-OBn (area\%)}]\} \times 100$$

(Hydrolysis treatment)

(1) When no amine was added

To the peptide-containing reaction solution prepared above, 5.0 mL of a 5% aqueous potassium carbonate solution was added, and the mixture was stirred with a stir bar at 25°C for 30 minutes. Stirring was terminated to separate the organic layer and the aqueous layer. Then, 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(1) When amine was added

To the peptide-containing reaction solution prepared above, 484 mg (4.0 mmol) of DMAP and 5.0 mL of a 5% aqueous potassium carbonate solution were added, and the mixture was stirred with a stir bar at 25°C for 30 minutes. Stirring was terminated to separate the organic layer and the aqueous layer. 5 μL of the organic layer was isolated and

added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(Work-up)

After stirring was terminated, the mixture was left to stand still to separate the organic layer and the aqueous layer, and the aqueous layer was removed. The organic layer was sequentially washed with 5 mL of a 10% aqueous potassium hydrogen sulfate solution and 2.5 mL of a 5% aqueous potassium carbonate solution. 5 μL of the organic layer was isolated and added to 100 μL (1.2 mmol) of propylamine to convert the residual C-terminal-activated substance to propylamide, and then the mixture was diluted with 0.9 mL of methanol and subjected to LC/MS analysis to determine the peak area values of the peptide of interest and the residual C-terminal-activated substance (a converting substance to propylamide). The remaining organic layer was concentrated to give a peptide. The concentrate (peptide) obtained by hydrolysis without addition of amine was 653.8 mg (yield 116%: calculated as solely containing peptide although the concentrate contained impurities (residual C-terminal-activated substance)). The concentrate obtained by hydrolysis with addition of amine was 549.4 mg (yield 97%). MS (ESI): m/z 427.3 $[M+H]^+$, 449.2 $[M+Na]^+$.

[Table 11]

| entry | | Immediately after hydrolysis treatment | Organic layer after liquid separation operation | | After concentration |
|---|---|---|---|---|---|
| | Amine | Residual rate of C-terminal-activated substance (%) | Dipeptide (%) | Residual C-terminal-activated substance (%)[1] (converting substance to propylamide) | Yield (%) |
| 1 | DMAP | 0 | 98.6 | nd | 97 |
| 2 | none | 16.2 | 83.4 | 13.7 | 116 |
| 1) Peak area ratio of LCMS | | | | | |

By hydrolysis with alkaline water alone, the residual C-terminal-activated substance was not completely hydrolyzed, and it was also not possible to sufficiently remove the residual C-terminal-activated substance by subsequent aqueous washing; however, it was found that when hydrolysis was carried out with addition of DMAP, hydrolysis of the residual C-terminal-activated substance was completely achieved, and the residual C-terminal-activated substance could be completely removed. At this time, the dipeptide of interest was obtained in a purity of 98.6% (yield 97%).

(Example 20) Synthesis of Cbz-Thr(tBu)-Phe-OtBu

(Condensation reaction)

300 mg (1.2 mmol) of Phe-OtBu hydrochloride, 855 mg (1.7 mmol) of Cbz-Thr(tBu)-OH dicyclohexylamine salt, and 237 mg (1.8 mmol) of HOBt were suspended in 4.2 mL of 2-MeTHF and 0.9 mL of acetonitrile, and 813 μL (4.6 mmol) of diisopropylethylamine was added. Then, 447 mg (2.3 mmol) of EDC hydrochloride was added at 25°C, and the mixture was stirred at 25°C for 3 hours to carry out a peptide bond forming reaction. 5 μL of the reaction solution was isolated and added to 100 μL (1.2 mmol) of propylamine to convert the residual C-terminal-activated substance to propylamide, and then the mixture was diluted with 0.9 mL of methanol. This solution was subjected to LC/MS analysis to determine the conversion rate from the peak area of LC/MS (conversion rate: 100%).

$$\text{Coversion ratio (\%)} = \{\text{Cbz-Thr(tBu)-Phe-OtBu (area\%)} / [\text{Phe-OtBu (area\%)} + \text{Cbz-Thr(tBu)-Phe-OtBu (area\%)}]\} \times 100$$

(Hydrolysis treatment)

(1) When no amine was added

To the peptide-containing reaction solution prepared above, 3.0 mL of a 5% aqueous potassium carbonate solution was added, and the mixture was stirred with a stir bar at 25°C for 5 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(2) When amine was added

To the peptide-containing reaction solution prepared above, 142 mg (1.2 mmol) of DMAP and 3.0 mL of a 5% aqueous potassium carbonate solution were added, and the mixture was stirred with a stir bar at 25°C for 5 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(Work-up)

After stirring was terminated, the mixture was left to stand still to separate the organic layer and the aqueous layer, and the aqueous layer was removed. The organic layer was sequentially washed with 3 mL of a 10% aqueous potassium hydrogen sulfate solution, 3 mL of a 5% aqueous potassium carbonate solution, and 1.5 mL of water. 5 μL of the organic layer was isolated and added to 100 μL (1.2 mmol) of propylamine to convert the residual C-terminal-activated substance to propylamide, and then the mixture was diluted with 0.9 mL of methanol and subjected to LC/MS analysis to determine the peak area values of the peptide of interest and the residual C-terminal-activated substance (a converting substance to propylamide). The remaining organic layer was concentrated to give a peptide. MS (ESI): m/z 401.2 $[M-2tBu+H]^+$, 457.2 $[M-tBu+H]^+$, 513.3 $[M+H]^+$, 535.3 $[M+Na]^+$.

[Table 12]

| entry | | Immediately after hydrolysis treatment | Organic layer after liquid separation operation | |
|---|---|---|---|---|
| | Amine | Residual rate of C-terminal-activated substance (%) | Dipeptide (%)1 | Residual C-terminal-activated substance (%)1 (converting substance to propylamide) |
| 1 | DMAP | 0 | 98.4 | nd |
| 2 | none | 8.8 | 86.8 | 3.2 |
| 1) Peak area ratio of LCMS | | | | |

By hydrolysis with alkaline water alone, the residual C-terminal-activated substance was not completely hydrolyzed, and it was also not possible to sufficiently remove the residual C-terminal-activated substance by subsequent aqueous washing; however, it was found that when hydrolysis was carried out with addition of DMAP, hydrolysis of the residual C-terminal-activated substance was completely achieved, and the residual C-terminal-activated substance could be completely removed. At this time, the dipeptide of interest was obtained in a purity of 98.4%.

(Example 21) Synthesis of Cbz-Leu-Thr(tBu)-Phe-OtBu

(Cbz deprotection reaction using dipeptide obtained by hydrolysis treatment without addition of amine)

An MTBE/2-MeTHF solution of Cbz-Thr(tBu)-Phe-OtBu synthesized under the amine-free condition of Example 20 was concentrated and replaced with 2-methyltetrahydrofuran. The mixture was subjected to a hydrogenolysis reaction with 99 mg of 5% Pd/C (50% wet) and hydrogen gas. Despite stirring at 25°C for 1 hour, the reaction did not complete (conversion rate 53%). The reaction conversion rate was determined from the peak area value of LC/MS by isolating 5 μL of the reaction solution, diluting the solution with 1.0 mL of acetonitrile, and then subjecting the solution to LC/MS analysis.

Conversion rate (%) = {Thr(tBu)-Phe-OtBu (area%) / [Cbz-Thr(tBu)-Phe-OtBu (area%) + Thr(tBu)-Phe-OtBu (area%)]} × 100

(Cbz deprotection reaction using dipeptide obtained by hydrolysis treatment with addition of amine)

An MTBE/2-MeTHF solution of Cbz-Thr(tBu)-Phe-OtBu synthesized under the amine-added condition of Example 20 was concentrated and replaced with 2-methyltetrahydrofuran. The mixture was subjected to a hydrogenolysis reaction with 104 mg of 5% Pd/C (50% wet) and hydrogen gas. The mixture was stirred at 25°C for 1 hour to give a Cbz-removed product Thr(tBu)-Phe-OtBu (conversion rate 100%). The reaction conversion rate was determined from the peak area value of LC/MS by isolating 5 μL of the reaction solution, diluting the solution with 1.0 mL of acetonitrile, and then subjecting the solution to LC/MS analysis.

Conversion rate (%) = {Thr(tBu)-Phe-OtBu (area%) / [Cbz-Thr(tBu)-Phe-OtBu (area%) + Thr(tBu)-Phe-OtBu (area%)]} × 100

(Condensation reaction)

A reaction solution of a Cbz-deprotected product of dipeptide obtained by hydrolysis treatment with addition of amine was passed through a filter to filter off Pd/C, and then concentrated to dryness. The dried residue was dissolved in 5.0 mL of 2-methyltetrahydrofuran, and 382 mg (1.4 mmol) of Cbz-Leu-OH and 814 μL (4.7 mmol) of diisopropylethylamine were added. Then, 1.37 mL (2.3 mmol) of a 50% T3P/2-methyltetrahydrofuran solution was added, and the mixture was stirred at 25°C for 30 minutes to carry out a peptide bond forming reaction (conversion rate: 100%). The reaction conversion rate was determined from the peak area value of LC/MS by adding 5 μL of the reaction solution to 100 μL of propylamine, diluting the mixture with 0.9 mL of methanol, and then subjecting the mixture to LC/MS analysis.

Conversion rate (%) = {Cbz-Leu-Thr(tBu)-Phe-OtBu (area%) / [Thr(tBu)-Phe-OtBu (area%) + Cbz-Leu-Thr(tBu)-Phe-OtBu (area%)]} × 100

To the prepared reaction solution, 139 mg (1.1 mmol) of DMAP and 3.0 mL of a 10% aqueous sodium carbonate solution were added, and the mixture was stirred at 25°C for 5 minutes. Stirring was terminated, the mixture was then left to stand still to separate the organic layer and the aqueous layer, and the aqueous layer was removed. Then, the organic layer was sequentially washed with 3 mL × 2 of a 10% aqueous sodium hydrogen sulfate solution, 3 mL of a 5% aqueous sodium carbonate solution, and 3 mL of common water. 5 μL of the resulting organic layer was added to 100 μL of propylamine, and the mixture was diluted with 0.9 mL of methanol. This solution was subjected to LC/MS analysis to determine the peak area percentages of the peptide of interest and the residual C-terminal-activated substance. The peptide of interest Cbz-Leu-Thr(tBu)-Phe-OtBu was 98.3%, and Cbz-Leu-NHPr derived from the residual C-terminal-activated substance was not detected. The remaining organic layer was concentrated to give 638.0 mg of a concentrate (yield 88% from Phe-OtBu of Example 20). MS (ESI): m/z 514.3 $[M-2tBu+H]^+$, 570.3 $[M-tBu+H]^+$, 626.5 $[M+H]^+$, 648.4 $[M+Na]^+$.

It was found that when the C-terminal-activated substance derived from EDC or HOBt remained, the progress of the Cbz deprotection reaction was slower than when the residual C-terminal-activated substance was completely removed. It was found that when a peptide solution completely free of a residual C-terminal-activated substance was used as a starting material, the Cbz deprotection reaction proceeded smoothly, thus enabling a peptide synthesis reaction.

That is to say, it was found that the use of the method of the present invention enabled the reductive removal reaction of the N-terminal protecting group of the produced peptide compound to proceed without stagnation as in Example 9 and Example 18. Accordingly, it was possible to efficiently produce a high-purity peptide compound having a desired amino acid sequence.

(Example 22) Synthesis of Cbz-Ile-Phe-OtBu

(Condensation reaction)

301 mg (1.2 mmol) of Phe-OtBu hydrochloride and 465 mg (1.8 mmol) of Cbz-Ile-OH were suspended in 3.6 mL of MTBE and 0.9 mL of acetonitrile, and 610 μL (3.5 mmol) of diisopropylethylamine was added. Then, 479 mg (1.8 mmol) of BEP was added at 25°C, and the mixture was stirred at 25°C for 45 minutes to carry out a peptide bond forming reaction. 5 μL of the reaction solution was isolated and added to 100 μL (1.2 mmol) of propylamine to convert the residual C-terminal-activated substance to propylamide, and then the mixture was diluted with 0.9 mL of methanol. This solution was subjected to LC/MS analysis to determine the conversion rate from the peak area of LC/MS (conversion rate: 100%).

Conversion rate (%) = {Cbz-Ile-Phe-OtBu (area%) / [Phe-OtBu (area%) + Cbz-Ile-Phe-OtBu (area%)]} × 100

(Hydrolysis treatment)

(1) When no amine was added

To the peptide-containing reaction solution prepared above, 3.0 mL of a 5% aqueous potassium carbonate solution was added, and the mixture was stirred with a stir bar at 25°C for 3 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

Residual rate (%) of C-terminal-activated substance = {Propylamide (area%) / [Propylamide (area%) + Dipeptide (area%)]} × 100

(2) When amine was added

To the peptide-containing reaction solution prepared above, 139 mg (1.1 mmol) of DMAP and 3.0 mL of a 5% aqueous potassium carbonate solution were added, and the mixture was stirred with a stir bar at 25°C for 3 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

Residual rate (%) of C-terminal-activated substance = {Propylamide (area%) / [Propylamide (area%) + Dipeptide (area%)]} × 100

(Work-up)

After stirring was terminated, the mixture was left to stand still to separate the organic layer and the aqueous layer, and the aqueous layer was removed. Then, the organic layer was sequentially washed with 3 mL of a 10% aqueous potassium hydrogen sulfate solution and 3 mL of a 5% aqueous potassium carbonate solution. After 2 mL of 2-MeTHF was added, the mixture was washed with 1.5 mL of water. 5 μL of the organic layer was isolated and added to 100 μL (1.2 mmol) of propylamine to convert the residual C-terminal-activated substance to propylamide, and then the mixture was diluted with 0.9 mL of methanol and subjected to LC/MS analysis to determine the peak area values of the peptide

of interest and the residual C-terminal-activated substance (a converting substance to propylamide). The remaining organic layer was concentrated to give a peptide. MS (ESI): m/z 413.3 $[M-tBu+H]^+$, 469.3 $[M+H]^+$, 491.3 $[M+Na]^+$.

[Table 13]

| entry | | Immediately after hydrolysis treatment | Organic layer after liquid separation operation | |
|---|---|---|---|---|
| | Amine | Residual rate of C-terminal-activated substance (%) | Dipeptide (%)1 | Residual C-terminal-activated substance (%)1 (converting substance to propylamide) |
| 1 | DMAP | 0 | 96.3 | nd |
| 2 | none | 4.8 | 91.5 | 3.2 |
| 1) Peak area ratio of LCMS | | | | |

By hydrolysis with alkaline water alone, the residual C-terminal-activated substance was not completely hydrolyzed, and it was also not possible to sufficiently remove the residual C-terminal-activated substance by subsequent aqueous washing; however, it was found that when hydrolysis was carried out with addition of DMAP, hydrolysis of the residual C-terminal-activated substance was completely achieved, and the residual C-terminal-activated substance could be completely removed. At this time, the dipeptide of interest was obtained in a purity of 96.3%.

(Example 23) Cbz-Phe-MeGly-Phe-piperidine

(Boc deprotection reaction)

334 mg (1.0 mmol) of Boc-Phe-piperidine[1] was dissolved in 3.4 mL of dichloromethane, and 131 μL (2.0 mmol) of methanesulfonic acid was added. The mixture was stirred at 35°C for 3 hours to carry out a Boc removal reaction (conversion rate: 100%). The reaction conversion rate was determined from the peak area value of LC/MS by isolating 5 μL of the reaction solution, diluting the solution with 1.0 mL of acetonitrile, and then subjecting the mixture to LCMS analysis.

$$\text{Conversion rate (\%)} = \{\text{Phe-piperidine (area\%)} / [\text{Boc-Phe-piperidine (area\%)} + \text{Phe-piperidine (area\%)}]\} \times 100$$

(Condensation reaction)

After 528 μL (3.0 mmol) of diisopropylethylamine was added to the above reaction solution, the solvent was distilled off. Then, 1.0 mL of acetonitrile, 3.4 mL of 2-methyltetrahydrofuran, 528 μL (3.0 mmol) of diisopropylethylamine, 505 mg (1.5 mmol) of Cbz-Phe-MeGly-OH[2], and 256 mg (1.6 mmol) of HOOBt were added. 388 mg (2.0 mmol) of EDC hydrochloride was added at 25°C, and the mixture was stirred at 25°C for 1 hour to carry out a peptide bond forming reaction. 5 μL of the reaction solution was isolated and added to 100 μL (1.2 mmol) of propylamine to convert the residual C-terminal-activated substance to propylamide, and then the mixture was diluted with 0.9 mL of methanol. This solution was subjected to LC/MS analysis to determine the conversion rate from the peak area of LC/MS (conversion rate: 100%).

$$\text{Conversion rate (\%)} = \{\text{Cbz-Phe-MeGly-Phe-piperidine (area\%)} / [\text{Phe-piperidine (area\%)} + \text{Cbz-Phe-MeGly-Phe-piperidine (area\%)}]\} \times 100$$

Then, 128 mg (1.0 mmol) of DMAP and 3.5 mL of a 5% aqueous potassium carbonate solution were added to the reaction solution prepared above, and the mixture was stirred with a stir bar at 25°C for 3 minutes. After stirring was terminated, the mixture was left to stand still to separate the organic layer and the aqueous layer, and the aqueous layer was removed. The organic layer was diluted with 3.5 mL × 2 of a 10% aqueous potassium sulfate solution and 3.5 mL of a 5% aqueous potassium carbonate solution. This solution was subjected to LC/MS analysis to determine the peak area percentages of the peptide of interest and the residual C-terminal-activated substance. The peptide of interest Cbz-Phe-MeGly-Phe-piperidine had a purity of 94.6%, and Cbz-Phe-MeGly-NHPr derived from the residual C-terminal-activated substance was not detected. The remaining organic layer was concentrated to give 520.4 mg of a concentrate (yield 94%).

When hydrolysis was carried out with addition of DMAP, degradation/removal of the residual C-terminal-activated substance derived from a peptide fragment was completely achieved, and it was possible to obtain the intended tripeptide in a purity of 94.6% (yield 94%).

1) J. Org. Chem., 2003, 68, 7505-7508.
2) Bull. Chem. Soc. Jpn., 2004, 77, 1187-1193.

(Example 24) Synthesis of Cbz-Val-Phe-OtBu

(Condensation reaction)

First, 200 mg (0.8 mmol) of Phe-OtBu hydrochloride and 294 mg (1.2 mmol) of Cbz-Val-OH were suspended in 2.4 mL of 2-methyltetrahydrofuran and 0.6 mL of acetonitrile, and 294 μL (2.3 mmol) of N-ethylmorpholine was added. Then, 447 mg (1.2 mmol) of HATU was added at 25°C, and the mixture was stirred at 25°C for 2.5 hours to carry out a peptide bond forming reaction. 5 μL of the reaction solution was isolated and added to 100 μL (1.2 mmol) of propylamine to convert the residual C-terminal-activated substance to propylamide, and then the mixture was diluted with 0.9 mL of methanol. This solution was subjected to LC/MS analysis to determine the conversion rate from the peak area of LC/MS (conversion rate: 100%). MS: (ESI) m/z 399.3 $[M\text{-}tBu+H]^+$, 455.3 $[M+H]^+$

$$\text{Conversion rate (\%)} = \{\text{Cbz-Val-Phe-OtBu (area\%)} / [\text{Phe-OtBu (area\%)} + \text{Cbz-Val-Phe-OtBu (area\%)}]\} \times 100$$

(Hydrolysis treatment)

(1) When no amine was added

To the peptide-containing reaction solution prepared above, 2.0 mL of a 5% aqueous potassium carbonate solution was added, and the mixture was stirred with a stir bar at 25°C. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. This solution was subjected to LC/MS analysis, and from the peak area value of LC/MS, the residual rate of the C-terminal-activated substance was calculated according to the following calculation formula (the table below).

$$\text{Residual rate (\%) of C-terminal-activated substance} = \{\text{Propylamide (area\%)} / [\text{Propylamide (area\%)} + \text{Dipeptide (area\%)}]\} \times 100$$

(2) When amine was added

An amine additive (0.8 mmol) shown in the table below and 2.0 mL of a 5% aqueous potassium carbonate solution were added to the peptide-containing reaction solution prepared above, and the mixture was stirred with a stir bar at 25°C. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. This solution was subjected to LC/MS analysis, and from the LC/MS peak area value, the residual rate of the C-terminal-activated substance was calculated according to the following calculation formula (the table below).

$$\text{Residual rate (\%) of C-terminal-activated substance} = \{\text{Propylamide (area\%)} / [\text{Propylamide (area\%)} + \text{Dipeptide (area\%)}]\} \times 100$$

[Table 14]

| Change in the residual rate of the C-terminal-activated substance | | | | | |
|---|---|---|---|---|---|
| | | Residual rate of C-terminal-activated substance (%) | | | |
| entry | Amine additive | 5 min | 15 min | 30 min | 60 min |
| 1 | non | 20.1 | 17.8 | 14.1 | 10.5 |
| 2 | DIPEA | 19.2 | 15.8 | 10.7 | 6.7 |
| 3 | DMAP | 0.0 | 0.0 | 0.0 | 0.0 |
| 4 | NMI | 7.1 | 2.6 | 1.1 | 0.0 |

Addition of DIPEA promoted hydrolysis of the residual C-terminal-activated substance slightly more than that attained in the case of alkaline water alone without addition of amine, but no significant effect was observed. On the other hand, it was found that addition of DMAP and NMI promoted hydrolysis of the residual C-terminal-activated substance more significantly than that attained with addition of DIPEA, and especially when DMAP was used, the residual C-terminal-activated substance was completely hydrolyzed within 5 minutes. Accordingly, it was found that amines such as DMAP, which have a small steric hindrance in the vicinity of nitrogen, promoted hydrolysis of the residual C-terminal-activated substance more than amines such as DIPEA that have steric hindrance in the vicinity of nitrogen.

(Example 25) Synthesis of Cbz-Ile-Val-OBn

(Condensation reaction)

300 mg (1.2 mmol) of Val-OBn hydrochloride and 495 mg (1.9 mmol) of Cbz-Ile-OH were suspended in 3.0 mL of cyclopentyl methyl ether and 0.9 mL of acetonitrile, and 859 μL (4.9 mmol) of diisopropylethylamine was added. Then, 705 mg (1.9 mmol) of HATU was added at 25°C, and the mixture was stirred at 25°C for 1 hour to carry out a peptide bond forming reaction. 5 μL of the reaction solution was isolated and added to 100 μL (1.2 mmol) of propylamine to convert the residual C-terminal-activated substance to propylamide, and then the mixture was diluted with 0.9 mL of methanol. This solution was subjected to LC/MS analysis to determine the conversion rate from the peak area of LC/MS (conversion rate: 100%).

$$\text{Conversion rate (\%)} = \{\text{Cbz-Ile-Val-OBn (area\%)} / [\text{Val-OBn (area\%)} + \text{Cbz-Ile-Val-OBn (area\%)}]\} \times 100$$

(Hydrolysis treatment)

(1) When no amine was added

To the peptide-containing reaction solution prepared above, 3.0 mL of neutral water was added, and the mixture was stirred with a stir bar at 25°C for 5 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(2) When amine was added

To the peptide-containing reaction solution prepared above, 91 mg (0.7 mmol) of DMAP and 3.0 mL of neutral water were added, and the mixture was stirred with a stir bar at 25°C for 5 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted

with 0.9 mL of methanol. From the peak area value of LC/MS, the residual rate of the C-terminal-activated substance was calculated according to the following calculation formula.

Residual rate (%) of C-terminal-activated substance = {Propylamide (area%) / [Propylamide (area%) + Dipeptide (area%)]} × 100

(Work-up)

After stirring was terminated, the mixture was left to stand still to separate the organic layer and the aqueous layer, and the aqueous layer was removed. Then, the organic layer was sequentially washed with 3 mL of a 10% aqueous sodium hydrogen sulfate solution, 3 mL×2 of a 5% aqueous sodium carbonate solution, and 1.5 mL × 3 of common water. 5 μL of the organic layer was isolated and added to 100 μL (1.2 mmol) of propylamine to convert the residual C-terminal-activated substance to propylamide, and then the mixture was diluted with 0.9 mL of methanol and subjected to LC/MS analysis to determine the peak area values of the peptide of interest and the residual C-terminal-activated substance (a converting substance to propylamide). The remaining organic layer was concentrated to give a peptide. The concentrate (peptide) obtained by hydrolysis without addition of amine was 744 mg (yield 132%: calculated as solely containing peptide although the concentrate contained impurities (residual C-terminal-activated substance)). The concentrate obtained by hydrolysis with addition of amine was 528 mg (yield 94%). MS (ESI): m/z 455.3 $[M+H]^+$, 477.3 $[M+Na]^+$.

[Table 15]

| entry | | Immediately after hydrolysis treatment | Organic layer after liquid separation operation | | After concentration |
|---|---|---|---|---|---|
| | Amine | Residual rate of C-terminal-activated substance (%) | Residual C-terminal-activated substance (%)1 (converting substance to propylamide) | Dipeptide (%)1 | Yield (%) |
| 1 | DMAP | 0 | nd | 99.5 | 94 |
| 2 | none | 19.5 | 17.1 | 81.2 | 132 |
| 1) Peak area ratio of LCMS | | | | | |

By hydrolysis with neutral water alone, the residual C-terminal-activated substance was not completely hydrolyzed, and it was also not possible to remove the residual C-terminal-activated substance by subsequent aqueous washing; however, it was found that when hydrolysis was carried out with addition of DMAP, hydrolysis of the residual C-terminal-activated substance was completely achieved, and the residual C-terminal-activated substance could be completely removed. At this time, the dipeptide of interest was obtained in a purity of 99.5% (yield 94%).

(Example 26) Synthesis of Cbz-MeAla-Phe-OtBu

(Condensation reaction)

200 mg (0.8 mmol) of Phe-OtBu hydrochloride and 260 mg (1.2 mmol) of Cbz-MeAla-OH were suspended in 2.4 mL of 2-methyltetrahydrofuran and 0.6 mL of acetonitrile, and 271 μL (1.6 mmol) of diisopropylethylamine was added. Then, 265 mg (0.8 mmol, water content 13 w%) of DMT-MM-n-hydrate (4-(4,6-dimethoxy-1,3,5-triazin-2-yl)-4-methylmorpholinium chloride n-hydrate) was added at 25°C, the mixture was stirred at 25°C for 1 hour, then 119 mg (0.4 mmol, water content 13 w%) of DMT-MM-n-hydrate was added, and the mixture was stirred at 25°C for 1 hour to carry out a peptide bond forming reaction. 5 μL of the reaction solution was isolated and added to 100 μL (1.2 mmol) of propylamine to convert the residual C-terminal-activated substance to propylamide, and then the mixture was diluted with 0.9 mL of methanol. This solution was subjected to LC/MS analysis to determine the conversion rate from the peak area of LC/MS (conversion rate: 100%).

Conversion rate (%) = {Cbz-MeAla-Phe-OtBu (area%) / [Phe-OtBu (area%) + Cbz-MeAla-Phe-OtBu (area%)]} × 100

(Hydrolysis treatment)

(1) When no amine was added

To the peptide-containing reaction solution prepared above, 2.0 mL of a 5% aqueous potassium carbonate solution was added, and the mixture was stirred with a stir bar at 25°C for 10 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(2) When amine was added

To the peptide-containing reaction solution prepared above, 96 mg (0.8 mmol) of DMAP and 2.0 mL of a 5% aqueous potassium carbonate solution were added, and the mixture was stirred with a stir bar at 25°C for 10 minutes. Stirring was terminated to separate the organic layer and the aqueous layer. 5 μL of the organic layer was isolated and added to 100 μL (1.2 mmol) of propylamine to convert the residual C-terminal-activated substance to propylamide, and then the mixture was diluted with 0.9 mL of methanol. From the peak area value of LC/MS, the residual rate of the C-terminal-activated substance was calculated according to the following calculation formula.

$$\text{Residual rate (\%) of C-terminal-activated substance} = \{\text{Propylamide (area\%)} / [\text{Propylamide (area\%)} + \text{Dipeptide (area\%)}]\} \times 100$$

(Work-up)

After stirring was terminated, the mixture was left to stand still to separate the organic layer and the aqueous layer, and the aqueous layer was removed. Then, the organic layer was sequentially washed with 2 mL of a 10% aqueous potassium hydrogen sulfate solution, 2 mL of a 5% aqueous potassium carbonate solution, and 1 mL × 2 of common water. 5 μL of the organic layer was isolated and added to 100 μL (1.2 mmol) of propylamine to convert the residual C-terminal-activated substance to propylamide, and then the mixture was diluted with 0.9 mL of methanol and subjected to LC/MS analysis to determine the peak area values of the peptide of interest and the residual C-terminal-activated substance (a converting substance to propylamide). The remaining organic layer was concentrated to give a peptide. The concentrate (peptide) obtained by hydrolysis without addition of amine was 387 mg (yield 114%: calculated as solely containing peptide although the concentrate contained impurities (residual C-terminal-activated substance)). The concentrate obtained by hydrolysis with addition of amine was 336 mg (yield 98%). MS (ESI): m/z 385.2 $[M\text{-}tBu+H]^+$, 441.3 $[M+H]^+$, 463.3 $[M+Na]^+$.

[Table 16]

| entry | | Immediately after hydrolysis treatment | Organic layer after liquid separation operation | | After concentration |
|---|---|---|---|---|---|
| | Amine | Residual rate of C-terminal-activated substance (%) | Residual C-terminal-activated substance (%)1 (converting substance to propylamide) | Dipeptide (%)1 | Yield (%) |
| 1 | DMAP | 0.0 | 0 | 98. 6 | 98 |
| 2 | none | 8. 1 | 6. 3 | 88. 8 | 114 |
| 1) Peak area ratio of LCMS | | | | | |

In the case of using DMT-MM as a condensing agent, the residual C-terminal-activated substance was not completely hydrolyzed by hydrolysis with alkaline water alone, and it was also not possible to remove the residual C-terminal-activated substance by subsequent aqueous washing; however, it was found that when hydrolysis was carried

out with addition of DMAP, hydrolysis of the residual C-terminal-activated substance was completely achieved, and the residual C-terminal-activated substance could be completely removed. At this time, the dipeptide of interest was obtained in a purity of 98.6% (yield 98%). It is known that a C-terminal-activated substance produced from DMT-MM, which is a condensing agent usable even in a water-containing solvent, is relatively resistant to hydrolysis. However, it was found that the use of an amine additive enabled even a residual C-terminal-activated substance prepared using DMT-MM to be completely hydrolyzed in a short period of time by a single treatment, and completely removed by the subsequent aqueous washing.

Reference Example 1: Synthetic method of MeAsp(tBu)-piperidine

(Condensation reaction)

10.2 g (19.6 mmol) of Cbz-MeAsp(tBu)-OH dicyclohexylamine salt was suspended in 100 mL of ethyl acetate, and 20.6 mL (118 mmol) of diisopropylethylamine and 9.7 mL (98.0 mmol) of piperidine were added. 35.0 mL (58.9 mmol) of a 50% T3P/ethyl acetate solution was added dropwise at 3 to 10°C over 45 minutes. After completion of dropwise addition, the reaction conversion rate was determined from the peak area value of LC/MS by isolating 5 $\mu$L of the reaction solution, diluting the solution with 1.0 mL of methanol, and then subjecting the solution to LCMS analysis (conversion rate: 100%).

$$\text{Conversion rate (\%)} = \{\text{Cbz-MeAsp(tBu)-piperidine (area\%)} / [\text{Cbz-MeAsp(tBu)-OH (area\%)} + \text{Cbz-MeAsp(tBu)-piperidine (area\%)}]\} \times 100$$

After the reaction solution was washed with 100 mL of a 10% aqueous potassium hydrogen sulfate solution and 100 mL of 10% potassium carbonate, the resulting organic layer was dried over magnesium sulfate, filtered, and concentrated. MS (ESI) m/z 349.1 $[M\text{-}tBu+H]^+$, 405.2 $[M+H]^+$, 427.3 $[M+Na]^+$.

(Cbz deprotection reaction)

The concentrate was dissolved in 100 mL of cyclopentyl methyl ether and subjected to a hydrogenolysis reaction with 2.0 g of 5% Pd/C (50% wet) and hydrogen gas. The mixture was stirred at room temperature for 5 hours to give the intended MeAsp(tBu)-piperidine (conversion rate 100%). The reaction conversion rate was determined from the peak area value of LC/MS by isolating 5 $\mu$L of the reaction solution, diluting the solution with 1.0 mL of acetonitrile, and then subjecting the mixture to LCMS analysis.

$$\text{Conversion rate (\%)} = \{\text{MeAsp(tBu)-piperidine (area\%)} / [\text{Cbz-MeAsp(tBu)-piperidine (area\%)} + \text{MeAsp(tBu)-piperidine (area\%)}]\} \times 100$$

After the reaction solution was passed through a filter, the filtrate was concentrated to give 5.69 g of a concentrate (yield: quant.). This concentrate was subjected to LC/MS analysis to determine the peak area percentage of the intended MeAsp(tBu)-piperidine (99.2 area%). MS (ESI): m/z 215.1 $[M\text{-}tBu+H]^+$, 271.1 $[M+H]^+$.

[Industrial Applicability]

The present invention is capable of producing a high-purity peptide compound without column purification by efficiently removing a C-terminal-activated substance remaining after a

## Claims

**1.** A method of producing a peptide compound, comprising:

step A: a step of obtaining a reaction mixture comprising a peptide compound obtained by condensing a C-terminal-activated substance of an acid component with an amine component in a solvent; and
step B: a step of mixing the reaction mixture, a tertiary amine, and water or an aqueous solution to remove the C-terminal-activated substance.

2. The method of claim 1, wherein the tertiary amine is nucleophilic to the C-terminal-activated substance.

3. The method of claim 1 or 2, wherein the tertiary amine is an amine having small steric hindrance in the vicinity of nitrogen.

4. The method of any one of claims 1 to 3, wherein the tertiary amine is NMI, DMAP, or trimethylamine.

5. The method of any one of claims 1 to 4, wherein the peptide compound comprises one or more non-natural amino acids.

6. The method of any one of claims 1 to 5, wherein a temperature for allowing the tertiary amine to act on the C-terminal-activated substance is 25°C to 60°C.

7. The method of any one of claims 1 to 6, wherein the tertiary amine is added in an amount of 0.5 equivalents or more relative to the amine component.

8. The method of any one of claims 1 to 7, wherein the C-terminal-activated substance is allowed to be acted on by the tertiary amine and hydrolyzed, and is removed.

9. The method of any one of claims 1 to 8, wherein step B further comprises separating the reaction mixture into an organic layer and an aqueous layer and then washing the organic layer, and wherein a residual amount of the C-terminal-activated substance after the washing is 1.0% or less.

10. The method of any one of claims 1 to 9, wherein the solvent in step A is toluene, acetonitrile, tetrahydrofuran, 2-methyltetrahydrofuran, isopropyl acetate, ethyl acetate, methyl tert-butyl ether, or cyclopentyl methyl ether, N,N-dimethylformamide, or a mixed solvent thereof.

11. The method of any one of claims 1 to 10, wherein in step B, the aqueous solution is an aqueous potassium carbonate solution or an aqueous sodium carbonate solution.

12. The method of any one of claims 1 to 11, wherein the acid component is a first amino acid having an amino group protected with a protecting group, and wherein a side chain of the first amino acid comprises one or more carbon atoms.

13. The method of claim 12, wherein the side chain is optionally substituted alkyl, optionally substituted alkenyl, optionally substituted alkynyl, optionally substituted cycloalkyl, optionally substituted alkoxyalkyl, optionally substituted cycloalkylalkyl, optionally substituted aralkyl, or optionally substituted heteroarylalkyl.

14. The method of any one of claims 1 to 13, wherein a time for allowing the tertiary amine to act on the C-terminal-activated substance is 2 hours or less.

15. The method of any one of claims 1 to 14, wherein the C-terminal-activated substance is formed in the presence of a condensing agent, and wherein the condensing agent comprises T3P, HATU, BEP, DMT-MM, a combination of EDC and PfpOH, a combination of EDC and HOOBt, or a combination of EDC and HOBt.

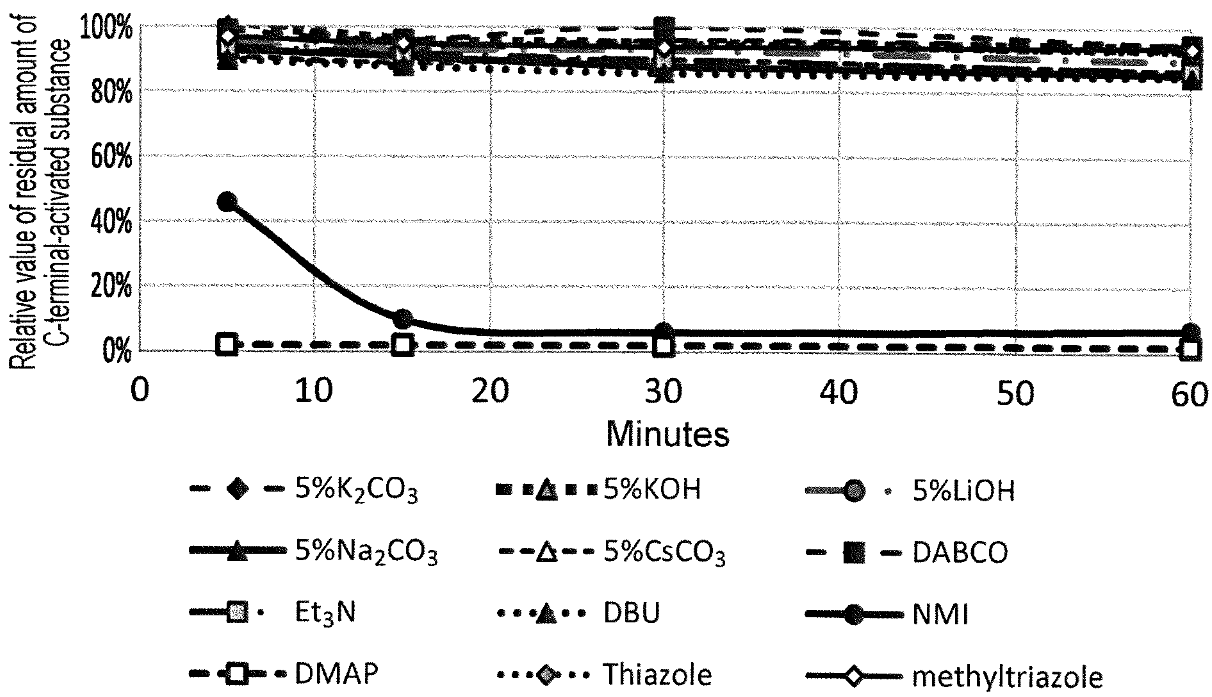
Relative value of residual amount of C-terminal-activated substance
100%
80%
60%
40%
20%
0%
0
10
20
30
40
50
60
Minutes
5%K2CO3
5%KOH
5%LiOH
5%Na2CO3
5%CsCO3
DABCO
Et3N
DBU
NMI
DMAP
Thiazole
methyltriazole

FIG. 1

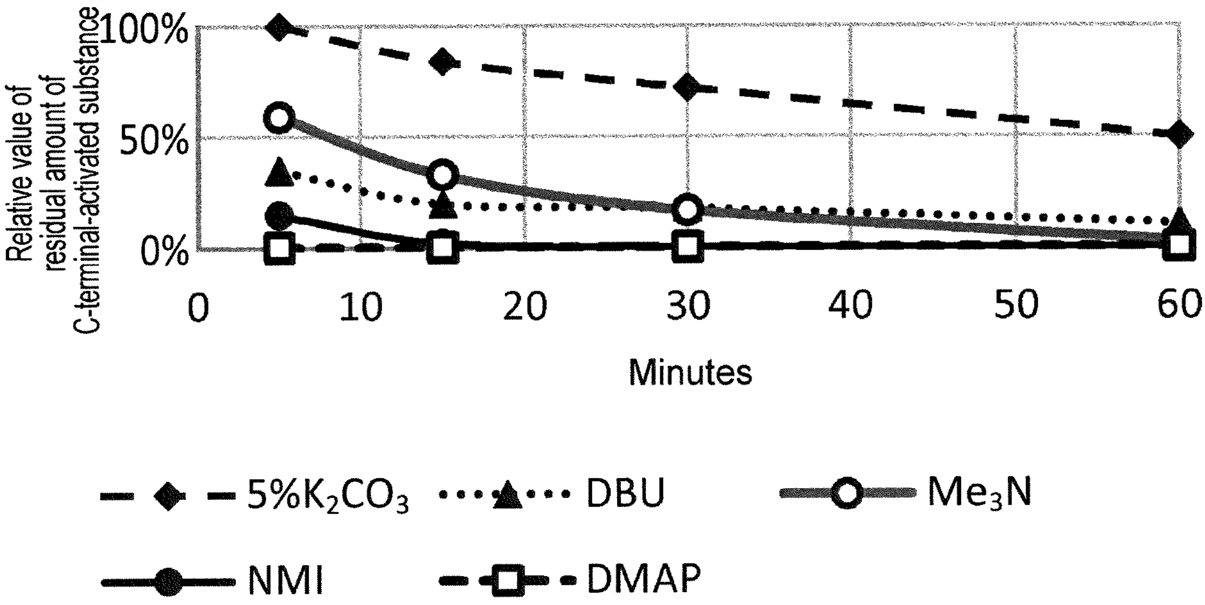
Relative value of residual amount of C-terminal-activated substance
100%
50%
0%
0
10
20
30
40
50
60
Minutes
5%K2CO3
DBU
Me3N
NMI
DMAP

FIG. 2

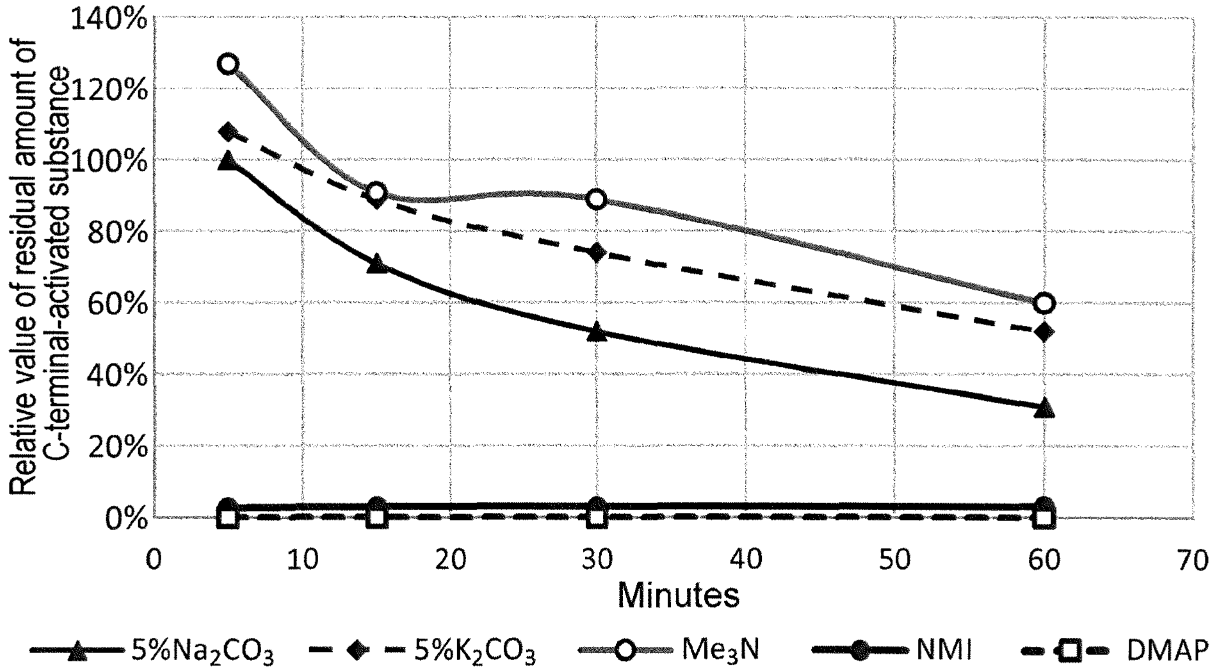
140%
120%
100%
80%
60%
40%
20%
0%
Relative value of residual amount of C-terminal-activated substance
0
10
20
30
40
50
60
70
Minutes
5%$Na_2CO_3$
5%$K_2CO_3$
$Me_3N$
NMI
DMAP

FIG. 3

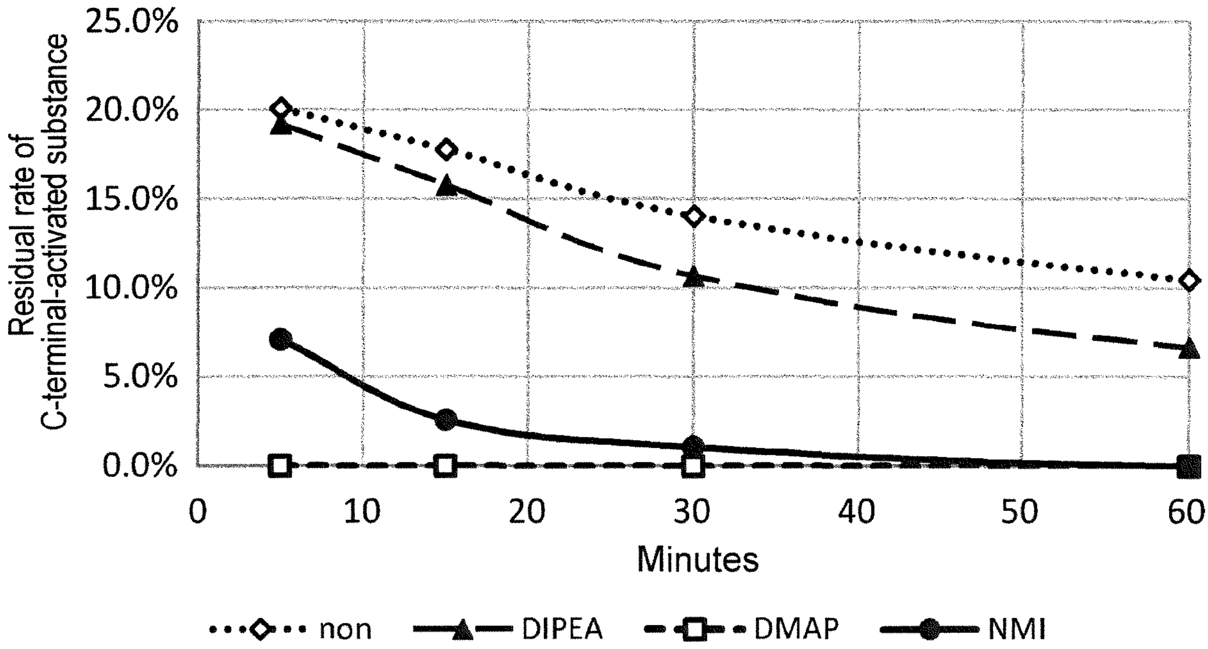
25.0%
20.0%
15.0%
10.0%
5.0%
0.0%
Residual rate of
C-terminal-activated substance
0
10
20
30
40
50
60
Minutes
non
DIPEA
DMAP
NMI

FIG. 4

## INTERNATIONAL SEARCH REPORT

International application No. PCT/JP2020/048651

A. CLASSIFICATION OF SUBJECT MATTER
C07K 1/06(2006.01)i
FI: C07K1/06 ZNA
According to International Patent Classification (IPC) or to both national classification and IPC

B. FIELDS SEARCHED
Minimum documentation searched (classification system followed by classification symbols)
C07K1/06

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | SHIINA, I. et al., "4-(Dimethylamino)pyridine N-oxide (DMAPO): An Effective Nucleophilic Catalyst in the Peptide Coupling Reaction with 2-Methyl-6-nitrobenzoic Anhydride", Chem. Asian J., 2008, vol. 3, pp. 454-461 page 458, synthesis | 1-7, 9-10, 12-15 |
| X | WO 2007/099656 A1 (KANEKA CORP.) 07 September 2007 (2007-09-07) paragraphs [0021], [0127], examples | 1-15 |
| A | JP 2003-55396 A (AKZO NOBEL N.V.) 26 February 2003 (2003-02-26) | 1-15 |
| A | WO 2009/014176 A1 (AJINOMOTO CO., INC.) 29 January 2009 (2009-01-29) | 1-15 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 February 2021 (09.02.2021) | 09 March 2021 (09.03.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application No.
PCT/JP2020/048651

| Patent Documents referred in the Report | Publication Date | Patent Family | Publication Date |
|---|---|---|---|
| WO 2007/099656 A1 | 07 Sep. 2007 | US 2009/0069538 A1 paragraphs [0027]-[0032], [0150], examples<br>US 2014/0288268 A1<br>EP 1995254 A1<br>ES 2729197 T | |
| JP 2003-55396 A | 26 Feb. 2003 | US 2003/0018164 A1<br>US 2004/0082760 A1<br>US 2004/0087768 A1<br>US 2006/0063918 A1<br>EP 1291356 A2<br>NO 324245 B<br>HU 202369 A<br>PL 355125 A<br>HR 20020609 A<br>BR 202783 A<br>CA 2390358 A<br>IL 150601 A<br>AT 302792 T<br>HK 1050904 A<br>DK 1291356 T<br>PT 1291356 E<br>ES 2248485 T<br>TW 247012 N<br>KR 10-2003-0009188 A<br>CN 1398876 A<br>RU 2002119630 A<br>SG 119160 A<br>ZA 200205409 A<br>EC SP024291 A<br>AR 34869 A<br>PE 2142003 A<br>MX PA02006998 A<br>HR P20020609 A<br>PE 20030214 A<br>NO 20023446 D0 | |
| WO 2009/014176 A1 | 29 Jan. 2009 | JP 5212371 B2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5171613 B
- JP 4142907 B
- JP 5212371 B


### Non-patent literature cited in the description


- *Chem. Rev.,* 2011, vol. 111, 6557
- *Organic Process Research & Development,* 2016, vol. 20, 140
- *Tetrahedron Lett.,* 1974, vol. 15, 1785
- *Organic Process Research & Development,* 2016, vol. 20 (2), 140
- **GREENE'S.** Protective Groups in Organic Synthesis. John Wiley & Sons, 2014
- *J. Org. Chem.,* 2003, vol. 68, 7505-7508
- *Bull. Chem. Soc. Jpn.,* 2004, vol. 77, 1187-1193